# EUROPEAN PATENT SPECIFICATION

(11) **EP 3 170 111 B1**
(45) Date of publication and mention of the grant of the patent: **27.10.2021**
(21) Application number: 15744826.7
(22) Date of filing: 17.07.2015
(51) Int. Cl.: G16H 15/00, G16H 40/67, H04W 4/70, G06F 16/36

(54) **M2M ONTOLOGY MANAGEMENT AND SEMANTICS INTEROPERABILITY**
M2-ONTOLOGIE-VERWALTUNG UND SEMANTIKINTEROPERABILITÄT
GESTION D'ONTOLOGIES EN M2M ET INTEROPÉRABILITÉ DE SÉMANTIQUES

(30) Priority: 18.07.2014 US 201462026569 P
(43) Date of publication of application: 24.05.2017
(73) Proprietor: Convida Wireless LLC, Wilmington, DE 19809-3727 (US)
(72) Inventor: DONG, Lijun, San Diego, CA 92130 (US); LU, Guang, Thornhill, ON L4J 9B6 (CA); MLADIN, Catalina M., Hatboro, PA 19040 (US); LI, Hongkun, Malvern, PA 19355 (US); LI, Xu, Plainsboro, NJ 08536 (US); SEED, Dale N., Allentown, PA 18104 (US); FLYNN, William Robert IV, Schwenksville, PA 19473 (US); WANG, Chonggang, Princeton, NJ 08540 (US)
(74) Representative: D Young & Co LLP
(86) International application number: PCT/US2015/040921
(87) International publication number: WO 2016/011361

(56) References cited:
- LUIS IRIBARNE ET AL: "Open-Environmental Ontology Modeling", IEEE TRANSACTIONS ON SYSTEMS, MAN AND CYBERNETICS. PART A:SYSTEMS AND HUMANS, IEEE SERVICE CENTER, PISCATAWAY, NJ, US, vol. 41, no. 4, 1 July 2011 (2011-07-01), pages 730-745, XP011366649, ISSN: 1083-4427, DOI: 10.1109/TSMCA.2011.2132706
- Gokce B Laleci ET AL: "SAPHIRE: A Multi-Agent System for Remote Healthcare Monitoring through Computerized Clinical Guidelines" In: "Agent Technology and e-Health", 1 January 2008 (2008-01-01), Birkhäuser Basel, Basel, XP055218125, ISBN: 978-3-76-438546-0 pages 25-44, DOI: 10.1007/978-3-7643-8547-7_3, the whole document
- Leonardo Lezcano ET AL: "COMBINING ONTOLOGIES AND RULES WITH CLINICAL ARCHETYPES PhD THESIS SUBMITTED TO THE UNIVERSITY OF ALCALÁ", , 1 January 2011 (2011-01-01), XP055219822, Retrieved from the Internet: URL:http://leo9r.com/pdf/Lezcano_thesis.pd f [retrieved on 2015-10-14]
- "Machine to Machine Communications (M2M); Study on Semantic support for M2M Data;TR_101_584_v050_Clean", ETSI DRAFT; TR_101_584_V050_CLEAN, EUROPEAN TELECOMMUNICATIONS STANDARDS INSTITUTE (ETSI), 650, ROUTE DES LUCIOLES ; F-06921 SOPHIA-ANTIPOLIS ; FRANCE, vol. M2M, no. V0.5.0, 7 December 2012 (2012-12-07), pages 1-36, XP014094602, [retrieved on 2012-12-07]
- PATRICK ZIEGLER ET AL: "Detecting Similarities in Ontologies with the SOQA-SimPack Toolkit", 1 January 2006 (2006-01-01), ADVANCES IN DATABASE TECHNOLOGY - EDBT 2006 LECTURE NOTES IN COMPUTER SCIENCE;;LNCS, SPRINGER, BERLIN, DE, PAGE(S) 59 - 76, XP019029247, ISBN: 978-3-540-32960-2

## Description

### CROSS-REFERENCE TO RELATED APPLICATIONS

This application claims the benefit of U.S. Provisional Patent Application No. 62/026569, filed on July 18, 2014, entitled "M2M ONTOLOGY MANAGEMENT AND SEMANTICS INTEROPERABILITY".

### FIELD

The present application is directed to a device and method for machine-to-machine, M2M, ontology processing as defined in the appended claims. The scope of the invention is determined by the independent claims. Preferable embodiments are determined by the dependent claims.

### BACKGROUND

The rapid increase in the number of network-enabled devices and sensors deployed in physical environments is changing communication networks. It is predicted that within the next decade billions of devices will generate a myriad of real world data for many applications and services by service providers in a variety of areas such as smart grids, smart homes, e-health, automotive, transport, logistics, and environmental monitoring. The related technologies and solutions that enable integration of real world data and services into the current information networking technologies are often described under the umbrella terms of the Internet of things (IoT) or machine-to-machine (M2M) communications. Because of the large amount of data created by devices there is a need for an efficient way to identify and query this data.

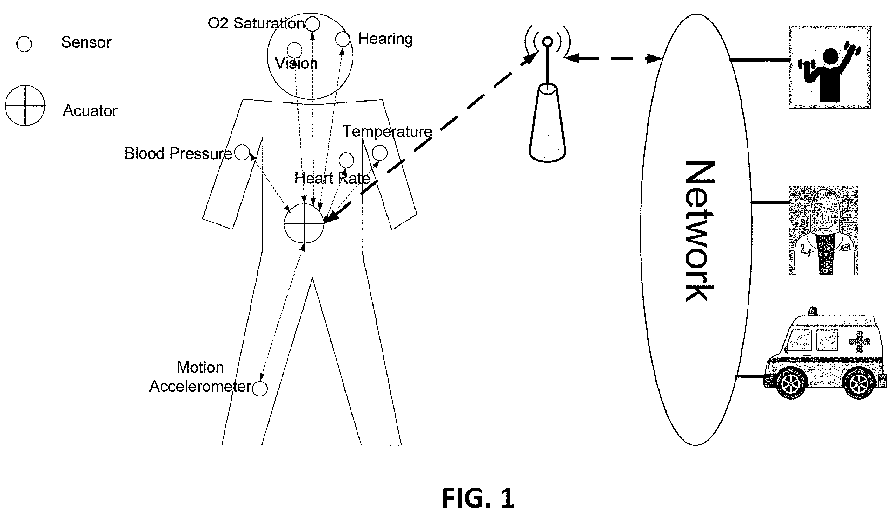

FIG. 1 illustrates an example patient monitoring application that may be provided by a patient's hospital or rehabilitation center using compact biomedical wireless sensor motes that use an actuator as an aggregation point. The actuator transmits data to the network. These small wearable resource constrained devices are examples of M2M devices that may be deployed on a patient to continuously monitor vital signs such as blood pressure and flow, core temperature, oxygen saturation, motion, heart rate, hearing, and vision, among other things. Various kinds of M2M data collected by the M2M devices may be used by the patient's doctor, personal trainer (e.g. from 24 hour fitness), and/or an ambulance service, as depicted in FIG. 1. In order to enable the doctor, personal trainer, and the ambulance service to use the data generated from those M2M device, the semantics of those resources need to be available too. The semantics provide a descriptive definition of the data such that the format and structure of the data can be understood (i.e., the semantics provide meaning for the data).

M2M semantic support is intended to enable semantics to the original resources for universal understanding/interpretation of them, as well as any advanced processing on them, e.g., semantic query, data analytics, etc. Introduced in the background section are the existing technologies that are developed in Semantics Web and the existing ontologies that are developed in different namespaces. In addition, the oneM2M architecture and service layer are introduced.

### Existing Technologies in Semantics Web

The Semantics Web uses a combination of a schema language and an ontology language to provide the capabilities of ontologies. An ontology uses a predefined, reserved vocabulary to define classes and the relationships between them for a specific area of interest, or more. Resource Description Framework Schema (RDFS) as defined by the World Wide Web Consortium (W3C) (e.g., RDF Vocabulary Description Language 1.0: RDF Schema) provides a specific vocabulary for Resource Description Framework (RDF) that may be used to define classes and properties. The Web Ontology Language (OWL) extends the RDFS vocabulary with additional resources that may be used to build more expressive ontologies for the web.

RDF is a framework for representing information in the Web. RDF is essentially a data-model. Its basic building block is a resource-property-value triple, called a statement. RDF has been given syntax in XML, JSON, etc.

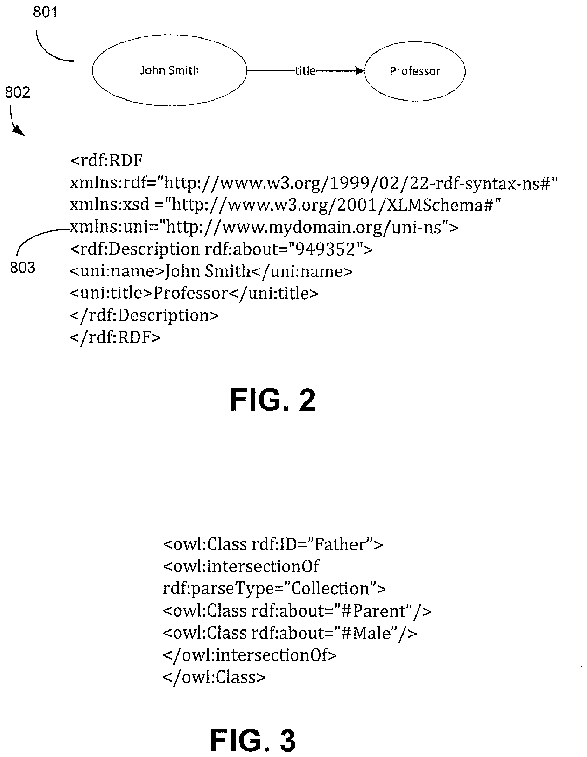

FIG. 2 illustrates a semantics example, which may use RDF. In the example, a statement 801 shows that John Smith's title is Professor, which may be represented in RDF language 802, as shown in FIG. 2. The xmlns:uni 803 is the self-defined domain name, in which the properties (Name and title) are defined as part of the RDFS. RDF is domain-independent, such that no assumptions about a particular domain of use are made. It is up to the users to define their own terminology in a schema language called RDF Schema (RDFS). RDFS defines the vocabulary used in RDF data models. In RDFS, the vocabulary may be defined, relationships between object may be described, and properties may be specified to apply to particular kinds of objects, which may include the values the objects can take.

The web ontology language (OWL) extends the RDFS vocabulary with additional resources that may be used to build more expressive ontologies for the web. OWL introduces additional restrictions regarding the structure and contents of RDF documents in order to make processing, reasoning more computationally feasible. For example, OWL defines properties that correspond to the standard set operators: intersection, union, and complement to define Boolean combinations of classes. FIG. 3 illustrates an example of using intersection to define the concept Father. Father is exactly the intersection of the classes Parent and Male. In other words, anyone who is a Father is both a Parent and a Male, and anyone who is both a Parent and a Male is a Father. At first glance, it might appear that the OWL is equivalent to saying Father is rdfs:subClassOf Parent and Father is rdfs:subclassOf Male. However, these two subClassOf statements only state that all fathers must be parents and male. They cannot be used to infer that someone is a father from only their gender and parenthood, as can be done using owl: intersectionOf.

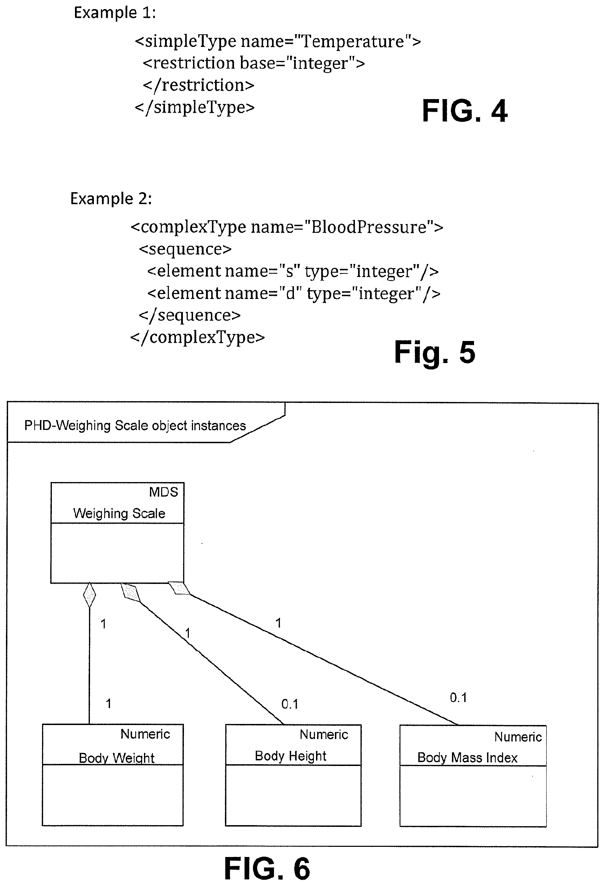

OWL uses the RDF and RDFS, XML schema datatypes, and OWL namespaces. The OWL vocabulary itself is defined in the namespace http://www.w3.org/2002/07/owl# and is commonly referred to by the prefix owl. Datatypes represent ranges of data values that are identified using URIs. OWL allows you to use a number of predefined datatypes, most of which are defined in the XML Schema Definition (XSD) namespace. OWL allows a user to define her own datatypes by extending XSD. FIG. 4 and FIG. 5 both illustrate examples of defining datatypes in XSD.

### Existing Ontologies from Different Namespaces

There are many ontologies that have been developed and shared. They can provide a basis for extension and reuse. There are also a number of ontologies that provide an in-depth representation of a narrowly scoped area of interest. With this kind of ontology, it may not necessarily be extended, rather simply be used directly or generalized. The followings are some examples of such kinds of ontologies that are highly relevant to M2M system.

The Friend of a Friend (FOAF) project maintains the FOAF ontology, which expresses information relevant to sharing information about friends on the World Wide Web. The ontology contains classes and properties for capturing personal information, email addresses, online account and instant messaging information, as well as online documents and images. The goal of the FOAF project is to provide a means to capture the user's online life in an application-independent and website-independent manner in order to break down the walls that divide the various communities on the Web. The examples of common properties defined for an individual (foaf:Person) include foaf:name, foaf:title, foaf:homepage, foaf:interest, and foaf:topic_interest, among others.

The Semantic Sensor Network (SSN) Ontology is developed by the W3C Semantic Sensor Networks Incubator Group (SSN-XG). The SSN ontology describes sensors and observations, and related concepts. SSN ontology model contains the classes and properties that may be used to represent particular aspects of a sensor or its observations, such as sensors, observations, features of interest, the process of sensing (e.g., how a sensor operates and observes), how sensors are deployed or attached to platforms, the measuring capabilities of sensors, as well as their environmental, and survival properties of sensors in particular environments. Examples of classes may include ssn:Sensor, ssn:SensorInput, and ssn:SensorOutput, among others. Examples of properties may include ssn:hasMeasurementCapability, ssn:observationSamplingTime, and ssn:qualityOfObservation, among others. See http://www.w3.org/2005/Incubator/ssn/XGR-ssn-20110628/images/OntStructure-Overview.jpg.

IEEE 11073 (ISO/IEEE 11073, Health informatics - Medical / health device communication standards) Personal Health Data (PHD) standards are a group of standards addressing the interoperability of personal health devices such as weighing scales, blood pressure monitors, blood glucose monitors, and the like.

IEEE 11073-20601 (IEEE 11073-20601, Health informatics--Personal health device communication--Part 20601: Application profile--Optimized exchange protocol) is the framework standard which defines the generic data types, message types and communication model. This supports any number of (relatively small) "device specialization" standards (such as the IEEE 11073-10415 standard for weight scale) which need only define the data model for that particular type of personal health device.

The IEEE 11073-20601 defines the classes such as medical device systems (MDS) and metric class. For MDS each Agent has one MDS object, which identifies it and reports its status. The attributes of the MDS object identify it to the Manager, represent the time and status, and provide other information. The MDS then contains zero or more of some of the objects represented by the classes below. Metric Class is the base class for all objects representing measurements, status and context data, which also has many subclasses, for example a numeric may represent a single measurement.

FIG. 6 illustrates a practical example in which the Domain Information Model of the weighing scales is defined by IEEE 11073-10415. FIG. 6 shows that every weighing scale contains one required numeric object for body weight and two optional numeric objects for body height and body mass index.

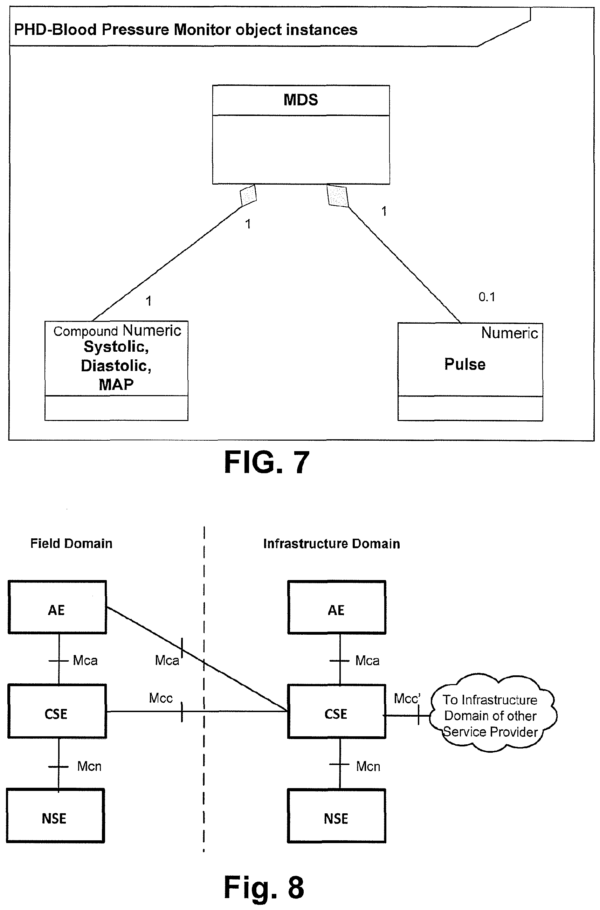

FIG. 7 illustrates another practical example in which the Domain Information Model of the blood pressure monitor is defined by IEEE 11073-10407. This shows that every blood pressure contains two numeric objects: one mandatory compound numeric object for systolic, diastolic, and mean arterial pressure (MAP), and one optional numeric object for pulse rate.

### oneM2M Service Layer

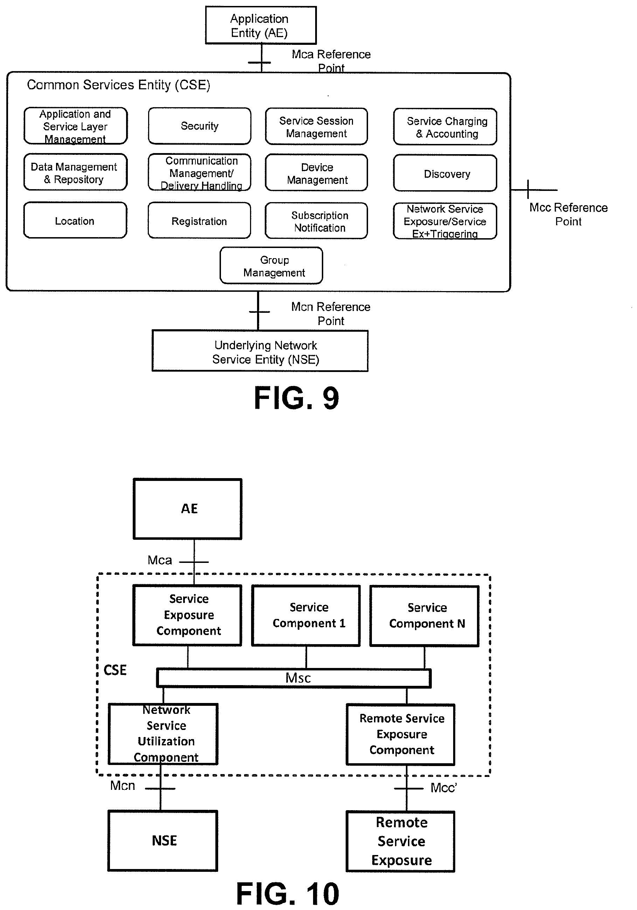

The oneM2M standard (oneM2M-TS-0001 oneM2M Functional Architecture-V-0.6.1) under development defines a Service Layer called "Common Service Entity (CSE)" as illustrated in FIG. 8. The Service Layer provides "horizontal" services that can be utilized by different 'vertical' M2M silo systems and applications, such as e-Health, fleet management, and smart homes. CSE supports four reference points, the Mca, Mcc, Mcc', and Mcn.. The Mca reference point interfaces with an Application Entity (AE). The Mcc reference point interfaces with another CSE within the same service provider domain and the Mcc' reference point interfaces with another CSE in a different service provider domain. The Mcn reference point interfaces with the underlying network service entity (NSE). An NSE provides underlying network services to the CSEs, such as device management, location services and device triggering. CSE contains multiple logical functions called "Common Service Functions (CSFs)", such as "Discovery", "Data Management & Repository". FIG. 9 illustrates the CSFs for oneM2M.

oneM2M architecture enables the application service node (ASN), application dedicated node (ADN), the middle node (MN), and the infrastructure node (IN). The ASN is a node that contains one CSE and contains at least one AE. An example of physical mapping is an ASN residing in an M2M Device. The ADN is a node that contains at least one AE and does not contain a CSE. An example of physical mapping is an ADN residing in a constrained M2M Device. An MN is a node that contains one CSE and contains zero or more AEs. An example of physical mapping for an MN is an MN residing in an M2M Gateway. The IN is a node that contains one CSE and contains zero or more AEs. An example of physical mapping for an IN is the IN residing in an M2M Service Infrastructure.

The M2M Service Architecture described in (Service Component Architecture, TS-0007 Service Component Architecture-V-0.2.0) augments the oneM2M Functional Architecture by specifying M2M Services provided to M2M Application and M2M Service Providers. The components, shown in FIG. 10, are a service exposure component, network service utilization component, and a remote service exposure component. The service exposure component exposes services to AEs. The network service utilization component consumes services from the NSE. And the remote service exposure component connects Services from different M2M environments.

In "Detecting Similarities in Ontologies with the SOQA-SimPack Toolkit", Patrick Ziegler et al., 2006, an ontology similarity tool is developed, which employs a tree structure for extracting and storing ontology classes and properties. In "SAPHIRE: A Multi-Agent System for Remote Healthcare Monitoring through Computerized Clinical Guidelines", Gokce B. Laleci et al., 2008, a software tool is provided that enables transformation of non-OWL formatted ontologies to OWL formatted ones.

### SUMMARY

Various aspects and features of the present invention are defined in the appended claims.

As discussed above, there are several existing common foundational ontologies that may be reused by the M2M system. It may not be effective if the M2M system re-defines those common foundational ontologies, which also reduces the interoperability between the M2M system to other systems without re-using the existing foundational ontologies. For example, in a use case discussed in more detail herein, in order to describe the semantics of gym member, devices deployed in the gym, the M2M system may need to have the corresponding ontologies, such as the ontology for describing a person, the ontology for describing sensors, or blood pressure monitor. Without using the existing foundational ones, such as FOAF ontology, SSN ontology, IEEE 11073 personal health data model, the M2M system may re-define those ontologies. It is possible that those ontologies may not be interoperable with the existing ones if they are re-defined, which prevents the M2M system and other systems from understanding each other.

The M2M system may also need to incorporate the ontologies from different verticals such that the ontologies defined by one vertical may be shared and used by other verticals through the M2M Service Layer. The existing M2M systems lack the support of ontology management. The issues include the following issues. A first issue, there is no high level ontology that can model the M2M system with other systems. Such ontology is needed to understand how the M2M system interacts with other systems from the perspective of ontology sharing and re-using. A second issue, there is a lack of mechanisms of how the M2M system may incorporate domain-specific ontologies from different namespaces outside of the M2M system, such that they can be stored, discovered, and re-used by the M2M system. A third issue, there is a lack of mechanisms of how the M2M system can incorporate the ontologies from different verticals inside of M2M system. A fourth issue, there is lack of mechanisms of how the ontologies that are published to the M2M system can be efficiently stored to facilitate the ontology discovery.

The scope of the invention is determined by the independent claims.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not constrained to limitations that solve any or all disadvantages noted in any part of this disclosure.

### BRIEF DESCRIPTION OF THE DRAWINGS

A more detailed understanding may be had from the following description, given by way of example in conjunction with the accompanying drawings wherein:
FIG. 1 illustrates a patient monitoring application;
FIG. 2 illustrates a semantic example;
FIG. 3 illustrates an example of owl:intersection;
FIG. 4 illustrates an example of defining a data type in XSD;
FIG. 5 illustrates an example of defining a data type in XSD;
FIG. 6 illustrates an exemplary domain information model of the weighing scales defined by IEEE 11073-10415;
FIG. 7 illustrates an exemplary domain information model of the blood pressure monitor Defined by IEEE 11073-10407;
FIG. 8 illustrates an exemplary oneM2M architecture;
FIG. 9 illustrates exemplary oneM2M Common Service Functions;
FIG. 10 illustrates an exemplary oneM2M Services Architecture;
FIG. 11 illustrates an exemplary M2M use case in a gym;
FIG. 12 illustrates an exemplary system that may implement ontology management;
FIG. 13 illustrates an exemplary M2M ontology processor;
FIG. 14 illustrates an exemplary method for an ontology processing unit;
FIG. 15 illustrates an exemplary message flow for publishing ontology messages;
FIG. 16 illustrates an exemplary ontology template in OWL;
FIG. 17 illustrates an exemplary method for OPC converting an ontology into a compatible format;
FIG. 18 illustrates an exemplary message flow of concept mapping consultation;
FIG. 19 illustrates an exemplary summary of the functionalities of the ontology classifying component;
FIG. 20 illustrates an exemplary structural architecture for an ontology repository;
FIG. 21 illustrates an exemplary hierarchy of resources stored in an ontology repository;
FIG. 22 illustrates exemplary resources stored in ontology repository for non-M2M namespace;
FIG. 23 illustrates exemplary resources stored in ontology repository for M2M namespace;
FIG. 24 illustrates an exemplary functional architecture of the ontology discovery component;
FIG. 25 illustrates an exemplary message flow for ontology discovery;
FIG. 26 illustrates an exemplary oneM2M ontology management capability service function;
FIG. 27 illustrates exemplary implementation architecture for Ontology Management in the oneM2M Service Component Architecture;
FIG. 28 illustrates an exemplary resource tree structure of an ontology repository;
FIG. 29 illustrates an exemplary message exchange used by an oneM2M implementation to publish its ontology to another outside namespace;
FIG. 30 illustrates an exemplary high-level ontology model;
FIG. 31 illustrates an exemplary oneM2M resource oriented architecture (ROA) of M2M high level ontology;
FIG. 32 illustrates an exemplary abstraction in a oneM2M namespace;
FIG. 33 illustrates an exemplary abstraction in a oneM2M namespace;
FIG. 34 illustrates an exemplary functional architectural for M2M semantic support;
FIG. 35 illustrates an ETSI M2M architecture;
FIG. 36 illustrates a M2M Architecture with semantics nodes;
FIG. 37 illustrates a M2M semantics node architecture;
FIG. 38A is a flow diagram illustrating one example of a method for establishing a semantics node hierarchy;
FIG. 38B illustrates steps in FIG. 38A in more detail;
FIG. 39 illustrates a message flow of semantics node registration;
FIG. 40 illustrates a parent-child relationship update initiated by child;
FIG. 41 illustrates a flow diagram of processing semantics related resource discovery;
FIG. 42 illustrates a message flow of RESTful semantic node operations;
FIG. 43 illustrates a message flow of the semantics related resource discovery, retrieval and validation process.
FIG. 44 illustrates a flow diagram of a semantics node that may be updated with the semantics related resources stored and managed by a sibling/parent/child semantics node;
FIG. 45 illustrates a flow diagram of grouping of resources with the same semantics;
FIG. 46 illustrates a semantics related resource pushing;
FIG. 47 illustrates a scenario in which a device moves from one area network to another one;
FIG. 48 illustrates a message flow of data/semantics related resources movement;
FIG. 49 illustrates a ETSI M2M architecture with stand-alone semantics nodes;
FIG. 50 illustrates a semantics node resource structure;
FIG. 51 illustrates a SSs resource structure;
FIG. 52 illustrates a ETSI M2M architecture with integrated semantics nodes;
FIG. 53 illustrates a xSCL resource structure;
FIG. 54 illustrates a semantics related resource structure on <scll>;
FIG. 55 illustrates a contentInstance semantics;
FIG. 56 illustrates a message flow of resource and semantics retrieval;
FIG. 57 illustrates a 3GPP MTC architecture with stand-alone semantics nodes;
FIG. 58 illustrates a 3GPP MTC architecture with stand-alone semantics nodes;
FIG. 59 provides an illustration of one example of the use of semantic nodes as described herein;
FIG. 60A is a system diagram of an example machine-to-machine (M2M) or Internet of Things (IoT) communication system in which one or more disclosed examples may be implemented;
FIG. 60B is a system diagram of an example architecture that may be used within the M2M / IoT communications system illustrated in FIG. 60A;
FIG. 60C is a system diagram of an example M2M / IoT terminal or gateway device that may be used within the communications system illustrated in FIG. 60A;
FIG. 60D is a block diagram of an example computing system in which aspects of the communication system of FIG. 60A may be embodied;
FIG. 61 is an exemplary graphical user interface for utilizing M2M ontology management and semantics interoperability;
FIG. 62 illustrates an exemplary Semantic Sensor Network Ontology;
FIG. 63 illustrates an exemplary oneM2M Base Ontology;
FIG. 64 illustrates an exemplary <ontology> resource structure;
FIG. 65 illustrates an exemplary <ontology> resource structure;
FIG. 66 illustrates an exemplary resource structure of <class>;
FIG. 67 illustrates an exemplary resource structure of <relationship>;
FIG. 68 illustrates an exemplary <Ontology> resource mapping to oneM2M Base Ontology;
FIG. 69 illustrates an exemplary <Ontology> resource structure; and
FIG. 70 illustrates an exemplary <ontology> resource that can be subscribed to via methods used by other resources.

### DETAILED DESCRIPTION OF ILLUSTRATIVE EXAMPLES

A machine-to-machine (M2M) system may include semantics nodes that house semantics related resources. Semantics related resources of the M2M system may be based on a set of proprietary ontologies. But there are many existing common foundational ontologies that may be reused by the M2M system. Relying on proprietary ontologies may affect the interoperability of M2M systems to other systems. It may be more efficient for the M2M system to use already exiting ontologies, such as Friend of a Friend (FOAF) or Semantic Sensor Network (SSN) ontologies. Within the M2M system there also may be different verticals, such as e-Health, fleet management, or smart homes. These verticals within the M2M system may have different ontologies as well. Disclosed herein is an ontology processor and other mechanisms for addressing the management and interoperability of ontologies. The ontology processor may reside on one or more semantics nodes of a M2M system, for example.

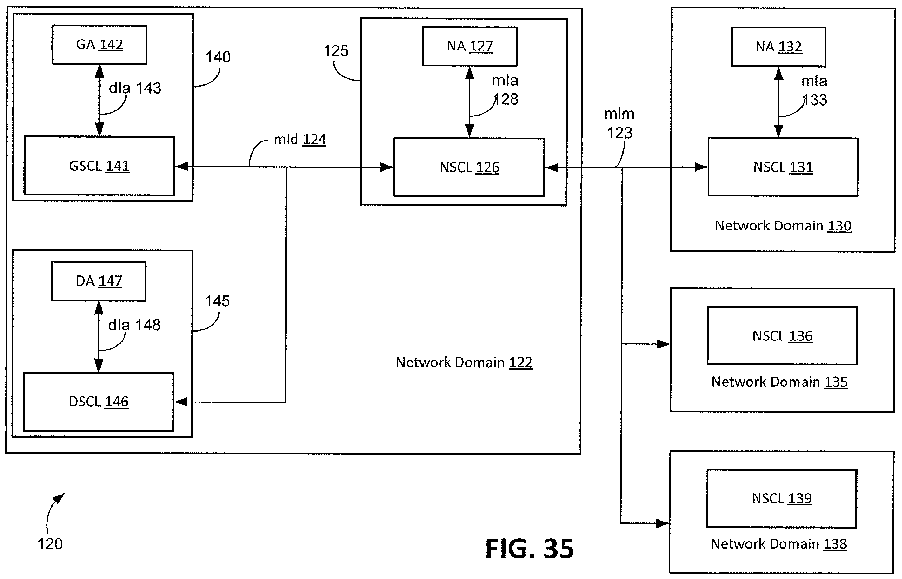

A brief summary of semantics node architecture is presented below to give context with regard to the ontology processor, which may reside with a semantics node. More details with regard to semantics node architecture are provided in the descriptions corresponding to FIG. 35-FIG. 59. These are examples in the context of ETSI, but may be considered in the context of other architectures.

In conventional machine-to-machine (M2M) systems, M2M applications (hosted on end devices as well as backend network servers) need to agree beforehand on a common definition of exchanged data. This is primarily due to a lack of semantic aware M2M service layers that are able to parse, interpret or process M2M data on behalf of applications. In current M2M systems, M2M service layers lack semantic awareness capabilities and hence data flowing through or stored within M2M service layers is treated as opaque information.

This lack of semantic awareness prevents M2M service layers from offering services which allow data produced by M2M applications to be effectively abstracted or virtualized by the M2M service layer such that it can be discovered, accessed, interpreted, and shared by different applications even if they do not have any prior knowledge of the application from which the data originated. As a result, the physical entities that are sensed and acted upon (e.g., appliances, people, cars, rooms of a building, etc.) may not be effectively virtualized/abstracted by M2M service layers and the physical entities are treated as generic entities, intrinsic to the environment, and not tied to a specific M2M application. In order to overcome this limitation, the data transmitted in M2M systems may be associated and integrated with semantic information, such that semantic aware M2M service layers can have the same knowledge of the data as M2M applications. In doing so, M2M service layers can better facilitate the sharing of data across applications and provide value-added semantic aware services to M2M applications (e.g., data aggregation, data sharing amongst different applications, etc.).

For example, in a patient monitoring application illustrated in FIG. 1, there could be separate applications hosted on each of the wireless sensor devices that monitor a patient's vital signs (e.g., blood pressure, temperature, oxygen, heart rate, etc.). Likewise, there could be separate applications hosted in the network that could make use of this information (e.g., applications associated with a patient's doctor, personal trainer, family members, ambulance paramedics, etc.). However, without M2M semantic aware services data from each of the wireless sensor devices, network applications may have difficulty discovering, sharing, and understanding the information from device applications unless the network applications have prior knowledge of the applications hosted on the wireless sensor devices and the type of information they produce (e.g., the location/address, units of the data, context of the data, etc.).

Semantics nodes may provide the following functionalities in an M2M system to support M2M service layer semantic awareness and data abstraction: (i) support for storing semantics related resources, and/or support for interfaces to servers storing semantics related resources; (ii) support for mechanisms for creating, retrieving, updating, and deleting semantics related resources; and (iii) capabilities to publish and discover semantics related resources.

As described herein, a semantics node is a logical entity that may be hosted on a stand-alone computing device (e.g., server) in the network or hosted on an existing entity within the network, such as an M2M gateway, M2M device, M2M server, or the like. A semantics node may be seen as a repository that stores semantics-related resources modeling. For example, a sensor device for blood pressure may want to understand how to describe its data, so it queries a nearby semantics node to find out if there is a blood pressure class already defined. If so, the semantics node responds to the sensor device with the blood pressure class it found locally. If not, the semantics node may query other semantics nodes (e.g., siblings or parents). The use of a semantics node may reduce the need to have end devices store models of data.

A semantics node stores and manages semantics related resources. Semantics related resources usually describe other resources, such as, for example, the ETSI M2M resources that are stored under the resource tree, <SCL>, <application>, <container>, <contentInstance> , which need to have semantics related resources associated with them in order to enable an understanding of their semantics. In one example, semantics related resources may have one of three types: class, relationship, and term. This categorization provides compatibility with current techniques of the semantics web and enables an M2M system to leverage existing semantics related resources. There is a mix of ETSI and oneM2M based examples herein. One skilled in the art will be able to understand that ETSI and oneM2M have similar resource tree structures and should be able to relay the concepts herein in either one.

As discussed herein, semantics nodes may be deployed in an M2M system in different levels, such as a M2M area network, a M2M access network, and a M2M core network, such that the different levels form into a hierarchical structure. Semantics nodes in the same level may be distributed and have a sibling relationship. Mechanisms are disclosed with regard to building and maintaining such a hybrid architecture of semantics nodes, which provides the benefits of different levels of abstraction and compatibility to an existing network hierarchy.

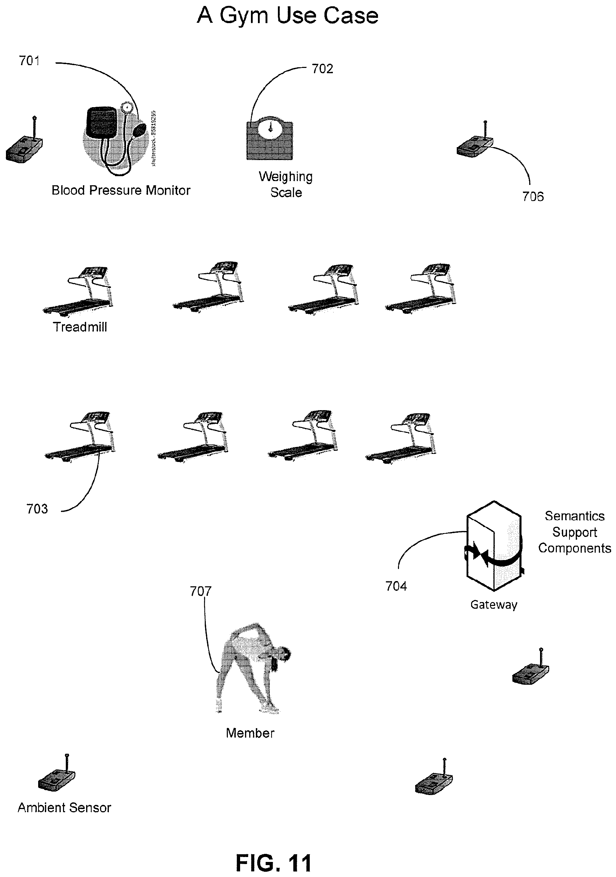

FIG. 11 - FIG. 34 and their accompanying description provide further information and understanding of examples of a semantics node architecture and platforms in connection with the methods, devices, and systems for ontology management described below. Disclosed herein is an M2M Ontology Processor (MOP), which is in charge of processing, storing, or providing discovery function to ontologies either published from outside or inside of the M2M domain.

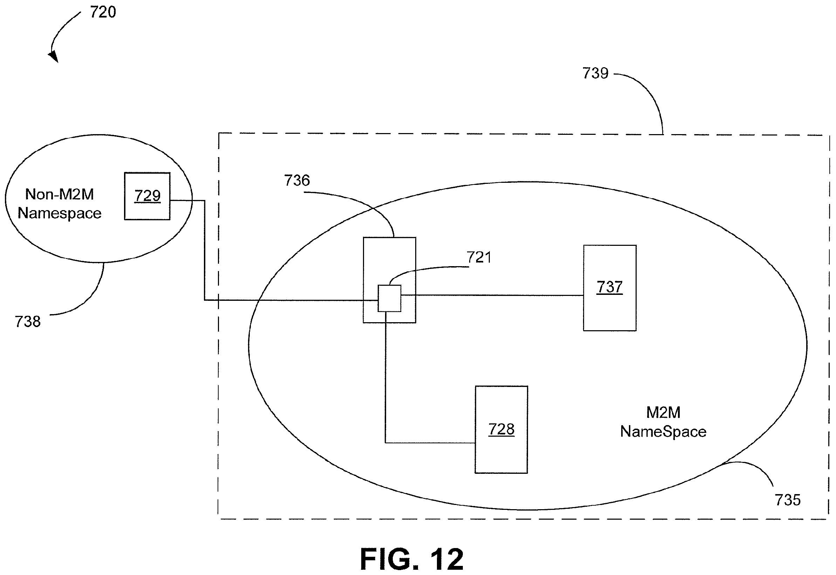

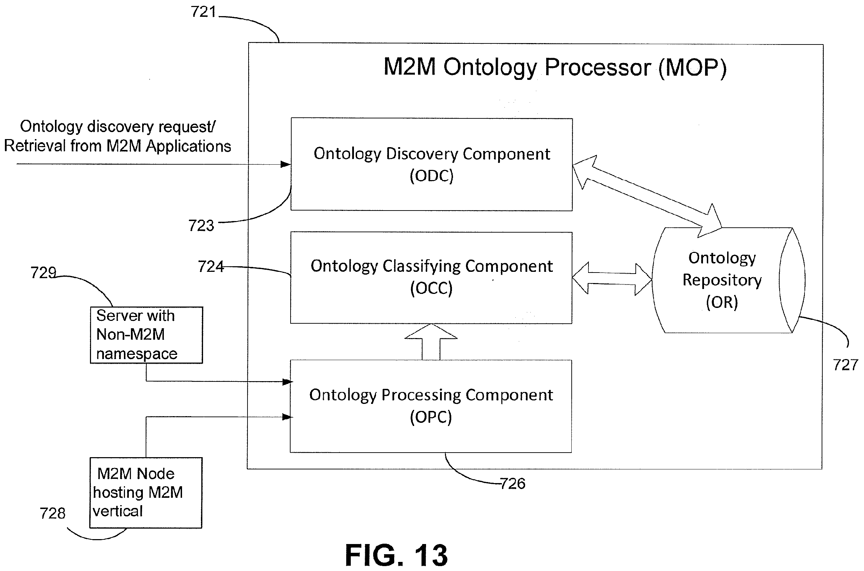

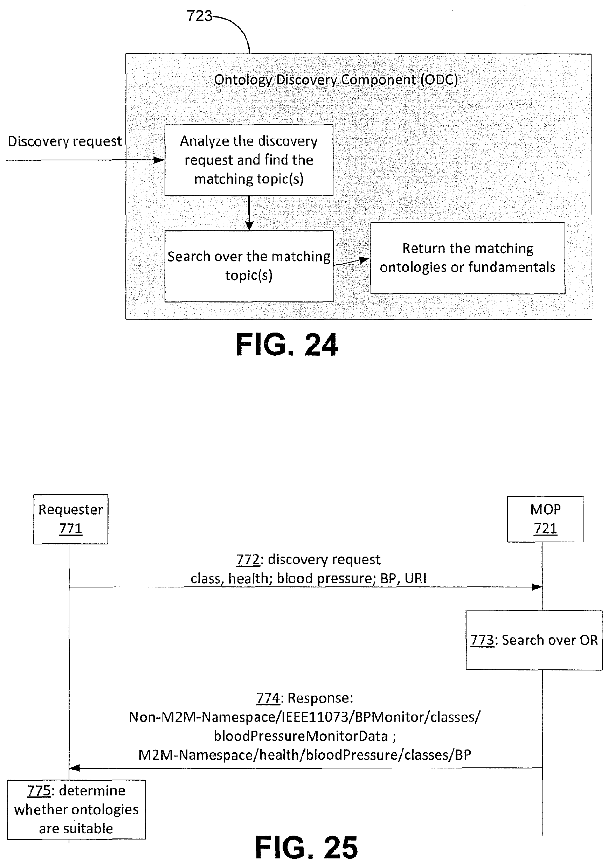

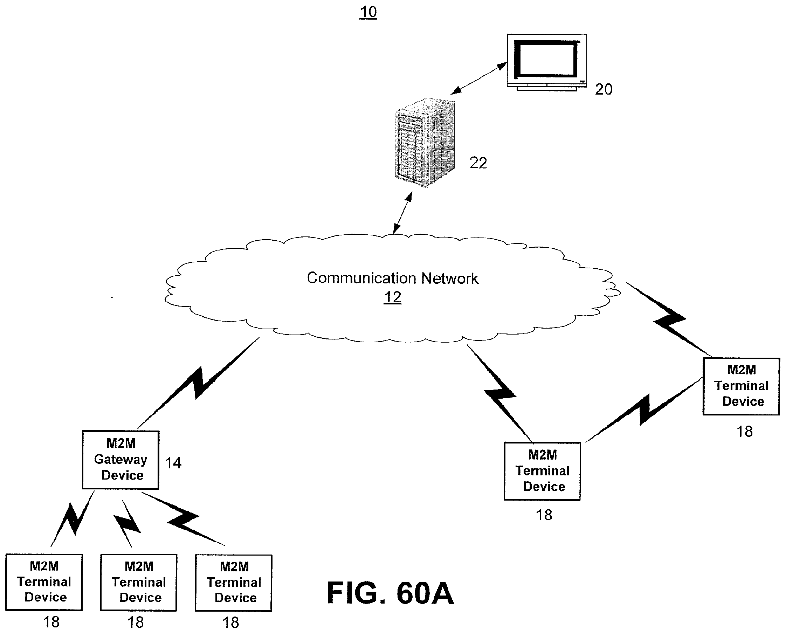

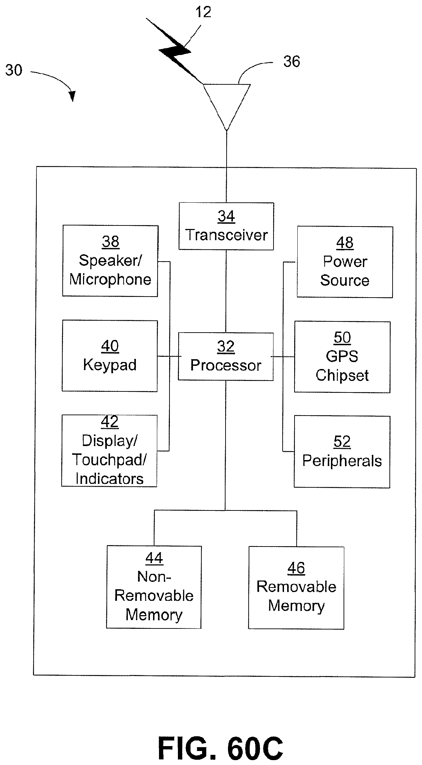

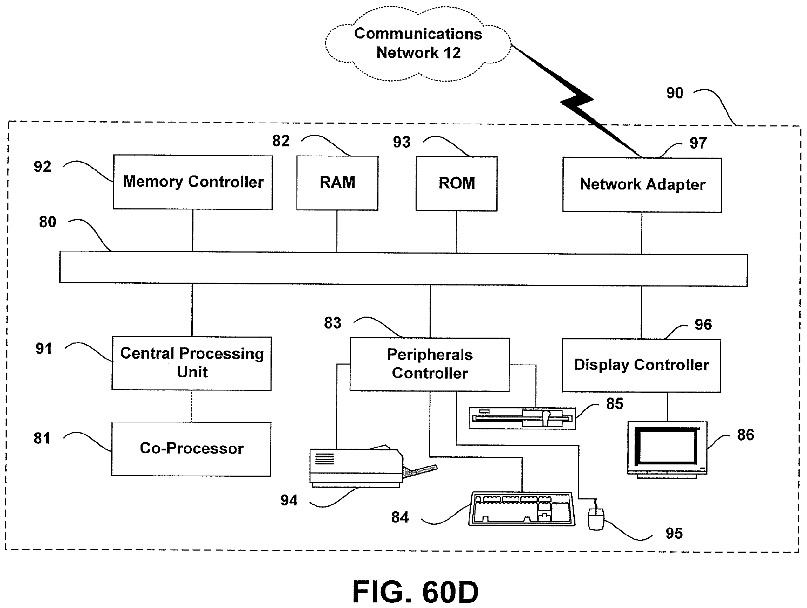

It is understood that the entities performing the steps illustrated herein (e.g., FIG. 12 - FIG. 34, etc.) are logical entities that may be implemented in the form of software (e.g., computer-executable instructions) stored in a memory of, and executing on a processor of, a device, server, or computer system such as those illustrated in FIG. 60C or FIG. 60D. That is, the method(s) illustrated in FIG. 12 - FIG. 34, etc. may be implemented in the form of software (e.g., computer-executable instructions) stored in a memory of a computing device, such as the device or computer system illustrated in FIG. 60C or FIG. 60D, which computer executable instructions, when executed by a processor of the computing device, perform the steps illustrated in FIG. 12 - FIG. 34, etc.. In an example, with further detail below with regard to the interaction of M2M devices, requester 771 of FIG. 25 may reside on M2M terminal device 18 of FIG. 60A, while MOP 721 or server 729 of FIG. 13 may reside on M2M gateway device 14 of FIG. 60A.

For additional perspective, FIG. 11 illustrates a gym use case with regard to ontology management. As shown in FIG. 11, a gym may have a plurality of different devices deployed for their customers, such as an ambient sensor 706 for sensing the surrounding temperature/humidity, a treadmill 703, weighing scale 702, and blood pressure monitor 701. The resources that are relevant to the devices (e.g., application resource) and the data resources that are produced and reported by the devices may be stored in a resource database.

In order to provide the semantics to the devices of FIG. 11 and their measured data, as well as the gym members, various ontologies are needed. Each ontology may be used for describing semantics information for each of the different types of devices, as well as the measured data. The MOP may reside in gateway 704. There are external ontologies that may be published to the MOP that may be leveraged and used to describe the semantics of the entities in the gym. For example, FOAF, SSN, and IEEE 11073-10415 may be published to the M2M namespace by a publishing message, as discussed in more detail with regard to Table 3 through Table 6. As a result, the MOP may process, store, discover, and otherwise assist in the use of the external ontologies.

With continued reference to FIG. 11, the MOP may store the published ontologies in a unified language that may be adopted by the M2M system. The language may be RDFS/OWL from the semantics web. For a simplified illustration of the examples, we assume the M2M system uses RDFS/OWL in describing the published ontologies. The M2M system may also adopt other languages or develop its own language. The proposed M2M high level ontology model as well as the functionalities of the MOP are standalone and do not depend on the specific ontology language used by the M2M system. In most cases, the M2M system represents knowledge as a hierarchy of concepts (ontologies), which may be external of the M2M domain or internal of M2M domain, and use a shared vocabulary to denote the classes, properties and interrelationships of those concepts. This will be discussed in more detail below.

FIG. 12 illustrates an exemplary system 720 that may implement ontology management. There may be a plurality of namespaces in system 720, such as M2M namespace 735 and non-M2M namespace 738. Non-M2M namespace 738 includes one or more ontologies that may be located on server 729. As shown in FIG. 12, M2M namespace 735 (e.g., oneM2M namespace) includes multiple M2M nodes that may be semantics nodes, such as a node 737, a node 728, and a node 736. Node 728 and Node 737 may be communicatively connected with node 736, wherein node 736 includes a MOP 721. Node 728 may include an M2M vertical that include e-Health. Node 737 may include an M2M vertical that includes smart homes. Node 737 and Node 728 may have a plurality of other M2M verticals as well. Node 736 may also be communicatively connected with server 729, which is located in non-M2M namespace 738.

FIG. 13 illustrates an exemplary of the MOP 721. MOP 721 may functionally consist of an ontology processing component (OPC) 726, an ontology classifying component (OCC) 724, an ontology discovery component (ODC) 723, and an ontology repository (OR) 727. OPC 726 may process published ontologies external and internal of M2M namespace, which may be used to provide semantics information to objects/entities. The processed ontologies are input to the OCC 724 to be properly stored in the right hierarchy for discovery and usage. OCC 724 classifies the ontologies and store them in the right hierarchy/location in the OR for discovery and usage. ODC 723 provides the ontology discovery and determine the matching one(s) for a requester to use. OR 727 may store any ontology models that are published or generated external and internal to the M2M domain. Those ontologies may be used for resources to enable semantics. Thus, the OR 727 may interact with both OCC 724 and ODC 723. The OR may be considered part of the logical entity semantics node. The MOP 721 functionalities may also be applicable to other namespaces/systems other than M2M. For example, W3C may also adopt the MOP functionalities to process, classify and store the ontologies published from the M2M namespace.

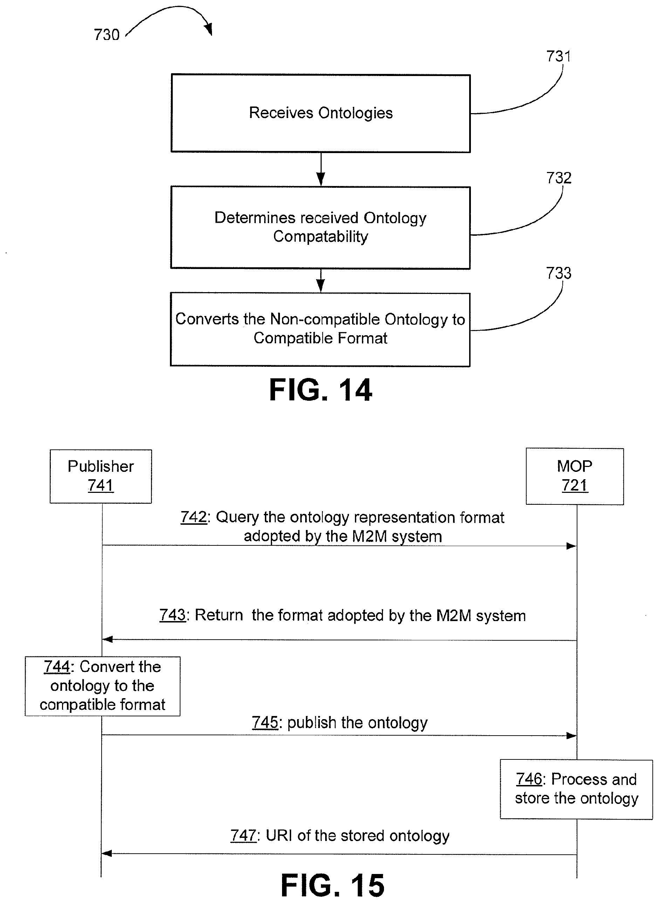

FIG. 14 illustrates an exemplary method 730 with regard to OPC 726 of MOP 721. At step 731, the OPC receives ontologies. The received ontology message (i.e., ontology publishing message) may have a particular format when published, which is discussed in more detail below. The ontologies may be from outside namespaces (non-M2M namespace 738) or an inside namespace (e.g., M2M namespace 735). At step 732, OPC 726 determines the compatibility of the received ontology. OPC 726 identifies if the ontology representation has the same format as what is used in the M2M namespace. If all the ontologies inside the M2M namespace are represented by OWL, those ontologies represented in other formats are considered to be not compatible within the M2M namespace. At step 733, OPC 726 converts a non-compatible ontology to a compatible format.

The ontology publishing message may include one or more fields as summarized in Table 1, such as a namespace, name, compatibility, access_right, topic, relevance, or ontology representation. The fields of Table 1 may be used for messages associated with external namespaces (e.g., non-M2M namespace 738) or associated with M2M verticals (e.g., M2M verticals e-health and smart home on node 728 and node 737) that have their ontologies published to OPC 726. The published ontologies may be an update of previously published ontologies to OPC 726.

With reference to Table 1, the namespace field indicates the namespace of the published ontology. The name field indicates the name of the published ontology. The compatibility field indicates whether the ontology representation contained in the ontology publishing message body is compatible with the ontology representation chosen by an M2M system 735 having an M2M namespace (e.g. M2M namespace 735). To simplify examples discussed herein with regard to ontology management, it will be assumed that the M2M system 721 is equivalently encompassed by the M2M namespace 735, as shown in FIG. 12. It is possible the M2M system 739 supports more than one compatible format. This field may be left blank, if the publisher (server 729) did not check the compatibility of its ontology representation beforehand with MOP 721. Otherwise, the server 729 may fill this field with the proper content, as discussed in more detail herein.

**Table 1: Ontology Publishing Message Field Description**

| **Message Field** | **Description** |
|---|---|
| names pace | It indicates the namespace of the published ontology. |
| name | It indicates the name of the published ontology. |
| compatibility | It indicates whether the ontology representation contained in the message body is compatible with the one chosen by the M2M system. |
| accessRight | It indicates how the ontology can be accessed by other parties. |
| topic | It designates the topic the ontology is relevant to. |
| relevance | It indicates whether the published ontology has any |
| | relevance to other ontologies. |
| ontology representation | It contains the representation of the published ontology, e.g., RDF/XML. |

With continued reference to Table 1, the access right indicates how the ontology may be accessed by other parties and entities. The access right may be different for different parties (e.g., individuals, businesses, governments, or other parties) with different relationships with the ontology publisher. The accessRight field may look like Table 2. The sub-field operation shows the permitted operation, such as RO (read only), RW (read and write), etc. The sub-field party shows the corresponding party/parties that are allowed to carry out the operation on the published ontology, which could be public (e.g., every party can have the operation on it), the parties with certain relationship with the publisher, individual parties, etc. Table 2 shows a few examples, every public party is able to have the read access to the published ontology, while only the applications with the same owner as the publisher (the publisher could be another application) can have the read and write access to the published ontology.

**Table 2: Access Right of Published Ontology**

| **operation** | **party** |
|---|---|
| RO (read only) | public |
| RW (read and write) | the applications controlled by the same owner which published the ontology |
| ... | ... |

With further reference to Table 1, topic designates the topic the ontology is relevant to. The topic field may contain one or more key words to describe the ontology. For example, when the FOAF ontology is published, the topic filed could be "person." The topic may come from a well-known/commonly-used vocabulary that is understood by all namespaces and may be standardized as well. This field may also be left blank, thus OCC 724 may decide the relevant topic for the published ontology, which is discussed in more detail below. Relevance, as shown in Table 1, indicates whether the published ontology has any relevance to other ontologies. For example, the published ontology may be extended or revised from the existing ontologies stored in OR 727. For example, FoAF ontology may be extended to include more attributes of a person other than those that are already defined. Ontology representation, as shown in Table 1, contains the representation of the published ontology. In some instances, the publisher may only publish the URI of the ontology. If ontology representation is in the non-M2M-compliant format, the schema of the ontology may be included.

As briefly discussed above, ontologies may be from outside namespaces (non-M2M namespaces) or an inside namespace (e.g., oneM2M namespace). With reference to FIG. 13, and FIG. 12 in conjunction with step 731 of FIG. 14, OPC 726 may receive ontologies from one or more servers that may host one or more non-M2M namespaces, such as server 729. Server 729 may proactively publish ontologies of a non-M2M namespace 738 to MOP 721. MOP 721 may also request updated non-M2M namespace ontologies from server 729. The request from MOP 721 may be based on an event, such as time, a user action (e.g., user entered command), among other things. Server 729 or the namespaces thereon may be presided over by one or more authorities, such as FOAF, SSN, IEEE, a university, a business, a private individual, a government, or other entity.

With continued reference to FIG. 13 and FIG. 12 in conjunction with step 731 of FIG. 14, OPC 726 may also receive ontologies from one or more M2M nodes, such as node 728 and node 737, that have one or more verticals associated with the M2M namespace 735 of MOP 721. Examples of verticals include e-Health (node 728), fleet management, and smart homes (node 737), among other things. Node 728 and node 737 may proactively publish ontologies to MOP 721. MOP 721 may also request updated M2M vertical ontologies from node 728 and node 737. The request may be based on an event, such as time, a user action (e.g., user entered command), among other things. Node 728, node 737, or the hosted verticals of node 728 and node 737 may be presided over by one more authorities, such as FOAF, SSN, IEEE, a university, a business, a private individual, a government, or other entity.

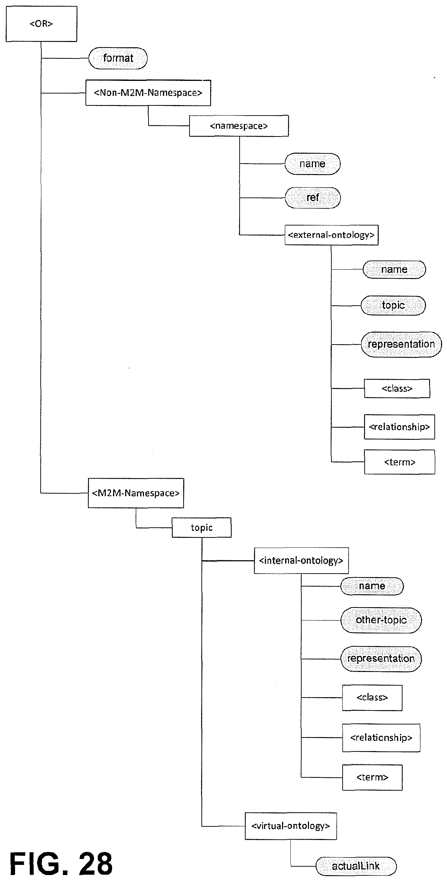

FIG. 15 illustrates an exemplary message flow for publishing ontology messages. The message flow of FIG. 15, may be applicable to an external namespace (e.g., non-M2M namespace 738) or an internal namespace (e.g., M2M namespace 735). For simplicity, publisher 741, as shown in FIG. 15, will represent an external or an internal namespace. At step 742, publisher 741 may query the ontology representation format adopted by M2M system 739. A prerequisite may be that the publisher understands the schema used by the M2M System (e.g., RDFS/OWL) to represent its original ontology in the ontology repository of M2M System 739. For example, in oneM2M (discussed in more detail herein), the query may be targeting to retrieve the format attribute of <OR> resource in FIG. 28. Alternatively, for step 742, optionally publisher 741 can choose to check the compatibility of the representation of the ontology it wants to publish with MOP 721. This message may be a separate message from the ontology publishing message. Publisher 741 may input the format it uses in representing the ontology in a message. MOP may determine if the received format is the one it is adopting. And a corresponding response message (yes or no) is returned to publisher 741. The format M2M system 739 uses may be attached in the response message to let the publisher know about this

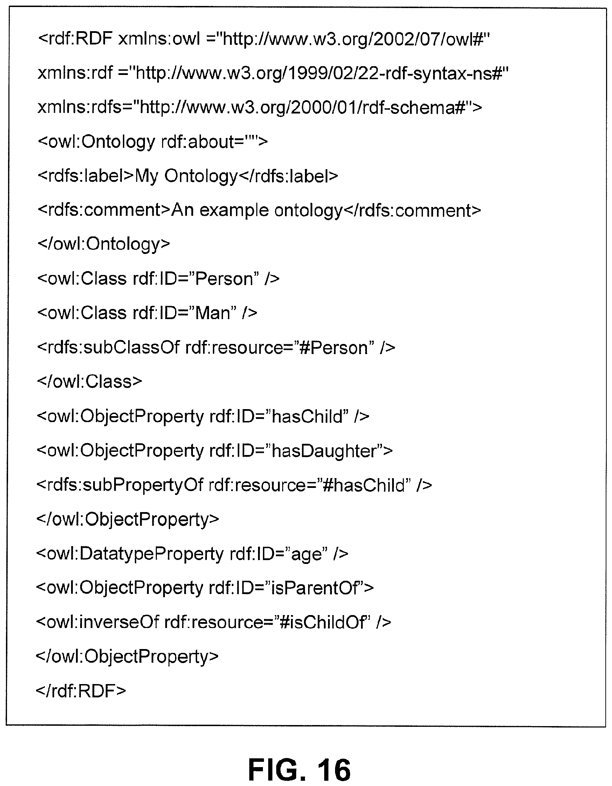

With continued reference to FIG. 15, at step 743 based on the request sent information, from the publisher at step 742, MOP may return the compatibility result or the format (which may be an ontology template) adopted by M2M system 739. The ontology template may be an ontology as shown in FIG. 16, which is relatively simple. At step 744, publisher 741 may choose to convert the ontology that is going to be published to the compatible format based on the feedback from MOP 721. A prerequisite may be that publisher 741 understands the RDFS/OWL schema to represent its original ontology in RDFS/OWL. MOP 721 processes the ontology and convert the ontology to the format that is adopted by M2M system 739. Optionally, publisher 741 may also publish the original ontology in its own format directly. The conversion by MOP 721 is based on the concept name mapping table as shown in Table 8, as discussed in more detail herein. FIG. 15 shows the scenario that publisher 741 converts the ontology to the compatible format.

With further reference to FIG. 15, at step 744, publisher 741 creates a publishing message, which may contain the fields of Table 1. For illustration purposes Table 3 through Table 7 show what may be contained in the fields of different ontologies. **Table 3: Publishing Message of FOAF Ontology**

| namespace | name | compatibility | Access Right | topic | ontology representation |
|---|---|---|---|---|---|
| FOAF_Project: http://xmlns.com/f oaf /0.1/ | FOAF | Y | Public | person | http://xmlns.com/ foaf /spec/index.rdf |

**Table 4: Publishing Message of SSN Ontology**

| namespace | name | compatibility | Access Right | topic | ontology representation |
|---|---|---|---|---|---|
| SSN_Project:http://purl.oc lc.org/NET/ssnx/SSN_Pro ject | SSN | Y | Public | sensor | www.w3.org./2005/Incu bator/ssn/ssnx/ssn.owl |

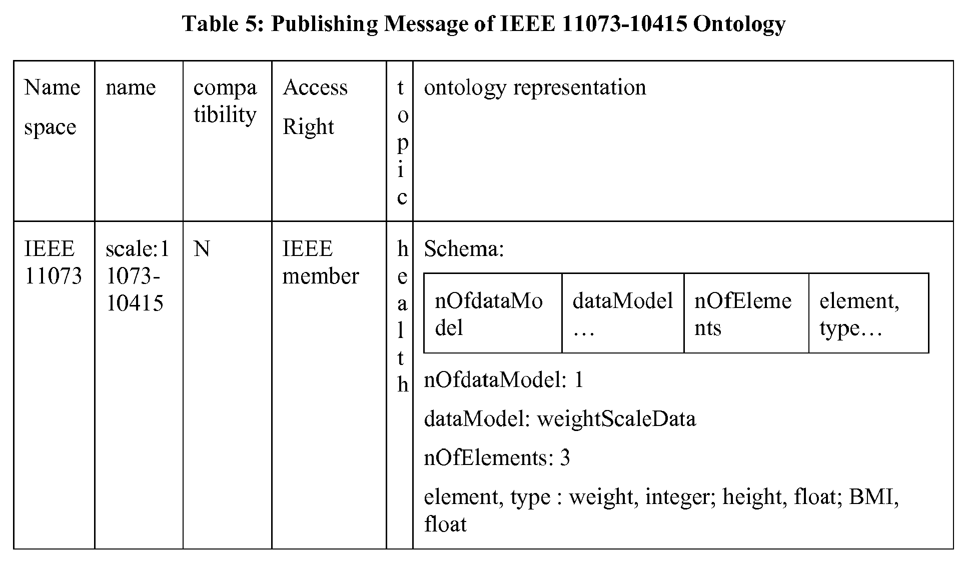

**Table 6: Publishing Message of IEEE 11073-10407 Ontology**

| names pace | name | comp atibili ty | Access Right | topic | ontology representation |
|---|---|---|---|---|---|
| IEEE 11073 | BPMonitor: 11 073-10407 | Y | IEEE member | health | xmlns:xsd ="http://www.w3.org/2001/XLMSchem a#" <xsd:complexType |
| | | | | | name="bloodPressureMonitorData"> |
| | | | | | <xsd:sequence> |
| | | | | | <xsd:element name="systolic " |
| | | | | | type="integer"/> |
| | | | | | <xsd:element name="diastolic" |
| | | | | | type="integer"/> |
| | | | | | <xsd:element name="MAP" |
| | | | | | type="integer"/> |
| | | | | | <xsd:element name="pulse" |
| | | | | | type="integer"/> |
| | | | | | </xsd: sequence> |
| | | | | | </xsd:complexType> |

**Table 7: Publishing Message of BP from a M2M Vertical with M2M Namespace**

| Name space | name | compatibility | Access Right | topi c | ontology representation |
|---|---|---|---|---|---|
| M2M | bloodPre ssure | Y | Public | | xmlns:xsd |
| | | | | | ="http://www.w3.org/2001/XLMSche ma#" |
| | | | | | <xsd:complexType name="BP"> |
| | | | | | <xsd: sequence> |
| | | | | | <xsd:element name="systolic " |
| | | | | | type="integer"/> |
| | | | | | <xsd:element name="diastolic" |
| | | | | | type="integer"/> |
| | | | | | </xsd: sequence> |
| | | | | | </xsd:complexType> |

Table 3 shows a publishing message, not falling within the scope of the claims, that may be from an FOAF_Project namespace with an FOAF ontology. The namespace field is set to "FOAF_Project: http://xmlns.com/foaf/0.1/ ". The name field is set to FOAF. FOAF ontology is also written in RDF/OWL, thus the compatibility field is set to Y. The accessRight is set to be Public (FOAF is open to the public). The topic field is set to be "person." The ontology representation's URL is contained in the message body, from which MOP 721 is able to download and obtain the ontology.

Table 4 shows a publishing message, not falling within the scope of the claims, from a SSN_Project namespace with an SSN ontology. The namespace field is set to "SSN_Project: http://purl.oclc.org/NET/ssnx/ssn." The name field is to SSN. SSN ontology is also written in RDF/OWL, thus the compatibility field is set to Y. The accessRight is set to be Public (SSN is open to the public). The topic field is set to be "sensor". The ontology representation's URL is contained in the message body, from which MOP 721 is able to download and obtain the ontology.

Table 5 shows the publishing message from the IEEE 11073 namespace of the weight scale's data ontology. The namespace field is set to IEEE 11073. The name is set to "scale: IEEE 11073-10415". The publisher publishes the ontology in a schema that is different from RDF/OWL. Thus the compatibility field is set to N. OPC 726 needs to take care of converting the ontology to the compatible format. The resulted ontology is discussed in more detail below with regard to Table 9. The accessRight is set to IEEE member (only IEEE members are able to access the specification of IEEE 11073-10415). Later if a requester wants to access this ontology, MOP 721 needs to check with an IEEE endpoint or application to validate the membership of the requester. The topic field is set to be "health". The ontology representation is contained in the message body, which contains the schema of the ontology followed by value of each field.

Table 6 shows a publishing message, not falling within the scope of the claims, from the IEEE 11073 namespace of the blood pressure monitor's data ontology. The namespace field is set to IEEE 11073. The name is set to "BPMonitor: IEEE 11073-10407". The publisher publishes the ontology in the compatible format that the M2M system 739 adopts. Thus the compatibility field is set to Y. The accessRight is set to IEEE member (only IEEE members are able to access the specification of IEEE 11073-10407). The topic field is set to be "health". The ontology representation is contained in the message body, which follows the patterns used in XSD to describe the simple and complex data types.

Table 7 shows a publishing message, not falling within the scope of the claims, from an M2M vertical (e.g., eHealth) of the blood pressure (BP) ontology. The namespace field is set to M2M. The compatibility field is set to Y, because the vertical follows the M2M compliant format in representing the ontology. The topic field is left blank. The ontology representation is contained in the message body, which follows the patterns used in XSD to describe the simple and complex data types.

With further reference to FIG. 15, at step 746, OPC 726 of MOP 721 processes the published ontology and the OCC 724 stores the ontology at right hierarchy in OR 727, which is discussed in more detail below. At step 747, after successful processing and storing the published ontology at step 746, MOP 721 may return the URI of stored ontology to publisher 741.

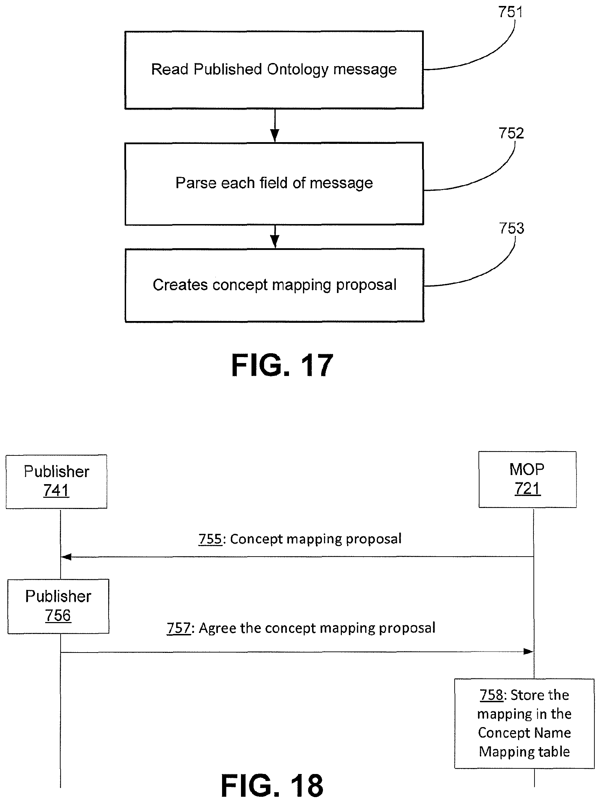

As discussed briefly above, for the ontology that is represented in a format not compatible with the M2M domain (e.g., Table 5), OPC 726 converts the ontology to the compatible format. If all the ontologies inside the M2M namespace are represented by OWL, those ontologies represented in other formats are considered to be not compatible within the M2M namespace. FIG. 17 illustrates an exemplary method for OPC 726 converting an ontology into a compatible format. At step 751, OPC 726 reads the message body of the ontology publishing message, which contains the schema of the ontology followed by value of each field. At step 752, OPC 726, based on the schema, OPC is able to parse each field. An ontology is a predefined, reserved vocabulary of terms to define classes and the relationships between which may be for a specific area of interest. Each ontology, from outside or inside of M2M namespace 735, usually is composed of two concepts: class and relationship. OPC 726 maintains possible mapping between the concept names that may be used by M2M namespace 735 and other namespaces. In RDF/OWL, the corresponding concept names are class and property, as well as xsd:complextype/xsd:simpletype and xsd:element. At step 752, OPC 726 creates a concept mapping proposal. Table 8 shows an exemplary mapping between concept names. This mapping table may be maintained by MOP 721 incrementally and dynamically, which means new mapping may be learnt and added by MOP 721. When MOP 721 encounters a new concept name that it is not able to find the corresponding mapping, hence not able to understand the published ontology, it may propose the possible mapping and consult with the publisher. The publisher can either confirm the mapping or proposes a new one. FIG. 18 illustrates the message flow of concept mapping consultation. **Table 8: Concept Name Mapping**

| **M2M Namespace** | **Other Namespace** |
|---|---|
| xsd:complexType | dataModel |
| xsd:element | element |
| property | relationship, attribute |

With reference to FIG. 18, at step 755, MOP 721 sends the concept mapping proposal to the ontology publisher 741, which contains the proposed mapping, as well as the meaning of the concept name from the M2M namespace 735. Note that an M2M namespace may also be called an M2M domain. At step 756, publisher 741 compares the meaning of the received concept names with its own name, and decides the concept mapping matches. At step 757, publisher 741 sends to MOP 721 a confirmation response of agreeing to the concept mapping proposal. At step 758, MOP 721 stores the mapping in a Concept Name Mapping table locally, for example similar to Table 8. Table 8 shows a new concept name mapping (underlined) is added to the table. Based on the mapping, OPC converts the published ontology to the compatible format. For example, Table 5 shows the published ontology in a different format. OPC maps the dataModel to xsd:complexType, element maps to xsd:element. As a result, the published ontology is converted to the representation as shown in Table 9.

**Table 9: Converted Ontology Representation of 11073-10415**

| |
|---|
| xmlns:xsd ="http://www.w3.org/2001/XLMSchema#" |
| <xsd:complexType name="weightScaleData"> |
| <xsd:sequence> |
| <xsd:element name="weight" type="integer"/> |
| <xsd:element name="height" type="float"/> |
| <xsd:element name="BMI" type="float"/> |
| </xsd:sequence> |
| </xsd:complexType> |

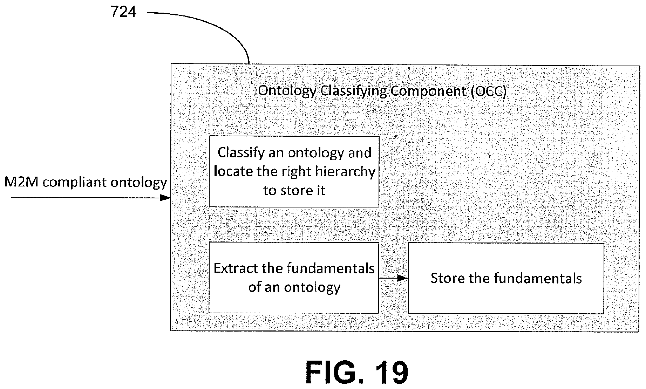

FIG. 19 illustrates a summary of the functionalities of the ontology classifying component. OCC 724 accepts the M2M compliant ontologies which are the output of OPC 726

OCC 724 classifies the ontology and locates the right hierarchy to store it. The classification includes deciding whether the ontology belongs to a non-M2M namespace (e.g. non-M2M namespace 738) or M2M namespace (e.g., M2M namespace 735), which namespace it belongs to, which topic it belongs to, etc. OCC 724 interprets the published ontology and extracts the fundamentals of the ontology. The fundamentals include the classes and properties that are contained in the ontology. Each of the classes and properties can be incorporated into the M2M system, which will be stored in the Ontology Repository (OR) 727.

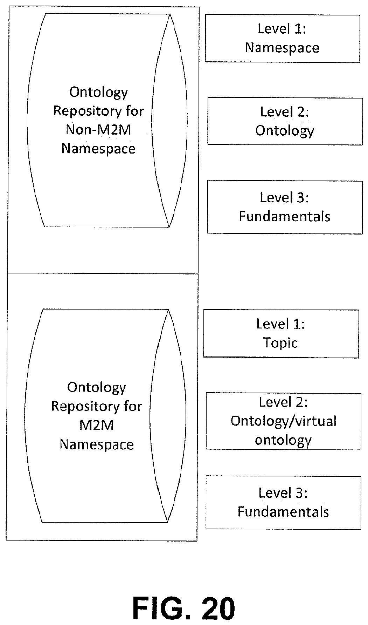

OR 727 stores the ontologies in a structure that may simplify discovery. An exemplary structural architecture is shown in FIG. 20. OR 727 differentiates the ontologies published from outside and inside of M2M namespace 735, thus it is logically composed of two parts: non-M2M namespace and M2M namespace. OR 727 for non-M2M namespace architecturally has three levels: the first level is individual namespace, which could be FOAF_Project, SSN_Project, IEEE 11073, as in the examples above. The second level contains the ontologies under each individual namespace. The third level contains the fundamentals of each individual ontology, which include the classes, relationships and terms defined in the ontology.

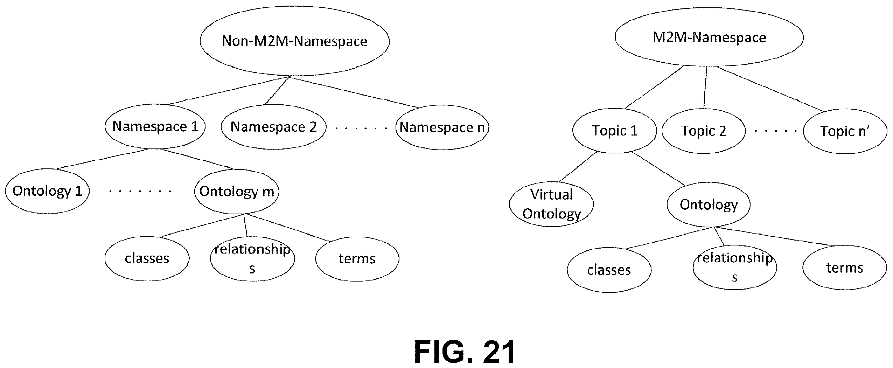

With continued reference to FIG. 20, OR 727 for the M2M namespace architecturally has three levels. The first level is individual topic, which is the possible key word that the published ontologies may be relevant to, e.g. person (which contains ontologies relevant to describing a person), appliance (which contains ontologies relevant to describing any kind of appliances). The second level contains the ontologies or virtual ontologies relevant to each individual topic. The virtual ontologies are those ontologies relevant to the topic published from non-M2M namespaces. The actual ontology representation is not stored under the virtual ontology, instead it has a link to the actual ontology stored in OR 727 for non-M2M ontologies hierarchy. The third level contains the fundamentals of each individual ontology, which include the classes, relationships and terms defined in the ontology. The detailed hierarchy of resources stored in OR 727 is shown in FIG. 21.

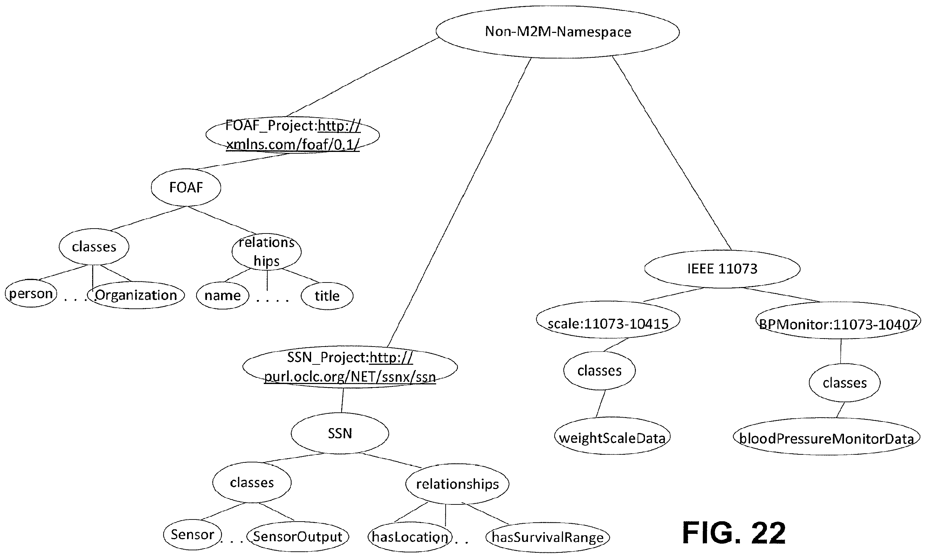

The following discussion uses one or more of the examples of the published ontologies described with regard to Tables 3 - Table 7, to discuss the processing of OCC 724, storing in OR 727, and the corresponding content in each level of OR 727. FIG. 22 illustrates the exemplary resources stored in OR 727 for non-M2M namespace 738. For the ontology published in the message shown in Table 3, the ontology in rdf/xml file was downloaded by OPC 726 from the link contained in the message body. OCC 724 stores the ontology under a new namespace named FOAF_Project. OCC 724 also extracts the classes and properties defined in the ontology and stores them under classes and relationships sub-level correspondingly. In the meantime, OCC 724 creates a new topic, person, and stores the link of the actual ontology as one of the virtual ontologies under this topic.

With continued reference to FIG. 22, for the ontology published in the message shown in Table 4, the ontology in OWL file was downloaded by OPC 726 from the link contained in the message body. OCC 724 stores the ontology under the namespace named SSN_Project. OCC 724 also extracts the classes and properties defined in the ontology and stores them under classes and relationships sub-level correspondingly. In the meantime, OCC 724 creates a new topic, sensor, and stores the link of the actual ontology as one of the virtual ontologies under this topic.

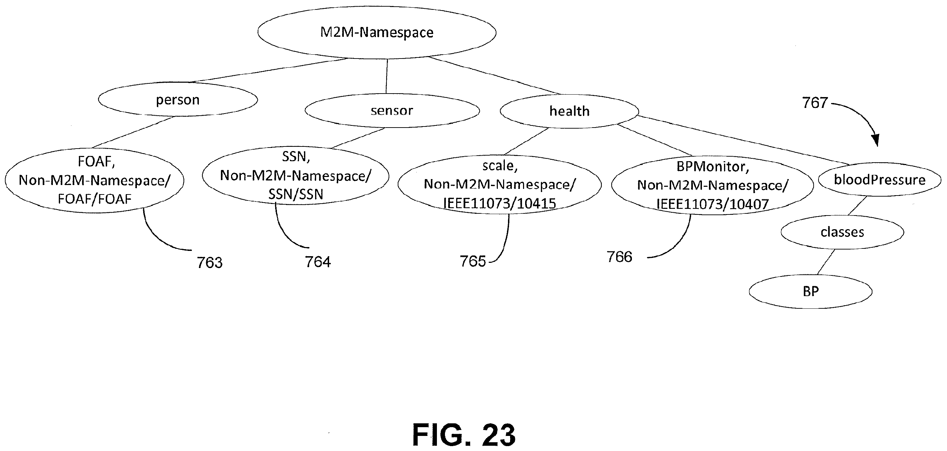

With continued reference to FIG. 22, for the ontologies published in the message shown in Table 5 and Table 9, OCC 724 stores the ontologies under a new namespace named IEEE 11073. OCC 724 also extracts the class defined in the ontology and stores them under classes sub-level. In the meantime, OCC 724 creates a new topic health, and stores the link of the actual ontologies as the virtual ontologies under this topic. Note that FIG. 23 has been drawn differently than FIG. 21 because of space considerations.

With reference to FIG. 23, for the ontology published in the message shown in Table 7, OCC 724 stores the ontologies under the M2M namespace. Since in topic field in the publishing message was left blank, OCC 724 decides the relevant topic for this ontology, which is the existing one - health. Note, the ontology may be relevant to more than one topic, the ontology representation may be stored once, and its link may be stored under the corresponding virtual ontology in other different topics. The ontologies referenced in oval 763, oval 764, oval 765, and oval 766 are real ontologies, which contain the URI of the original ontologies. Oval 767 is a real ontology.

FIG. 24 illustrates an exemplary functional architecture of the ODC. The Ontology Discovery Component (ODC) 723 accepts the discovery requests from any entity such as M2M applications (e.g., AE in oneM2M), analyzes the discovery request and finds the matching topic(s) from the OR 727 for M2M namespace 735, and searches over the matching topic(s) - returning the matching ontologies or fundamentals requested to the requester. The ODC discussion herein is similar to what is discussed with regard to discovery with regard to the discovery, retrieval and validation process of semantics related resources, but with an adjusted resource hierarchy.

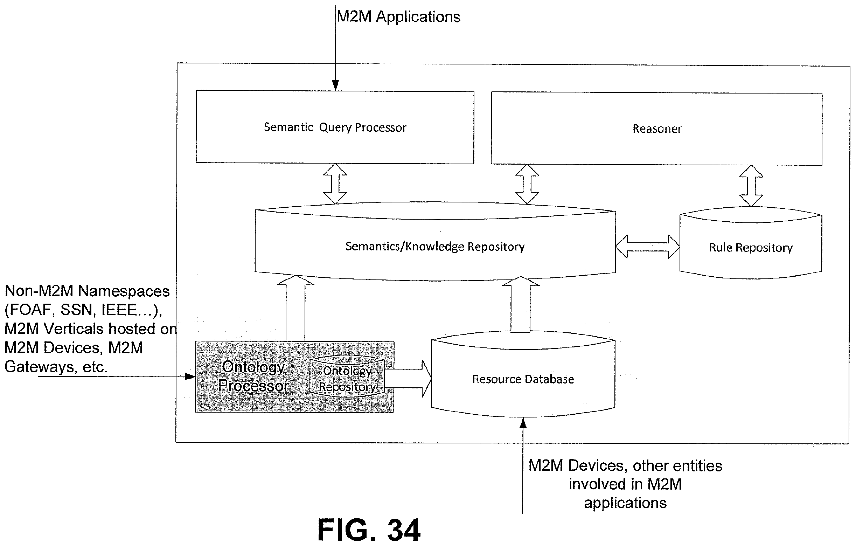

Fields and descriptions in a discovery request message include a request type, a search key, and a response type. Table 10 shows a summary of the fields. The request type indicates what the requester is looking for, which could be an entire ontology, or fundamentals inside of an ontology. Thus the possible value of this field may be the following: ontology, class, relationship. The search key includes the search keys sent by the requester. Note, the ontologies may also have their own semantics (e.g. namespace or topic they belong to), which are maintained by a semantics Repository in FIG 34, as a result, ontology discovery may be carried out through Semantic Query Processor in FIG. 34. The response type indicates the response that the requester desires, which is either the URI of the matching result or the URI and representation of the matching result.

**Table 10: Discovery Request Message Field and Description**

| **Message Field** | **Description** |
|---|---|
| request type | It indicates what the requester is looking for, which could be entire ontology, or fundamentals inside of an ontology. |
| search keys | It includes the search keys set by the requester |
| response type | It indicates the response that the requester desires |

FIG. 25 illustrates an exemplary message flow for ontology discovery. At step 772, requester 771 (e.g. M2M application, for example, another blood pressure monitor application in another gym) sends a discovery request to MOP 721. The request type is set to class and the search keys are set to "health" and "blood pressure" and "BP", which indicates that the requester is interested in discovering the classes modeling blood pressure. The response type is set to URI, which means the requester only needs the URI of the matching classes. The requester may use the discovered ontology to describe the semantics information of itself or its measured data, etc.

At step 773, ODC 723 of MOP 721 analyzes the request, and searches over OR 727 for M2M namespace 735 at the topic level. ODC 723 finds the possible matching topic is health. ODC 723 further searches over the ontologies under the health topic, and finds two matching ontologies. One is virtual, which contains the URI of the original ontology: Non-M2M-Namespace/IEEE11073/11407/. Another is with URI of M2M-Namespace/health/bloodPressure/. ODC 723 eventually finds the two matching classes under the two ontologies and, at step 774, returns the URI of them to requester 771. At step 775, requester 771 may retrieve the representations of the two ontologies, and see if any of them is suitable for describing its data. The requester 771 may also extend any of the existing ones to suit its own data. For example, Table 6 shows the ontology published by IEEE 11073-10407, it has 4 elements. Table 7 shows the ontology published by M2M vertical, which has 2 elements. The client may have data that only has two elements that needs to be described. So the ontology in Table 7 will be the suitable ontology.

With reference to the gym use case associated with FIG. 11, there may be different devices deployed, e.g. ambient sensors for sensing the surrounding temperature/humidity etc., weighing scale and blood pressure monitor for gym users to keep track of their vitals. The resources that are relevant to the devices (e.g. application resource) and the data resources that are produced and reported by the devices are may be stored in a resource database.

In order to provide the semantics to those devices and their measured data, each device may need to discover the ontology satisfying its own requirement and use it. On the other hand, the members of the gym also need ontology to describe their profiles and relationships to each other. The followings show how the published ontologies maintained by OR 727 may be discovered and used in the gym use case, such that they do not need to be recreated by the M2M namespace again.

A phone of a gym member 707 (hereinafter gym member) may send a discovery request (containing class, person, URI in the message) to MOP 721 to find the ontology to describe his/her profile and relationship to others, the MOP may match the FOAF ontology under the person topic to this request and return the URI of the ontology to the gym member 707. In order to use it, the gym member 707 retrieves the ontology representation from OR 727 by targeting the URI.

An application of an ambient sensor 706 may send a discovery request (containing class, sensor, URI in the message) to MOP 721 to find the ontology to describe its measured data, the MOP 721 can match the SSN ontology under the sensor topic to this request and return the URI of the ontology to the sensor. In order to use it, the ambient sensor 706 retrieves the ontology representation from OR 727 by targeting the URI. Note, since the ambient sensor 706 is resource constrained device, it may not have the ability to process the whole representation of the SSN ontology. The ambient sensor 706 may further discover the fundamentals of the ontology from the URI, and only retrieve the one(s) it needs, (e.g., ssn:SensorOutput).

An application of a blood pressure monitor 701 may send a discovery request (containing class, health; blood pressure, URI in the message) to MOP 721 to find the ontology to describe its measured data, the MOP 721 may match the two ontologies under the health topic to this request and return the URIs of the ontologies to the application of the blood pressure monitor 701. In order to use one of them, the application of the blood pressure monitor 701 retrieves the ontology representation from OR 727 by targeting the URI.

An application of weighing scale 702 may send a discovery request (containing class, health; scale, URI in the message) to MOP 721 to find the ontology to describe its measured data, the MOP 721 may match the ontology under the health topic to this request and return the URI of the ontology to the application of weighing scale 702. In order to use it, the application of weighing scale 702 retrieves the ontology representation from OR 727 by targeting the URI. It is possible that one of the devices, such as the ambient sensor 706, gym member 707, application of blood pressure monitor 701, and application of weighting scale 702, may not be satisfied with the ontologies discovered and retrieved from OR 727. The devices may extend or modify the ontologies per their requirements.

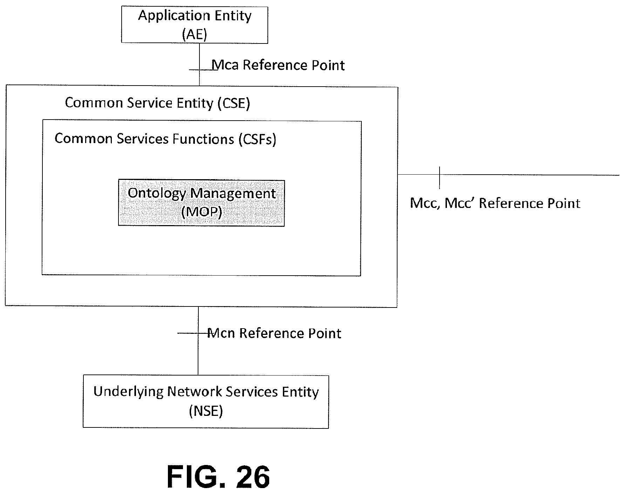

Below is a discussion of a particular example that may be applicable to oneM2M. oneM2M defines the capabilities supported by the oneM2M Service Layer. The oneM2M Service Layer is instantiated as a Common Services Entity (CSE) which comprises a set of Common Service Functions (CSF). In one example, the MOP 721 may be hosted in a CSE as an oneM2M CSF as shown in FIG. 26. As another example, MOP 721 may be part of a semantics node as discussed (which also may be an oneM2M CSF).

Any oneM2M verticals, i.e. AEs, may talk to the Ontology Management CSF via the Mca reference point to publish their ontologies or discover the existing ontologies, or other CSEs may talk to the Ontology Management CSF via the Mcc/Mcc' reference point to publish their ontologies or discover the existing ontologies. The publishers from outside of oneM2M namespace may talk to the Ontology Management CSF via the Mcc' reference point to publish their ontologies. As a result, the corresponding messages and procedures proposed discussed herein with regard to the MOP will apply to Mca, Mcc and Mcc' reference points.

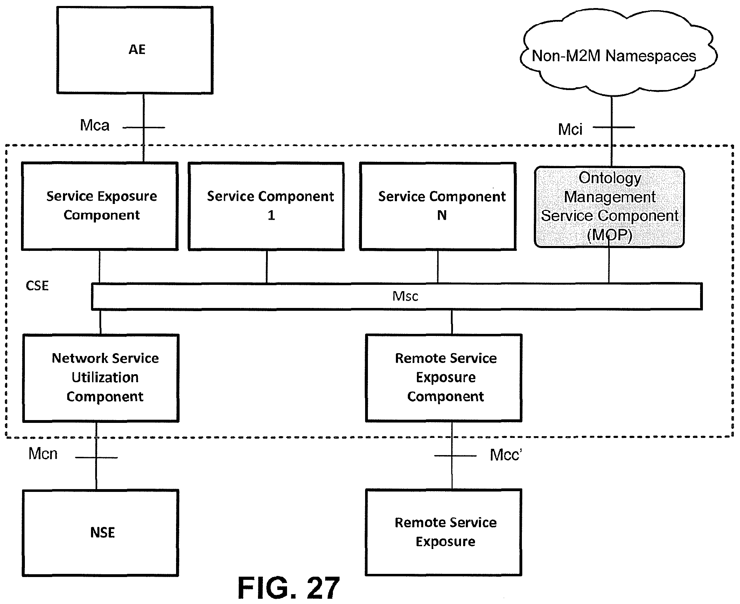

FIG. 27 also shows exemplary implementation architecture of Ontology Management in the oneM2M Service Component Architecture (Service Component Architecture, TS-0007 Service Component Architecture-V-0.2.0). oneM2M verticals, i.e. AEs may talk to the Ontology Management Service Component via the Mca reference point to publish their ontologies or discover the existing ontologies. The publishers from outside of oneM2M namespace may talk to the Ontology Management Service Component via a new reference point Mcc/Mcc' to publish their ontologies. The messages and procedure proposed in the above sections apply to those reference points.

An exemplary oneM2M based resource tree structure of Ontology Repository (OR) is shown in FIG. 28, which has two sub-resources: <Non-M2M-Namespace> and <M2M-Namespace>. The format attribute of <OR> indicates the ontology representation format adopted by the oneM2M architecture. <Non-M2M-Namespace> stores all the ontologies that are published from the namespaces other than oneM2M. <M2M-Namespace> stores all the ontologies that are published from the oneM2M verticals. Table 11 shows the attributes of <namespace> and their descriptions. Table 12 shows the attributes of <external-ontology> and their descriptions. Table 13 shows the attributes of <internal-ontology> and their descriptions. The actualLink attribute of <virtual-ontology> shows the link of the original ontology that is stored under <Non-M2M-Namespace>.

**Table 11 Attributes of <namespace> Resource**

| **Attribute Name** | **Multipli city** | **RW/RO/WO** | **Description** |
|---|---|---|---|
| *expiration Time* | 1 | RW | See clause 9.6.1 in oneM2M-TS-0001 oneM2M Functional Architecture-V-0.6.1. (hereinafter [1]) |
| *labels* | 0..1 | RW | See clause 9.6.1 in [1]. |
| *creation Time* | 1 | RO | See clause 9.6.1 in [1]. |
| *lastModifiedTime* | 1 | RO | See clause 9.6.1 in [1]. |
| *announce To* | 1 | RW | See clause 9.6.1 in [1]. |
| *announcedAttrib ute* | 1 | RW | See clause 9.6.1 in [1]. |
| *name* | 1 | RW | Denotes the name of the namespace. |
| *ref* | 1 | RO | Denotes the link of the external namespace. |

**Table 12 Attributes of <external-ontology> Resource**

| **Attribute Name** | **Multiplicity** | **RW/RO/WO** | **Description** |
|---|---|---|---|
| *expiration Time* | 1 | RW | See clause 9.6.1 in oneM2M-TS-0001 oneM2M Functional Architecture-V-0.6.1. (hereinafter [1]) |
| *labels* | 0..1 | RW | See clause 9.6.1 in [1] |
| *creation Time* | 1 | RO | See clause 9.6.1 in [1] |
| *lastModifiedTime* | 1 | RO | See clause 9.6.1 in [1] |
| *announceTo* | 1 | RW | See clause 9.6.1 in [1] |
| *announcedAttribute* | 1 | RW | See clause 9.6.1 in [1] |
| *name* | 1 | RW | Denotes the name of the ontology |
| *topic* | 1..n | RW | Defines the list of topics that the ontology is relevant to. |
| *representation* | 1 | RW | The actual representation of the ontology in the oneM2M compatible format. |

**Table 13 Attributes of <internal-ontology> Resource**

| **Attribute Name** | **Multiplicity** | **RW/RO/WO** | **Description** |
|---|---|---|---|
| *expiration Time* | 1 | RW | See clause 9.6.1 in [1] |
| *labels* | 0..1 | RW | See clause 9.6.1 in [1] |
| *creation Time* | 1 | RO | See clause 9.6.1 in [1] |
| *lastModifiedTime* | 1 | RO | See clause 9.6.1 in [1] |
| *announce To* | 1 | RW | See clause 9.6.1 in [1] |
| *announcedAttribute* | 1 | RW | See clause 9.6.1 in [1] |
| *name* | 1 | RW | Denotes the name of the ontology. |
| *other-topic* | 1..n | RW | Defines the list of other topics that the ontology is relevant to other than the current one. |
| *representation* | 1 | RW | The actual representation of the ontology in the oneM2M compatible format. |

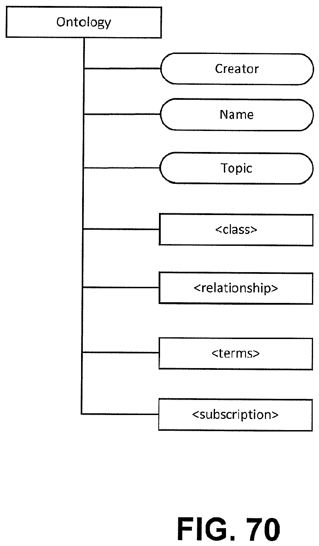

In addition, FIG. 28 shows the child resources of a proposed <ontology> resource, instantiated as <internal-ontology> and <external-ontology>. This resource is exemplified in FIG. 70 and contains the <class>, <relationship> and <term> sub-resources. FIG. 70 also indicates that the <ontology> resource can be subscribed to via methods used by another resource.

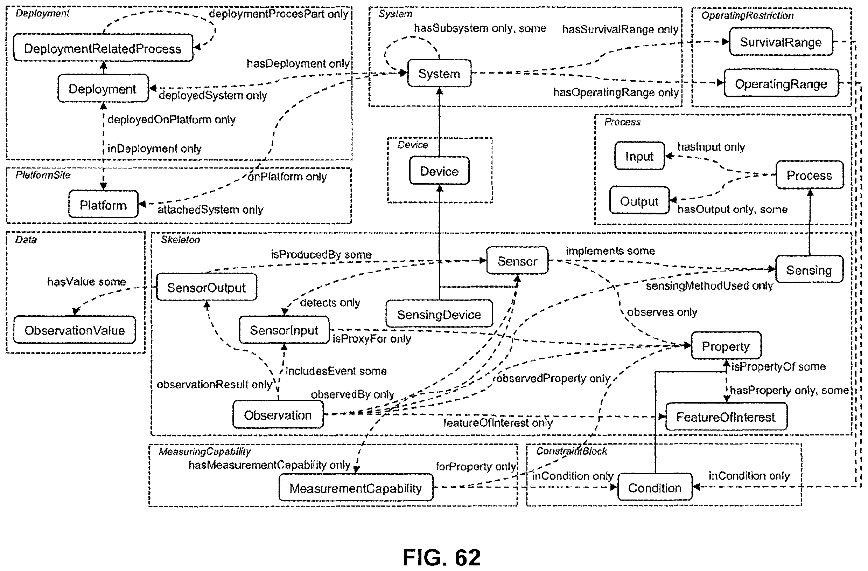

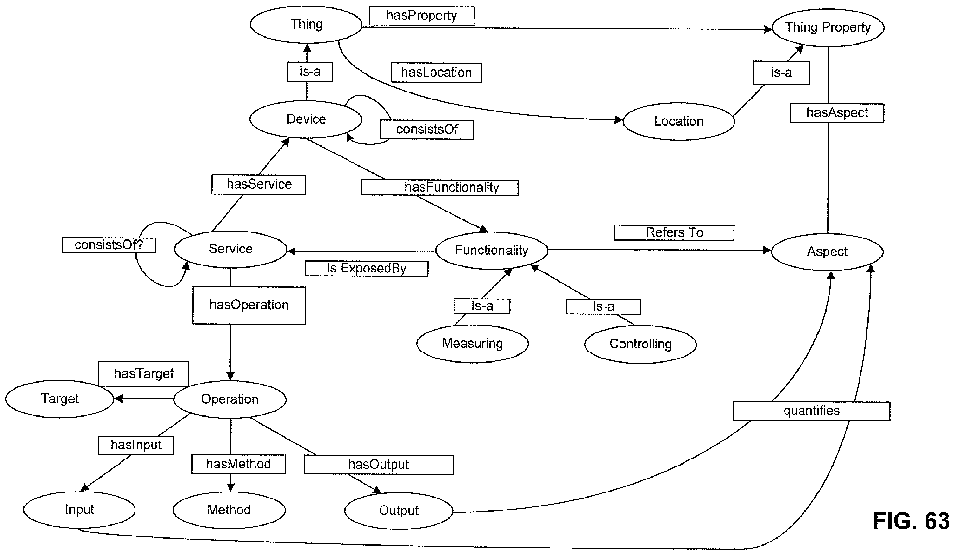

FIG. 62 illustrates an exemplary ontology representation of the semantics sensor network (SSN) ontology. As provided in the below tables, examples may be provided with regard to semantics ontology. FIG. 63 illustrates a oneM2M Base Ontology. As discussed in more detail herein, there may be a resource mapping to the oneM2M Base Ontology. oneM2M Base Ontology is discussed briefly to be used to help put in context the discussion below. The oneM2M Base Ontology is under development as part of a larger body of specifications for a M2M service layer that is required to provide enablement of semantic functionality. This ontology is being designed currently as minimal ontology (e.g.,. mandating the least number of conventions) that is required so that other ontologies may also be mapped into and utilized by oneM2M platforms, e.g. the semantic sensor network ontology described herein and in reference to FIG. 62. In oneM2M, external ontologies might be used to describe specific types of devices or real-world "Things" (like buildings, rooms, cars, cities, etc.) that should be represented in an oneM2M implementation. The goals are maximizing re-use of existing work as well as providing semantically-driven interworking solutions.

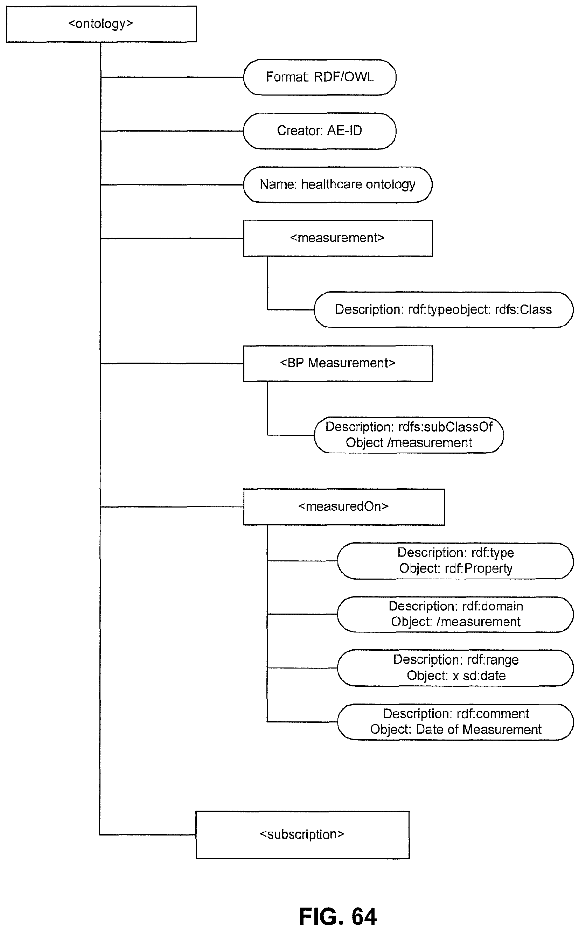

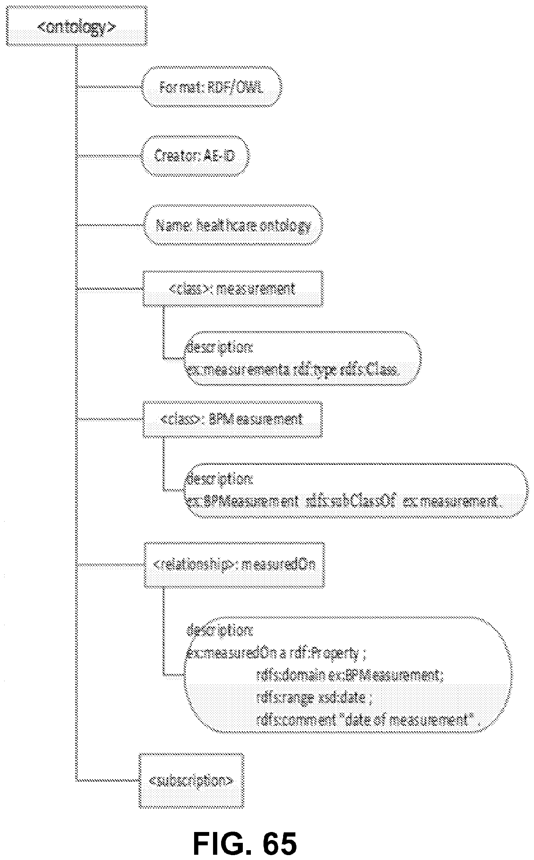

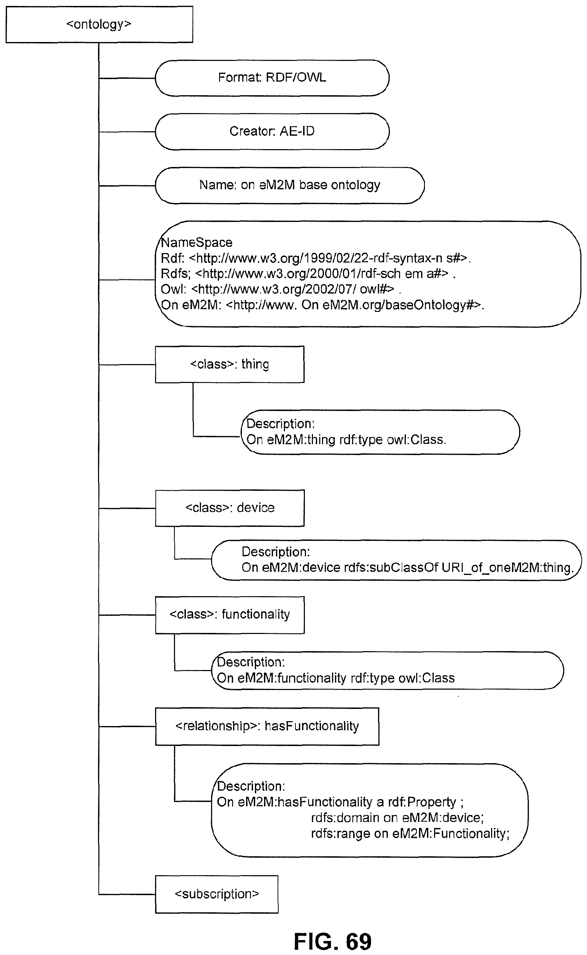

Discussed below with reference to FIG. 64 through FIG. 68 are different considerations for the <class> and <relationship> sub-resources. A first consideration of the <class> and <relationship> resources is provided below. FIG. 64, FIG. 65, and FIG. 69 are <ontology> resources that illustrate different ways of structuring the attributes. FIG. 64 and FIG. 65 have different ways of structuring their description attributes to maintain class and relationship information in the ontology definition. The blood pressure measurement is used as the example. A difference of FIG. 64 and FIG. 65 is that FIG. 65 defines class and relationship in RDF triples, which is the original expression of semantics information. FIG. 64 further splits a triple into relationship and object. These two ways may affect the semantics discovery and reasoning, as well as the way of creating ontology resource.

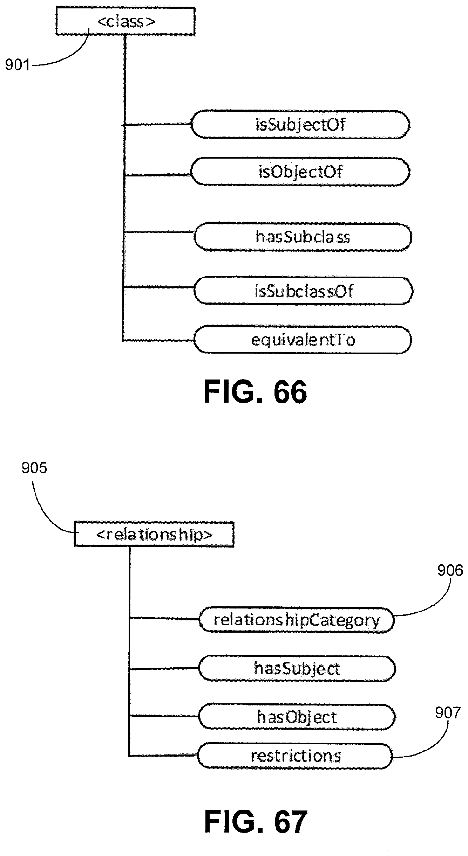

A second consideration of the resource structure for the <class> and <relationship> resources are shown in FIG. 66 and FIG. 67. Table 14 and Table 15 show the attributes of these two resources and their descriptions. In this second example, the two resources (<class> 901 and <relationship> 905) have multiple attributes describing relationships within the ontology, as well as other characteristics, such as relationship category 906 or restrictions 907. Attributes such as "isSubjectOf' (for <class> 901) are detailed with different options, one in which the attribute contains a single value (URI of a <relationship> resource) another where the attribute contains a tuple (URIs to both <relationship> resource and corresponding object <class> resource). In another variant, these values might be literal values instead of URIs of the corresponding resources.

**Table 14: Attributes of <class> Resource**

| **Attribute Name** | **Multiplicity** | **RW/RO/WO** | **Description** | **Examples** |
|---|---|---|---|---|
| *expiration Time* | 1 | RW | See clause 9.6.1 in [1]. | |
| *labels* | 0..1 | RW | See clause 9.6.1 in [1]. | |
| *creation Time* | 1 | RO | See clause 9.6.1 in [1]. | |
| *lastModifiedTime* | 1 | RO | See clause 9.6.1 in [1]. | |
| *isSubjectOf* | 1..n | RW | URI(s) to a relationship for which the class is a subject or to a tuple consisting of both the relationship and object class | E.g., in SSN (FIG. 62) for class "Sensor" this attribute may be: |
| | | | | a. Tuple of links (relationship "detects only", class "Sensorlnput"); or |
| | | | | b. Link to relationship "detects only" |
| | | | | Other variants might use literals instead of pointers, e.g., literal "detects only" instead of the URI of the corresponding resource |
| *isObjectOf* | 1..n | RW | URI(s) to a relationship for which the class is an object or to a tuple consisting of both the relationship and subject class | E.g., in SSN (FIG. 62)for class "Sensorlnput" this attribute may be either: |
| | | | | a. Tuple of links (relationship "detects only", class "Sensor") |
| | | | | b. Link to relationship "detects" |
| | | | | |
| | | | | Other variants might use literals instead of pointers, i.e. literal "detects only" instead of the URI of the corresponding resource |
| *has Subclass* | 0..n | RW | URI to another class which is a subclass of the done being defined | E.g. from FIG. 25 URI to "M2M node" when defining class "Physical Object". Other variants might use the literals instead of pointers, e.g., literal "M2M node" instead of the corresponding URI |
| *isSubclassOf* | 0..n | RW | URI to another class which is a superclass of the done being defined | E.g., from FIG. 25. URI to "Physical Object" when defining class "M2M node". Similar note to above about the use of literals instead of URIs |
| *equivalentTo* | 0..n | RW | URI to a class from a different ontology which is the equivalent of this class. | E.g., ssn: device and oneM2M:device may be two equivalent classes |

**Table 15: Attributes of <relationship> Resource**

| **Attribute Name** | **Multiplicity** | | **RW/RO/WO Description** | **Examples** |
|---|---|---|---|---|
| *expiration Time* | 1 | RW | See clause 9.6.1 in [1]. | |
| *labels* | 0..1 | RW | See clause 9.6.1 in [1]. | |
| *creation Time* | 1 | RO | See clause 9.6.1 in [1]. | |
| *lastModifiedTime* | 1 | RO | See clause 9.6.1 in [1]. | |
| *relationship Category* | 0..n | RW | Optional, describes the relationship type, e.g., Synonymy, Antonymy, Hyponymy, Meronymy, Holonymy. The attribute may indicate only the type/category, or be a tuple of type and associated relationship. | E.g., "is_a" belongs to the Hyponomy category, meaning that the object class is a specialization of the subject class. In reverse, the subject class is a generalization of the object class. The relationshipCategory would allow reasoning to be able to find for example all the hyponomous relationships for its logic. Meronymy and Holonymy provide information about the aggregation/composition of classes, while synonymy and antonomy would identify similar and opposite relationships. Other categories may be symmetrical, asymmetrical, etc., or object/data relationship categories |
| *has Subject* | 1..n | RW | URI(s) to a class who is a subject for this relationship | E.g., in SSN (FIG. 62)for Object property "detects" this attribute is a link to class "Sensor" |
| *hasObject* | 1..n | RW | For Object relationships/properties, a URI(s) to a class which is the object for this relationship. For Data properties it would contain a data type | E.g., in SSN (FIG. 62)for Object property "detects" this attribute is a link to class "Sensorlnput" For the Data property "hasManufacturer" with subject class "Sensor", this attribute may be the data type "Literal" |
| *restriction* | 0..n | RW | Restrictions posed by this relationship, as they map to the OWL use of restriction | E.g., owl:someValuesFrom |

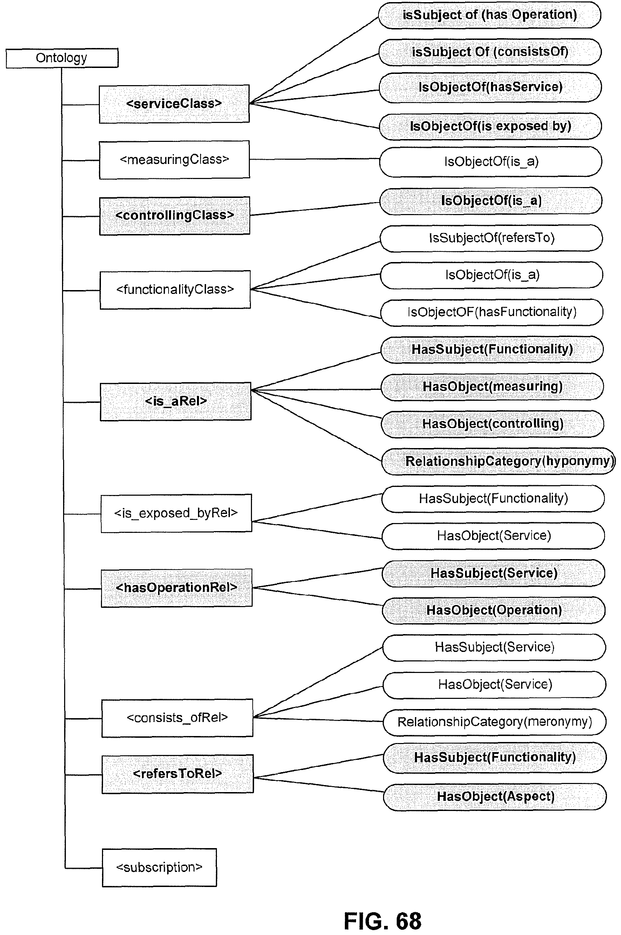

The second embodiment of the <class> and <relationship> resources (FIG. 66-FIG. 67, Table 14, Table 15), with its variants (some attributes provided as single values or tuples, or being provided as directly or as URIs), may be especially useful for advanced functionality. Understanding the relationship type may be used for advanced reasoning. For example, being able to understand all the relationships which denote generalization vs. specialization relationships available in an ontology, or the equivalent classes between several ontologies, may be used for advanced reasoning. Similarly, understanding what are the relationships that have a specific class as a subject may allow for derived or expanded logic to be applied for queries based on specific classes. In addition to the attribute examples provided in Table 14 and Table 15 which use concepts from the SSN ontology, FIG. 68 illustrates an example of the <Ontology> resource mapping to the oneM2M base ontology as introduced herein (e.g., with regard to FIG. 63). When considering the example as shown in FIG. 68, it may be assumed that the attributes contain single URI values. Notations like <serviceClass> and <hasOperationRel> are meant to convey that these represent the <service> resource of <class> type and <hasOperation> resource of <relationship> type. The notation isSubjectOf (hasOperation) denotes an attribute of isSubject type with the value hasOperation. Not all attributes are shown, only exemplary ones.

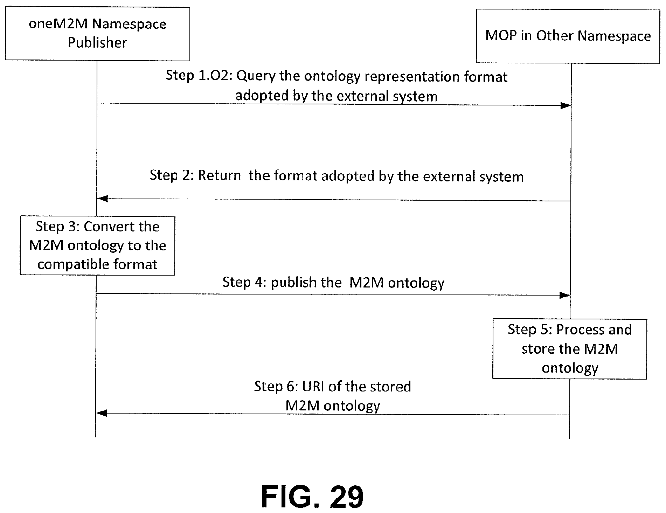

M2M system (e.g., oneM2M) may also publish an M2M ontology to other namespaces/systems (e.g., W3C) that may also adopt the MOP functionalities to process, classify and store ontologies. The message flow shown in FIG. 29 is an example of the message exchange used by an oneM2M implementation to publish its ontology to another outside namespace. The external namespace or system may include a MOP or may implement only basic functionality to support these messages and store the M2M ontology or its link, without some of the classification and discovery features of the MOP as presented above, such as the description associated with FIG. 13 -FIG. 19. The messages used are similar to the ones described above, such as the description associated with FIG. 13 -FIG. 19. In addition to the message exchange, the oneM2M MOP will perform Step 3, the conversion of the M2M ontology to another format if necessary. The MOP functionalities may be applicable to other namespaces/systems other than M2M. For example, W3C may also adopt the MOP functionalities to process, classify and store the ontologies published from the M2M namespace.

### M2M High Level Ontology Model

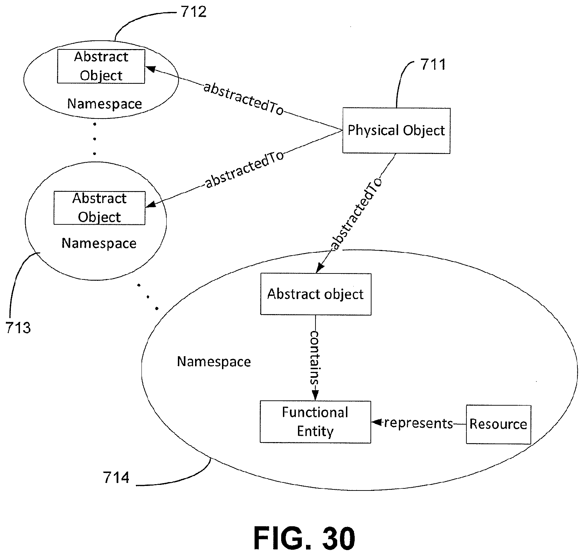

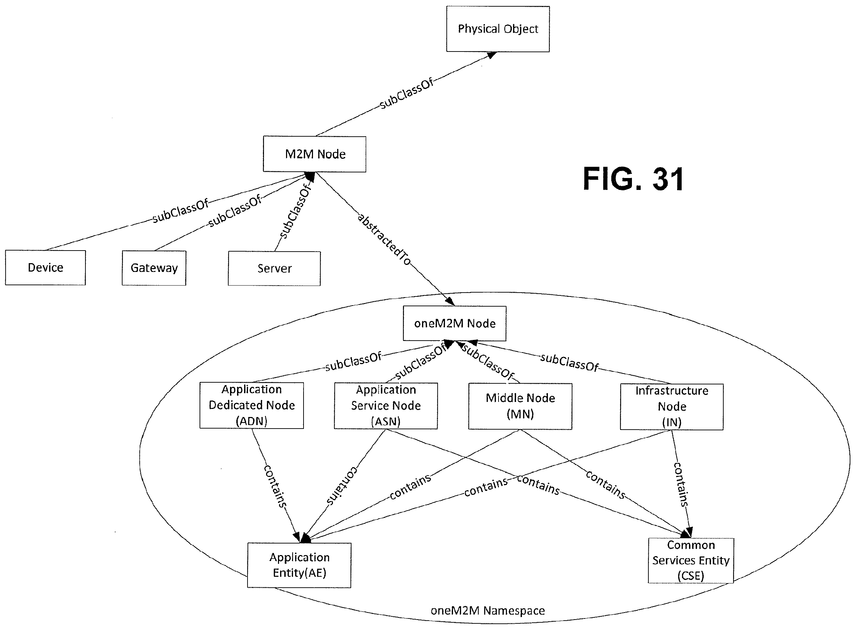

FIG. 30 illustrates a high level ontology model. It is considered that things in the physical world may be physical objects. Each of the physical objects may be modeled as an abstract object in different namespaces. For example, person/gym member can be modeled by FOAF ontology with the FOAF namespace, sensor may be modeled by SSN ontology with the W3C SSN namespace, health device (weighing scale, blood pressure monitor) can be modeled by IEEE 11073 namespace. Meanwhile, the M2M node in the M2M system (e.g. M2M device, M2M gateway, M2M server) is abstracted to the abstract object in M2M namespace(s), one example of which is oneM2M. The abstract object contains the functional entities, which could be represented by resources maintained by the M2M system. For example, as shown in Section FIG. 31 a physical object is abstracted to an M2M node in the oneM2M namespace, each of which may contains AE or/and CSE.

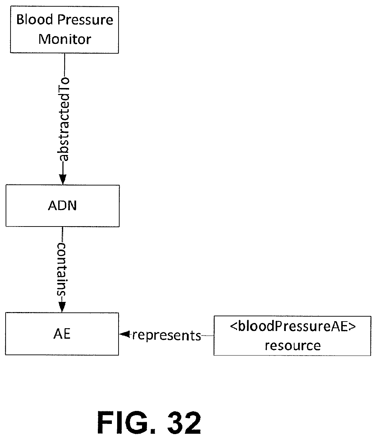

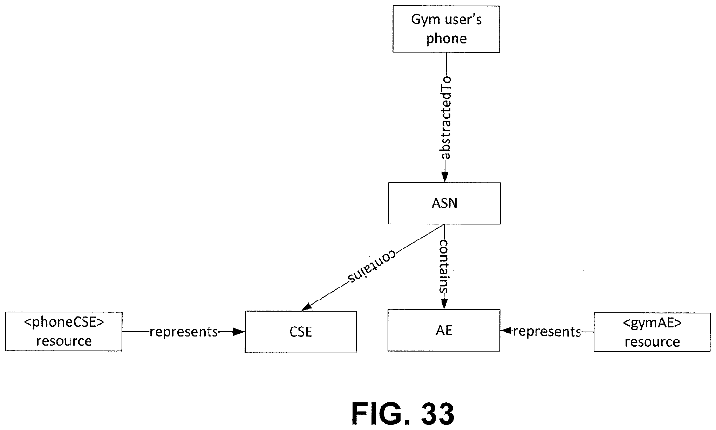

FIG. 31 illustrates and exemplary oneM2M resource oriented architecture (ROA) of M2M high level ontology model shown in FIG. 30. The M2M Node is one subclass of physical object, which has subclasses of Device, Gateway, and Server. The M2M Node can be abstracted to oneM2M Node in the oneM2M namespace. In oneM2M namespace, four types of Nodes are defined: ADN, ASN, MN and IN, which are subclasses of oneM2M Node. An ADN contains only AE. An ASN contains one CSE and one or more AEs. MN contains one CSE and zero or more AEs. IN contains one CSE and zero or more AEs. Those can be shown by the "contains" relationship as shown in FIG. 31. FIG. 32 and FIG. 33 are different examples of abstractions that may be determined in a oneM2M namespace. FIG. 32 illustrates an example where the physical object is a blood pressure monitor. The blood pressure monitor is abstracted to an ADN, which contains an AE. The AE is represented by <bloodPressureAE> resource in the resource tree. FIG. 33 illustrates an example is a phone which may belong to a gym user. The phone is abstracted to an ASN, which contains CSE and AE. The CSE is represented by the <phoneCSE> resource, and the AE may be a gym AE installed on the phone, which is represented by the <gymAE> resource in the resource tree. A physical object can only be modeled by an ontology after it is abstracted. MOP 721 is in charge of process those ontologies.

### Functional Architecture of M2M Semantic Support

Below is further discussion with regard to ontologies to give further perspective. All things in the physical world may be considered as physical objects. Each of the physical objects may be modeled as an abstract object in different namespaces. FIG. 30 illustrates an exemplary overview of abstractions of physical object and different namespaces. There may be any number of namespaces such as namespace 712, namespace 713, and namespace 714 that stem from an abstraction of a physical object 711. Each namespace may have one ontology or multiple ontologies that model one or more physical objects.

FIG. 34 illustrates an exemplary functional architectural for M2M semantic support, the components may be one or more of the following:
- Resource Database stores resources that are collected from the physical M2M Devices and any resources that are relevant to the M2M applications, such as person, devices, etc. involved in the M2M applications (normal resources). The M2M semantic support is enables semantics to the original resources for universal understanding/interpretation of them, as well as any advanced processing on them, e.g. semantic query, data analytics, etc.
- Ontology Processor is in charge of processing, classifying, storing and providing discovery function of published/generated ontologies external and internal of the M2M domain. It contains Ontology Repository which stores the ontologies. Those ontologies can be used for normal resources to enable semantics. The Ontology Repository may be considered to be part of the logical entity Semantics Node introduced herein. The M2M system may process, manage and otherwise use the ontologies by the Ontology Processor.
- Semantics Repository stores the semantics information of normal resources in certain representations, which may have the option of utilizing RDF.
- Rule Repository stores the rules that are used to represent new knowledge that often goes beyond the existing semantics that is associated with the resources in Resource Database. Rules are typically conditional statements: if-then clauses.
- Reasoner takes input from the Rule Repository and the existing resource semantics information in the Semantics Repository and generates new resource semantics information if the conditions in the rules are satisfied. New resource semantics information is added to the Semantics Repository.
- Semantic Query Processor handles the queries from clients to search over the knowledge/resource semantics information stored in the Semantics Repository and returns the results to the clients.

With regard to the gym use case as discussed regarding FIG. 11 above:
- The semantic repository may store the semantics information about the gym members, devices in the gym and the data produced by the devices.
- Semantic-based query service may be needed when the member wants to select a treadmill to use based on availability, function etc.
- The Rule Repository may store rules such as if the member has senior membership, it is granted to use certain machines, the Reasoner may use the rule to add the new semantics information to a resource, e.g. person's accessible device.
- The functions of semantics support shown above may be hosted on the Gateway 704 in the gym use case.

FIG. 35 is a diagram illustrating a communication system 120 that implements the ETSI M2M architecture defined by ETSI in its TS 102 690. Note that this diagram is used to assist in understanding of the disclosure and is simplified to facilitate description of the subject matter disclosed herein. As shown in FIG. 35, the system 120 may comprise a plurality of network domains, such as network domain 122, network domain 130, network domain 135, and network domain 138. Each network domain may include a network service capability layer (NSCL), such as NSCL 126, NSCL 131, NSCL 136, and NSCL 139. Each NSCL may interface with a respective network application, such as network application 127 and network application 132 in network domain 122 and network domain 130, respectively.

As further shown, a network domain, such as network domain 122, may further comprise one or more devices, such as device 145 (which for example may be one of the M2M devices used in the patient monitoring application of FIG. 1), and one or more gateways, such as gateway 140. In 3GPP parlance, devices and gateways are examples of UEs. As shown, the device 145 may be running a device service capability layer (DSCL) 146 which communicates with the NSCL 126 over the mId reference point defined by the architecture. A device application (DA) 147 may also be running on the device 145, and it may communicate with the DSCL 146 over a dIa reference point. Similarly, the gateway 140 may implement a gateway service capability layer (GSCL) 141 that communicates with the NSCL 126 over the mId reference point. A gateway application (GA) 142 running on the gateway 140 may communicate with the GSCL 141 via the dIa reference point. In general, dIa reference points allow device and gateway applications to communicate with their respective local service capabilities (i.e., service capabilities available at a DSCL or a GSCL, respectively). The mId reference point allows an M2M SCL residing in an M2M Device (e.g., DSCL 146) or an M2M Gateway (e.g., GSCL 141) to communicate with the M2M service capabilities in the network domain (e.g., NSCL 126) and vice versa.

Still referring to FIG. 35, in greater detail, NSCL 126 may be in domain 122 and be configured with network application (NA) 127 on an M2M server platform 125. NA 127 and NSCL 126 may communicate via reference point mIa 128. The mIa reference points may allow an NA to access the M2M service capabilities available from an NSCL in an M2M domain.

Typically, the device 145, gateway 140, and M2M server platform 125 comprise computing devices, such as the devices illustrated in FIG. 60C and FIG. 60D and described below. The NSCL, DSCL, GSCL, NA, GA, and DA entities typically are logical entities that are implemented in the form of software, executing on the underlying device or platform, to perform their respective functions in the system 120.

As further shown in FIG. 35, NSCL 131 may be in domain 130 with NA 132. NA 132 and NSCL 131 may communicate via mIa reference point 133. There could also be an NSCL 136 in network domain 135, and NSCL 139 in network domain 138. mIm reference point 123 may be an inter-domain reference point that allows M2M network nodes in different network domains, such as NSCL 126 in network domain 122, NSCL 131 in network domain 130, NSCL 136 in network domain 135, or NSCL 139 in network domain 138, to communicate with one another. For simplicity herein, the term "M2M server" may be used to indicate a service capability server (SCS), NSCL, application server, NA, or an MTC server. In addition, the term user equipment (UE), as discussed herein, may apply to a GA, GSCL, DA, or DSCL. A UE, as discussed herein, may be considered a mobile station, a fixed or mobile subscriber unit, a pager, a cellular telephone, a personal digital assistant (PDA), a smartphone, a laptop, a netbook, a personal computer, a wireless sensor or actuator, consumer electronics, and the like. A machine-to-machine services capabilities layer entity as discussed herein may include an M2M server or a UE.

### I. M2M Architecture with Semantics Nodes

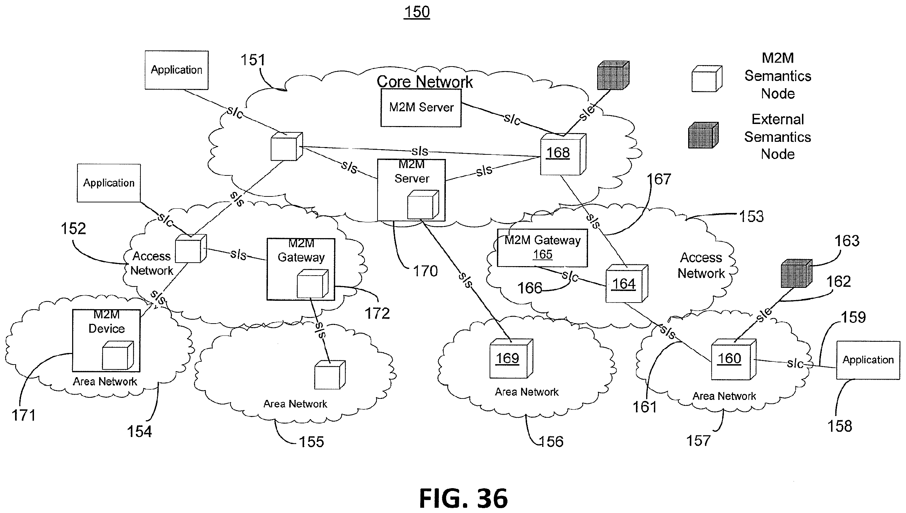

One example of a M2M system that includes semantics nodes is illustrated in in FIG. 36. As shown in M2M system 150 the semantics nodes are deployed at three levels. There may be an area level, which may include networks, such as area network 154, area network 155, area network 156, and area network 157. There may be an access level, which may include networks, such as access network 152 and access network 153. And there may be a core level, which includes a network, such as core network 151.

As shown in system 150, application 158 may be communicatively connected with M2M semantics node 160 located in area network 157 via reference point sIc 159. The sIc reference point is generally used by applications, other non-semantics node entities in the area network, access network, and core network entities to talk to a semantics node. M2M semantics node 160 is communicatively connected with external semantics node 163 via reference point sIe 162. The semantics nodes in M2M systems may interface with external semantics nodes via the sIe reference point. The external semantics nodes may manage other existing semantics related resources such as those defined by RDFS for the semantics web. M2M semantics node 160 is also communicatively connected with M2M semantics node 164 located in access network 153 via sIs reference point 161. M2M semantics node 164 is communicatively connected with M2M gateway 165 via sIc reference point 166 and communicatively connected with M2M semantics node 168 located in core network 151 via reference point sIs. In this example, the M2M Gateway 165 itself is not a semantics node, but in other examples, the M2M Gateway 165 could incorporate the functionality of semantics node.

Semantics node(s) may be deployed at the area level for the purpose of offloading, load balancing, easy access, etc. At the area level, it is possible that there is no semantics node deployed if all the devices in the local area network communicate with a semantics node in the attached access or core networks. A semantics node at the area level may have a corresponding parent semantics node at the access level (e.g., M2M semantics node 160 connected with M2M semantics node 164) or at the core level (e.g., M2M semantics node 169 connected with M2M server 170 that includes a semantics node). Semantics nodes talk to each other through the sIs reference point. The details of the reference points are defined further hereinafter. A semantics node at the access level may also have a parent at the core level, which it communicates with over the sIs reference point. Likewise, a semantics node at the access level may have children semantics nodes at the area level, which it communicates with via the sIs reference point. Semantics nodes at the core level may also have children semantics nodes at the access or area level.

In addition to the parent-child relationships illustrated in FIG. 36, the semantics node also supports a notion of siblings at any of the semantics node levels (e.g., access, area, or core). Sibling semantics nodes are nodes at the same level in the hierarchy and can be used to distribute the load of the semantics node. For example, in core network 151, M2M semantics node 168 is connected with M2M server 170 that includes a semantics node. From a vertical perspective, if there is an established hierarchy of more than one semantics node, then the nodes can talk to each other and share semantics information, via notification, broadcast, discovery, etc. over the sIs reference point.

For a constrained device, due to the limit of capacity, the semantics can be a code referring to a specific semantic or a link pointing to the semantics stored in a remote semantics node. Such semantic information may be provided by the application that created the data or by the service layer.

At the access level, there might be one or more semantics nodes deployed in one access network. If so, the siblings may communicate with each other for semantics information notification, broadcast and discovery through the sIs reference point. A semantics node at the access level may also have a parent at the core level which it communicates with over the sIs reference point. Likewise, a semantics node at the access level may have children semantic nodes at the area level which it communicates with via the sIs reference point.

At the core level, there might be one or more semantics nodes deployed in the core network. These nodes may be siblings and communicate with each other over sIs reference point for sharing semantics information using notification, broadcast and discovery. Semantics nodes at the core level may also have children semantic nodes at the access or area level. Applications, other non-semantics-node entities in the area network, access network and core network talk to a semantics node via the sIc reference point.

As mentioned above, a semantics node may be a standalone physical node in a network (e.g., standalone M2M semantics node 160) or it can be a logical entity hosted on another physical node in the network, such as an M2M device 171, M2M gateway 172, or M2M server 170, as shown in system 150 of FIG. 36. In the other words, an M2M device, M2M gateway, and M2M server can support semantics node functionalities.

A feature of the multi-layer semantics node hierarchy illustrated in FIG. 36 is that it can provide different levels of abstraction. A semantics node may only be in charge of managing the semantics related resources in a localized area, such as in a M2M area network, such that the area network specific semantics related resources may be found locally. Any semantics related resources that are not typical in the localized area can be stored and found in higher-level parent or parallel sibling semantics nodes. Another feature is that the semantics related resources may have a hierarchy themselves due to the Internet hierarchy, the location hierarchy, etc. As a result, the multiple layers of semantics are coherent with the existing network architecture. In addition, semantics nodes may be distributed in each level, which prevents a single failure point if a centralized semantics node is deployed only in the core network.

### II. Semantics Node Architecture

More details regarding the architecture of a semantics node will now be discussed. As mentioned, a semantics node stores and manages semantics related resources. As defined herein, a semantics related resource comprises information that can be used to describe the semantics information of a thing, such as the meaning of data generated by an M2M device or application or the M2M device or application itself. In one example, semantics related resources may be expressed in extensible markup language (XML) using existing schemas, such as XML Schema Definition (XSD), RDF Schema/Web Ontology Language (RDFS/OWL), or the like. In an example, three types of semantics related resources may be stored in a semantics node - classes, relationships, and terms - each of which is described more fully below. The categorization of semantics related resources in this manner provides compatibility with the current techniques of the semantics web. This compatibility enables an M2M system to leverage existing semantics related resources, such as for example those core classes and core properties defined by W3C. Applications and entities external to the M2M system are able to use the semantics related resources hosted by the semantics node, without incurring any extra overhead due to format conversion or modification necessary to make the semantics compatible.

**Classes.** Discussed herein is the concept of classes of objects/things in an M2M domain. In the example health monitoring system of FIG. 1, for example, the classes of objects relevant to the system include patient, doctor, ambulance dispatcher, blood pressure, core temperature, oxygen saturation, motion accelerometer and the like. A class may be identified by a uniform resource identifier (URI) or a uniform resource locator (URL). A class contains field descriptions that contain information defining the class. For example, a temperatureReading class that represents temperature data as an integer in units of Celsius and can be used, for example, to provide those semantics to data generated by a temperature sensor may be defined in XML with the schema of XSD as follows:

```
<simpleType name="temperatureReading">
        <restriction>
            description ="temperature in Celsius" unit="Celsius" base="integer"
        </restriction>
       </simpleType>
```

Here, the class contains the fields "description," "unit," and "base," and the information in those fields is "temperature in Celsius," "Celsius," and "integer," respectfully. As another example, a BloodPressure class may contain two fields, one for systolic pressure and another for diastolic pressure. The class description for this BloodPressure class would include both fields' descriptions and may be expressed using XML/XSD as follows:

```
       <complexType name="BloodPressure">
        <sequence>
         <element name="systolicINmmHG " type="integer"/>
         <element name="diastolicINmmHG" type="integer"/>
        </sequence>
       </complexType>
```

Again, however, it is understood that classes (and the other types of semantic related resources - relationships and terms) are not limited to expression using XML/XSD but rather may be expressed in any of a variety of suitable description languages, including, for example, RDFS/OWL and the like. Classes may also be related to each other. For example, "blood pressure" may be a subclass of "vitals", or equivalently, "vitals" may be a superclass of "blood pressure." The subclass/superclass relationships define a hierarchy of classes. In general, A is a subclass of B if every instance of A is also an instance of B. A class may have multiple super classes.

**Relationships.** Relationships are a special kind of semantics related resource. Relationships describe relations between semantics related resources, for example "created by," "lifetime," "subscribed by," and so on. A relationship can also be identified by a URI/URL, which gives a global and unique naming scheme to relationships of the M2M domain. Classes and inheritance are known in other fields of computing, for example in object-oriented programming. But while there are similarities, differences exist too. In object-oriented programming, an object class defines the relationships or properties that apply to it. To add new relationships or properties to a class means to modify the class. However, here, relationships are defined globally, that is, they are not encapsulated as attributes in class definitions. A new relationship may be defined that applies to an existing class without changing the definition of the class itself. Like classes, the relationships can also be related to each other. For example, "energy mode" and "usage mode" are sub-relationships of "mode". If a device has an "energy mode" of electric and a "usage mode" of manual, then it has a "mode" of electric and manual.

**Terms.** Terms are concepts that are commonly used in an M2M domain. As defined herein, a term is a value that may be used by many parties to describe the semantics of a resource. The definition of a term may be universally acknowledged in certain domains where it is published. For example, manual, user-directed, and autonomous are each examples of terms that may be used to describe, for example, an appliance's usage mode. In general, the creator of a semantics related resource will determine whether that semantics related resource is a class, relationship, or term. A semantics node will then store the semantics related resources under the categories defined or determined by their creator. For example, in the patient monitoring example of FIG. 1, semantics related resource classes, relationships, and terms may be created by a blood pressure monitor manufacturer, by a doctor, or by another appliance manufacturer. In other examples, semantics related resource classes, resources, and terms may be defined or created by a vertical application.

The semantics of a resource (including data, thing, etc.) can be described by a resource-relationship-value triple, consisting of a resource, a relationship and a value. Values can either be classes, terms, or other resources. The following are some examples,
- **A content instance** (resource) **hasType** (relationship) **temperatureReading** (class)
- **An appliance** (resource) **hasUsageMode** (relationship) **user-directed** (term)
- A content instance (resource) **generatedBySameApplicationAs** (relationship) **another content instance** (resource)

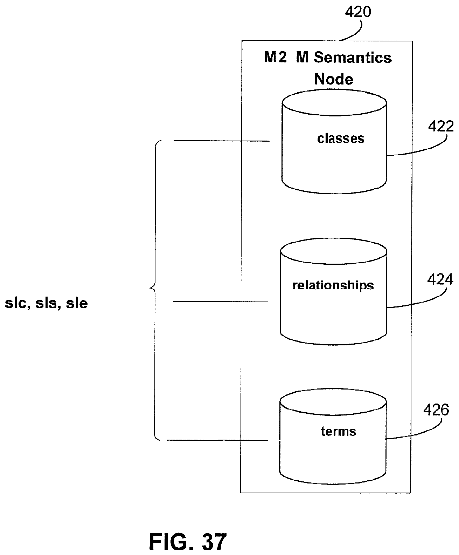

Through the proposed sIc, sIs and sIe reference points, requests to a semantics node's class, relationship and term resources can be made. FIG. 37 is an exemplary illustration of an M2M semantics node's class, relationship and term resources. As illustrated in this example, a semantics node 420 may be configured to store various classes 422, relationships 424 and terms 426 from different applications and domains. Alternatively, the semantics node 420 may be configured to store class, relationship, and term resources for a unique application or domain, such as appliance semantics, vehicle semantics, health care application semantics, or the like.

### III. M2M Semantics Node Functions and Reference Points

In this section, further details concerning the functions and reference points of a semantics node are provided. In one example, a semantics node may perform the following functions: authenticating other semantics nodes including authenticating lower level children or parallel sibling semantics nodes in a semantic node hierarchy to allow them to register and make requests for a semantic node's resources; authenticating applications, devices, and/or users to allow them to publish, create, delete, update, and retrieve semantics related classes, relationships and terms resources; storing and managing semantics related classes, relationships and terms resources; providing support for discovery of semantics related resources; and providing support for semantics nodes to communicate with one another (between parent-child, siblings) in order to collaborate on discovery queries and share semantics related resource information.

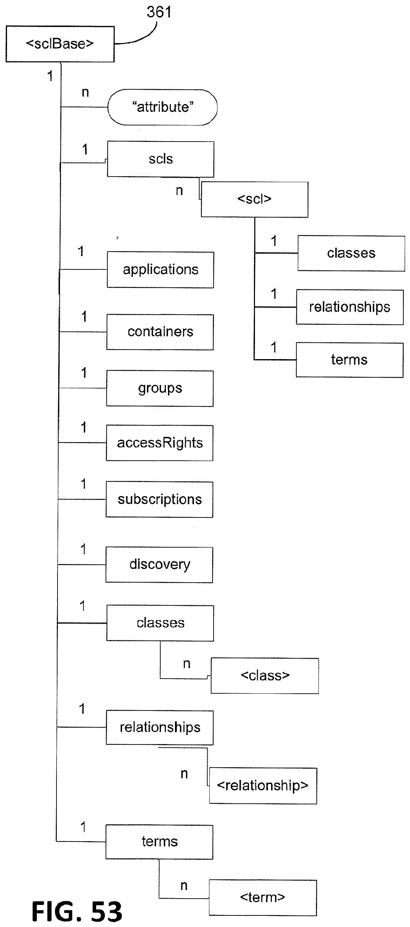

A semantics node may communicate with other entities in the network via one or more reference points or interfaces. In one example, three reference points are defined - a sIs reference point, a sIc reference point, and sIe reference point. The sIs reference point is used for communication between semantics nodes. The sIs reference point may also be used by a semantics node to register to another semantics node to form parent-child or sibling relationships, to discover other semantics nodes, to notify others about its status (e.g., online, offline, overloaded, etc.), to trigger another semantics node to perform a particular operation (e.g., de-registration, registration), to publish, create, delete, update, and retrieve semantics related resources in another semantics node. In addition, a semantics node may use the sIs reference point to subscribe to semantics related resources in another semantics node and to receive corresponding notifications, to discover semantics related resources on the siblings and parent semantic nodes in its hierarchy, to move a group of semantics related resources from one semantics node to another semantics node, and to allow the semantics related resources stored in another semantics node to be linked and associated with a resource to provide the semantics to that resource, as described further below in connection with FIG. 53, FIG. 54, and FIG. 55.

In the present example, the sIc reference point may be used by an application or non-semantics node to communicate with a semantics node from various network domains (e.g., area network, access network, or core network). The sIc reference point also allows an application or non-semantics node to publish, create, delete, update, retrieve, subscribe to, or discover semantics related resources in a semantics node and to receive notifications from the semantics node. In addition, the sIc reference point allows the semantics related resources stored in a semantics node to be linked and associated with a resource to provide the semantics to that resource.

The sIe reference point may be used for communication between a semantics node that is part of an existing hierarchy of nodes in an M2M system and an external semantics node. An external semantics node stores semantics-related resources outside of the M2M domain. One example of an external semantics node may be a server that stores the semantics-related resources for Semantic Sensor Network Ontology that is defined by the W2C Semantic Sensor Network Incubator Group, as described at http://www.w3.org/2005/Incubator/ssn. The sIe reference point allows a semantics node to retrieve, discover, and subscribe to semantics related resources in an external semantics node and vice versa. Also via the sIe reference point, external semantics nodes may discover semantics nodes in the M2M system and receive notifications.

One example of the messages associated with the sIs, sle, and sIc reference points, which effectively define those reference points, are set forth below in Table 16.

Table 16 lists semantics node related messages, there corresponding meanings, and reference points used. **Table 16: Semantics Node Messages**

| Message | Description | Reference Point |
|---|---|---|
| SEMANTICS_NODE_DISCOVERY_REQ / RESP | Discover individual semantics nodes to build the hierarchy of semantics nodes and sibling relationship. | sIs, sIc, sIe |
| SEMANTICS_NODE_REGISTER_REQ/RESP | Register to the upper-level semantics ode to build the parent-child relationship. | sIs |
| SEMANTICS_NODE_DEREGISTER_REQ/RESP | De-register from the upper-level semantics node to release the parent-child relationship. | sIs |
| SEMANTICS_NODE_STATUS_NOTIFY | Notify the status of a semantics node, e.g. online, offline, overloaded, etc. | sIs |
| SEMANTICS_NODE_DE-REGISTER_TRIGGER | Trigger a semantics node to de-register from the current parent, the new candidate parent may be piggybacked. | sIs |
| SEMANTICS_NODE_REGISTER_TRIGGER | Trigger a semantics node to register to another semantics node as parent while the current parent goes offline or proactively releases the parent-child relationship. | sIs |
| SEMANTICS_RELATED_RESOURCE _CREATE_REQ/RESP | Create semantics related resources in types of class, relationship, term in a semantics node | sIs, sIc |
| SEMANTICS_RELATED_RESOURCE RETRIEVE_REQ/RESP | Retrieve semantics related resources in types of class, relationship, term in a semantics node | sIs, sIc, sIe |
| SEMANTICS_RELATED_RESOURCE UPDATE_REQ/RESP | Update semantics related resources in types of class, relationship, term in a semantics node | sIs, sIc |
| SEMANTICS_RELATED_RESOURCE_DELETE_REQ/RESP | Delete semantics related resources in types of class, relationship, term in a semantics node | sIs, sIc |
| SEMANTICS_RELATED_RESOURCE_SUBSCRIBE_REQ / RESP | Subscribe to semantics related resources in types of class, relationship, term in a semantics node | sIs, sIc, sIe |
| SEMANTICS_RELATED_RESOURCE_SUBSCRIBE_NOTIFY | Notify the subscribers of the semantics related resource update | sIs, sIc, sIe |
| SEMANTICS_NODE_RESOURCE_SUBSCRIBE_REQ/RESP | Subscribe to a semantics node's stored and managed semantics related resources, with conditions set up. | sIs, sIc, sIe |
| SEMANTICS_NODE_RESOURCE_SUBSCRIBE_NOTIFY | Notify the subscribers of the semantics related resource update from the semantics node. | sIs, sIc, sIe |
| SEMANTICS_RELATED_RESOURCE_MODIFY_REQ/RESP | Modify a semantics related resource to suit the requester's requirement, which may be implemented by UPDATE and CREATE RESTFUL operations. | sIc |
| SEMANTICS_RELATED_RESOURCE_DISCOVERY_REQ | Discover existing semantics related resources in types of class, relationship, term by setting up the search strings (key words) | sIs, sIc, sIe |
| SEMANTICS_RELATED_RESOURCE_ DISCOVERYRESP | Respond to a semantics related resource discovery request by returning the address (e.g. URL/URI) of the matching semantics related resources | sIs, sIc, sIe |
| SEMANTICS_LINKAGE_CREATE_REQ/RESP | Create semantics linking and association between a M2M data/resource and a semantics related resource. | sIs, sIc, sIe |
| SEMANTICS_LINKAGE_UPDATE_REQ/RESP | Update semantics linking and association between a M2M data/resource and a Semantics related resource. | sIs, sIc, sIe |
| SEMANTICS_LINKAGE_RETRIEVE_REQ/RESP | Retrieve semantics linking and association between a M2M data/resource and a semantics related resource. | sIs, sIc, sIe |
| SEMANTICS_LINKAGE_DELETE_REQ/RESP | Delete semantics linking and association between a M2M data/resource and a semantics related resource. | sIs, sIc, sIe |

Protocols such as hypertext transfer protocol (HTTP) or constrained application protocol (CoAP) may be used as an underlying transport protocol for carrying the different types of messages. Examples are presented below of the use of these messages to perform the various semantic node functionalities discussed above

### IV. M2M Semantics Node Procedures

### A. Build Semantics Node Hierarchy

In this section, additional details are provided regarding how a hierarchy of semantics nodes (hereinafter "semantics node hierarchy") may be built, in accordance with one example. In this example, a semantics node hierarchy is built up by determining the parent-child and sibling relations between the semantics nodes located at the different area, access, and core levels in a network.

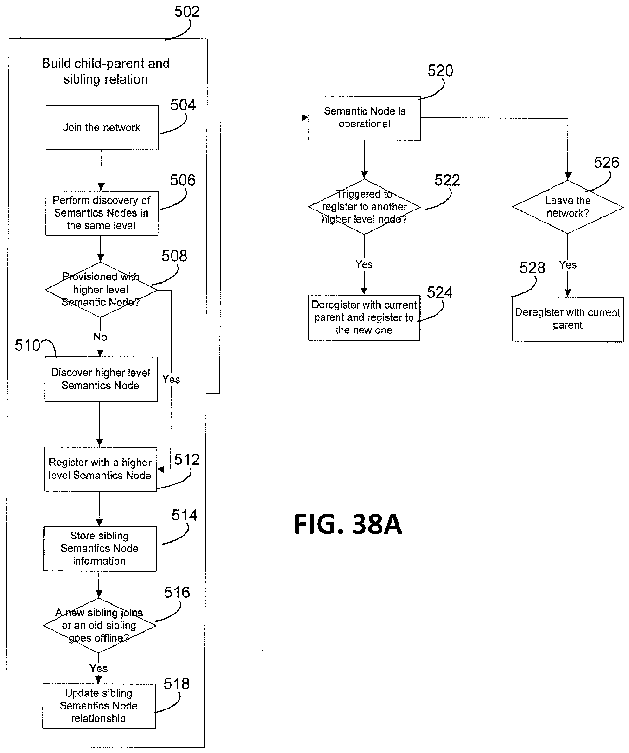

FIG. 38A is a flow diagram illustrating one example of a method for establishing a semantics node hierarchy. When a semantics node joins the network (step 504), i.e. becomes online, it needs to first build the child-parent and sibling relations before it can become an operational Semantic Node in the network. To this end, in step 506, the semantic node may perform discovery of sibling semantics nodes in the same level.

The discovery of sibling semantics nodes may be based on sending out semantics node discovery requests (e.g., multicast, broadcast, anycast) where a discovery request can be issued in an attempt to discover sibling semantics nodes. The request can have a defined hop-limit that limits the flooding of discovery request and response messages, which may congest the network. Alternatively, the discovery can leverage existing discovery mechanisms available within the network, such as domain name system (DNS), DNS service discovery (DNS-SD), service location protocol (SLP), etc., if available. A semantics node may store the returned information of the neighboring sibling semantics nodes, such as IP address or type(s) of semantics information managed. As discussed further below, the sibling semantics node discovery response message may also piggyback the address of a higher-level semantics node discovery server, the address of a parent semantics node of the sibling, or both.

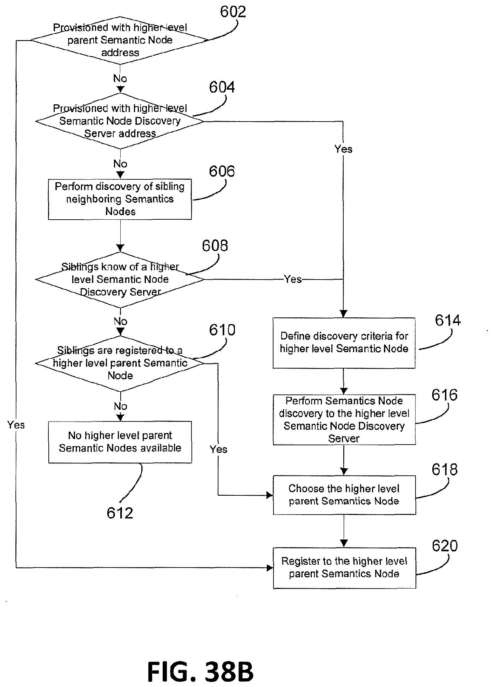

Referring still to FIG. 38A, following discovery of sibling semantic nodes, the semantic node may next attempt to discovery and/or register with higher level semantic nodes in step 508, step 510, and step 512. If the semantics node is provisioned with the higher level node that it needs to register with, it can simply register with this provisioned semantics node and build the parent-child relationship (step 508, step 512). Otherwise, the semantics node needs to discover the existing higher level semantics nodes, and choose one of them to register with (step 510). The choice may be based on a criterion such as nearest in the adjacent upper level, supporting the same type(s) of semantics resources, and the like. FIG. 38B illustrates these step 508, step 510, and step 512 in more detail.

In the present example, at each level, there may be one semantics node discovery server, which accepts a higher-level semantics node discovery request by default. The address of the semantics node discovery server may be well known to the lower level semantics nodes. As shown in FIG. 38B, if the new semantic node is not provisioned with a higher level parent semantic node address (step 602), and if the new semantics node is not provisioned with the semantics node discovery server in the upper level (step 604), it can firstly perform sibling semantics node discovery 606. As part of sibling discovery, the address of the semantics node discovery server may be shared (e.g., piggybacked in the sibling discovery response received from a discovered sibling semantic node) (step 608). On the other hand, it may also explicitly request the siblings' parent semantics node information (address) such that it may choose one as its own parent to which to register (steps 610, 618).

If the new semantics node is provisioned with the upper level semantics node discovery server's address, it can perform the semantics node discovery directly (control passes from step 604 to step 614). Otherwise, it decides whether it wants to choose from the siblings' parent list and whether it wants to retrieve the default semantics node discovery server's address from siblings (steps 608 and 610). If the new semantics node chooses an upper-level semantics node from the siblings' parents (step 618), it may choose not to perform the semantics node discovery further. Otherwise, it decides the criteria of choosing the parent (nearest in hop count, supporting the same type(s) of semantics resources, etc.) (step 614). Based on the criteria, it sets up the information it wants to discover in addition to the semantics nodes' addresses in the upper level (distance, supported type(s) of semantics resources, etc.) (step 616).

At step 620, the new semantic node may register to the higher level parent semantics node it discovered or otherwise selected. If the new semantic node is unable neither to learn the address of a higher-level semantic node discovery server nor to identify any higher-level parent semantic nodes of its siblings to which it might register, it may determine that no higher level parent semantic nodes are available and end the process at step 612.

Referring back to FIG. 38A, once discovery of siblings and discovery and registration with a parent node is complete, the information about the new semantic node's siblings and parent is stored (step 514). As further illustrated in FIG. 38A, if new siblings join the network or an existing sibling goes offline, the semantic node may update it semantic node relationships (steps 516, 518). At step 520, the new semantic node is now operational. As further illustrated in FIG. 38A, the operational semantic node may at some later point be triggered to register to another higher level node (step 522) of it may leave the network (step 526). In the former case, the semantic node may deregister with its current parent and register with the new one (step 524). In the latter case, the semantic node may simply deregister with its current parent (step 528).

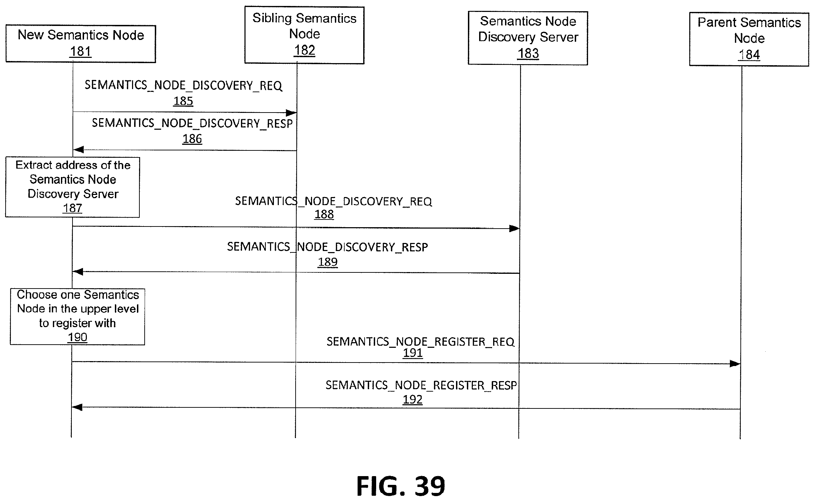

FIG. 39 is a message flow diagram further illustrating the semantics node discovery and registration process discussed above and illustrated in FIG. 38A and FIG. 38B. At step 185, new semantics node 181 sends a semantics node discovery request to sibling semantics node 182. Sibling semantics node 182 is in the same network level as new semantics node 181. The semantics node discovery request of step 185 may contain a request for information (e.g., an address) with regard to semantics node discovery server 183 or information for a semantics node that is the parent (e.g., upper level) semantics node 184 of sibling semantics node 182. A request for the parent semantics node may allow new semantics node 181 to choose its own parent to register with.

Semantics node discovery server 183 may be considered a centralized point for storing information of the semantics nodes scattered in the same level or a rendezvous point to flood the discovery request in the same level of a network and collect the returned responses of semantics nodes. Semantics node discovery server 183 may be a server that resides in a network at a higher, same, or lower level network level than the network level of new semantics node 181. This example assumes that semantics node discovery server 183 is in an upper level in relation to the network level of new semantics node 181. The address of the semantics node discovery server 183 can be well known to lower level semantics nodes (e.g., sibling semantics node 182). If new semantics node 181 is not provisioned with semantics node discovery server 183, then new semantics node 181 may perform sibling semantics node discovery. If new semantics node 181 is provisioned with the address of the semantics node discovery server 183, it can perform the semantics node discovery directly.

At step 186 of FIG. 39, sibling semantics node 182 sends a semantics node discovery response. The semantics node discovery response may contain information (e.g., address information) for semantics node discovery server 183 or information for a semantics node that is the parent of sibling semantics node 182. Each sibling node in a level of a network may respond with parent semantics node information and semantics node discovery server information, which may be different from the information provided by sibling semantics node 182.

At step 187, new semantics node 181 extracts the received address of semantics node discovery server 183. At step 188, new semantics node 181 sends a semantics node discovery request to semantics node discovery server 183. The request at step 188 may be a query for one or more parent semantics nodes that new semantics node 181 may connect with. At step 189, semantics node discovery server 183 sends a semantics node discovery response to new semantics node 181. The response at step 189 may contain one or more parent semantics nodes. At step 190, new semantics node 181 chooses one parent semantics node to register with. At step 191, semantics node 181 sends a request for registration with its chosen parent semantics node 184. At step 192, parent semantics node 184 sends a response to the request for registration at step 191.

Generally, if new semantics node 181 is provisioned with the address of semantics node discovery server 183, it can perform the semantics node discovery directly. Otherwise new semantics node 181 decides whether it wants to choose from the list of parent semantics node received from one or more siblings and whether it wants to retrieve the default address of semantics node discovery server 183 from siblings. At each level, there may be one or more semantics node discovery servers, which accepts the higher-level semantics node discovery request by default. If new semantics node 181 chooses an upper-level semantics node from the siblings' parents, new semantics node 181 may choose not to perform the semantics node discovery further. New semantics node 181 may have the option to decide the criteria of choosing the parent (e.g., choosing from options, such as nearest in hop count, supporting the same type(s) of semantics related resources, etc.). Based on the criteria, new semantics node 181 sets up the information it wants to discover in addition to the addresses of semantics nodes in the upper level (e.g., distance, supported type(s) of semantics related resources, etc.).

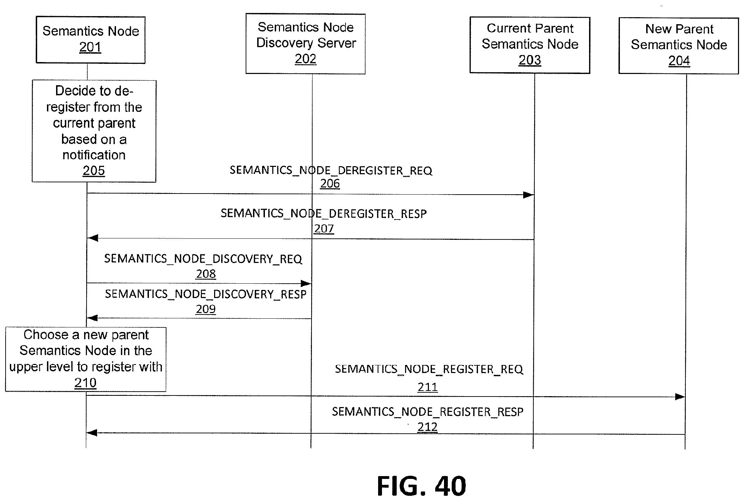

FIG. 40 provides a message flow providing further details of a parent-child relationship update process (e.g., steps 522 and 524 of FIG. 38A), in accordance with one example thereof. The update may be initiated by a child or parent semantics node. At step 205, semantics node 201 decides to de-register from current parent semantics node 203 based on a notification. The notification may be a message from current parent semantics node 203 to initiate de-registration, a determination that current parent semantics node 203 is not reachable (e.g., offline, disconnected, or other communication issue), or a received status update associated with parent semantics node 203 (e.g., network traffic congestion, device or line errors, memory capacity issues, etc.), among other things.

At step 206, semantics node 201 sends a de-register request to current parent semantics node 203, which includes a request to end the parent-child relationship. At step 207, semantics node 201 may receive a response to the de-register request sent in step 206 from current parent semantics node 203 or another device that is able to communicate a perceived status of current parent semantics node 203. Similar to the steps illustrated with regard to FIG. 39, semantics node 201 tries to register with a new parent semantics node. At step 208, semantics node 201 sends a semantics node discovery request to semantics node discovery server 202. At step 209, semantics node discovery server 202 sends a semantics node discovery response to semantics node 201. At step 210, semantics node 201 chooses one upper level semantics node to register with. At step 211, semantics node 201 sends a request for registration with its chosen new parent semantics node 204. At step 212, new parent semantics node 204 sends a response to the request for registration at step 211, which confirms the update of the parent-child relationship.

In an example (not shown, but with reference to elements in FIG. 40), a parent semantics node may trigger the de-registration of a child and provide a new parent semantics node for the child to register with. This new parent information may be included in a de-registration trigger message or alternatively in a separate trigger message. Semantics node 201 can register to new parent semantics node 204 by having current parent semantics node 203 send the registration request to new parent semantics node 204. Current parent semantics node 203 has the option to forward information of semantics node 201 to new parent semantics node 204 for registration purposes. Current parent semantics node 203 or semantics node 201 may terminate the parent-child relationship before switching current parent semantics node 203 to new parent semantics node 204.

Generally, parent-child relationships of semantics nodes are terminated by de-registering to the current parent semantics node when the child semantics node goes offline or when the child semantics node registers to another higher level parent semantics node.

If a neighboring sibling semantics node joins a network, the corresponding sibling information is updated by adding the new semantics node. If a neighboring sibling semantics node leaves a network, the corresponding sibling information is updated by deleting the semantics node or otherwise updating a table to indicate the status of the sibling semantics node that left the network (e.g., status = offline). A semantics node may broadcast or otherwise communicate its status to sibling semantics nodes using, for example, the SEMANTICS_NODE_STATUS_NOTIFY() message shown above in Table 14. The status update may affect how a sibling and parent-child relationships are maintained.

### B. Semantics Related Resource Discovery, Retrieval, and Validation

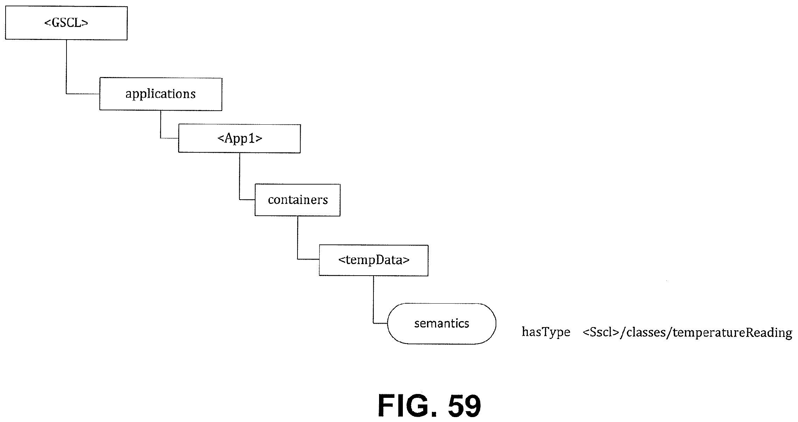

An application, a device, a user, a peer semantics node, an external semantics node, or a non-semantics node may send semantics related resource discovery requests to a semantics node through the sIc, sIs, and sIe reference points. The discovery request message may include the type of the semantics related resource (class, relationship, or term) and search string(s). For example, assume a temperature sensing application (Appl) in an M2M system that needs to report its temperature reading data - as an integer in units of Celsius - to its M2M gateway. In order to enable the gateway service capability layer (GSCL) to understand the data, App1 needs to associate the data with proper semantics information. In accordance with the procedures described herein, App1 may discover a semantic node that stores a semantic related resource class - the temperatureReading class - that represents temperature data as an integer in units of Celsius. After discovery, App1 will retrieve the representation of the temperatureReading class, and validate that it is the one it wants to use to provide semantics for its temperature data. It will then link the data with the temperatureReading class as one of its attributes (semantics attribute). In the GSCL, the data may be stored under a <tempData> container for App1, which will have a semantics attribute that links to the temperatureReading class using a hasType relationship - as illustrated for example in FIG. 59. As a result, all the App1 data stored under the <tempData> container in the GSCL will have the same semantics - that each item of data is an integer and has the unit of Celsius. As another example, App1 may be an application which retrieves a resource from an NSCL. The resource may have a semantics attribute which links to the temperatureReading class (similar to the above example). In order to understand and interpret the data in a container of the resource, App 1 will need to retrieve the semantics-related resource to which the semantics attribute of the resource is linked - in this case the temperatureReading class. After App1 retrieves the representation of the temperatureReading class semantics-related resource, it will be able to interpret the resource data - which it now knows is an integer and has the unit of Celsius.

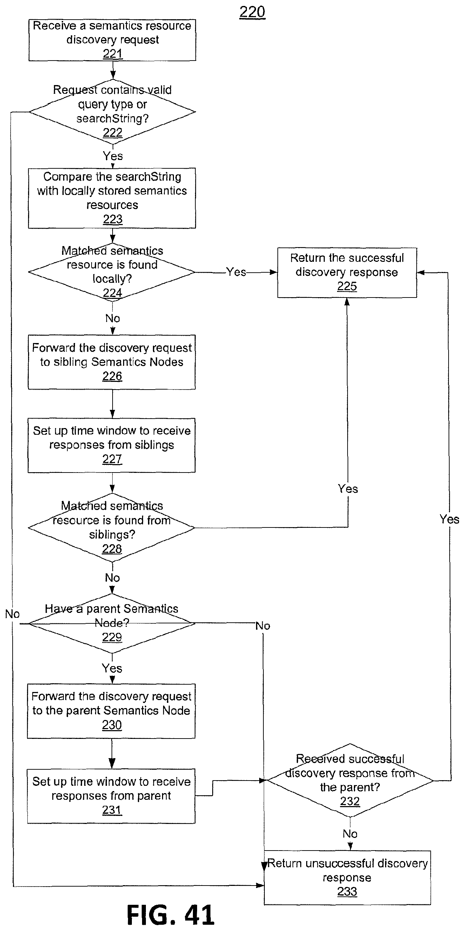

FIG. 41 is flow diagram illustrating the processing of a semantics related resource discovery request at a semantics node, in accordance with one example. At block 221, a semantics node receives a semantics related resource discovery request that includes the type of the requested semantics related resource and potential search string(s). At block 222, the semantics node checks the discovery request. If the request is insufficient or malformed (e.g., missing type of requested resource) the discovery request is considered invalid and an invalid discovery response will be returned to the issuer (e.g., requesting client device) as indicated by block 233. If the request is valid, as indicated by block 223, the search string is compared with locally stored semantics related resources. In particular, based on the type of the requested semantics related resource, the semantics node is able to determine which type (i.e., class, relationship, or term) of semantics related resource on which it will search. As indicated by block 224, using the search string(s) as a key word(s), the semantics node searches its local semantic database to find one or more matching semantics related resources. If there is a matching semantics related resource found locally, the address (e.g., URL/URI) of the semantics related resource is returned to the issuer in a discovery response, as indicated by block 225.

If there is not a matching semantics related resource found locally, the semantics node will try to find a matching semantics related resource from its sibling semantics nodes. As indicated by block 226 and block 227, the semantics node forwards the discovery request to the siblings and sets up a time window it will wait for the response to come back. At block 228, it is determined whether a matching semantics related resource is found from the contacted siblings. If matching semantics related resources are returned from its siblings, the corresponding address (e.g., URI/URL) of the semantics related resource(s) is sent back to the issuer with a successful discovery response (block 225).

If there are no matching semantics related resources returned from siblings of the semantics node, then it is determined whether a parent semantics node can be contacted, as indicated by block 229. If there is no parent semantics node, then a discovery response indicating a negative outcome will be returned to the issuer (block 233). If there is a parent semantics node, the semantics node will try to find a matching semantics related resource from its parent semantics nodes. As indicated by block 230 and block 231, respectively, the semantics node forwards the discovery request to the parent semantics node and sets up a time window it will wait for the response to come back. At block 232, it is determined if a matching semantics related resource is found from the contacted parent. If matching semantics related resources are returned from the contacted parent, the corresponding address (e.g., URI/URL) of the semantics related resources is sent back to the issuer with a successful discovery response (block 225). If there are no matching semantics related resources returned from the parent of the semantics node, then a discovery response indicating a negative outcome will be returned to the issuer (block 233).

After the issuer receives a successful discovery response, which contains the address (e.g. URL/URI) of the matching semantics resource, the issuer can retrieve the representation of the semantics resource.

In one example, the semantics node may support a RESTful architecture style (representational state transfer), which consist of clients and servers. Clients (e.g., Issuers) initiate semantic requests to servers (e.g., semantics nodes). In this example, servers (e.g., semantics nodes) process requests for semantics and return appropriate semantic responses. Requests and responses are built around the transfer of representations of semantics related resources. A client can be an application, a user, a device, a semantics node, etc., which can request RESTful operations on the semantics related resources (e.g., classes, relationships, or terms) to a semantics node.

When handling resources in a RESTful architecture, there are four basic methods that may be applied to the semantic related resources:
- CREATE: Create a class, relationship, or term resource.
- RETRIEVE: Read the content of a class, relationship, or term resource.
- UPDATE: Write the content of a class, relationship, or term resource.
- DELETE: Delete a class, relationship, or term resource.

A semantics node acting as a RESTful server may validate a received request. The operation is allowed, if the issuer is authorized with the proper access rights.

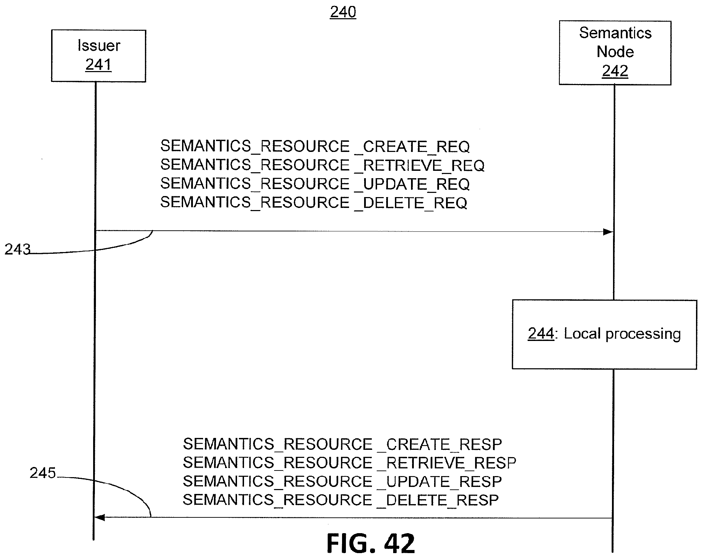

FIG. 42 is a message flow diagram further illustrating these RESTful semantic node operations in accordance with this RESTful example. At step 243, issuer 241 requests to create, update, retrieve, or delete a semantics related resource (a class, relationship, or term) using the RESTful CREATE, UPDATE, RETRIEVE, or DELETE verbs correspondingly. Issuer 241 may be an application, another semantics node, a device, a user, or the like. To create a semantics related resource at step 243, issuer 241 issues a CREATE request, providing the type and representation of the semantics related resource. To update a semantics related resource at step 243, issuer 241 issues a UPDATE request, providing the unique identity or address and the updated or partially updated representation of the semantics related resource. To retrieve a semantics related resource at step 243, issuer 241 issues a RETRIEVE request, providing the unique identity or address of the semantics related resource and optionally a searchString parameter(s). To delete a semantics related resource at step 243, issuer 241 issues a DELETE request, providing the unique identity or address of the semantics related resource.

At step 244, semantics node 242 acts as a server and validates and otherwise processes the received request. The received request is allowed, if issuer 241 is authorized with the proper access rights. If the create operation is allowed by the semantics node 242, the new semantics related resource is created under the proper resource pool based on whether it is a class, relationship, or term. And the semantics related resource is assigned a unique identity or address by semantics node 242. If the update operation is allowed by semantics node 242, the representation of the semantics related resource is updated. If the retrieve operation is allowed by semantics node 242, the representation of the semantics related resource is prepared in the format issuer 241 requests. If the delete operation is allowed by semantics node 242, the requested semantics related resource is deleted.

At step 245, semantics node 242 returns a response to issuer 241. For a create operation, the identity or address of the newly created semantics related resource is returned to the issuer. For an update operation, a response code is returned to issuer 241 whether the operation is successful or not. For a retrieve operation, the representation of the semantics related resource in a format issuer 241 requests are returned to issuer 241. For a delete operation, a response code is returned to issuer 241 to indicate whether the operation is successful or not.

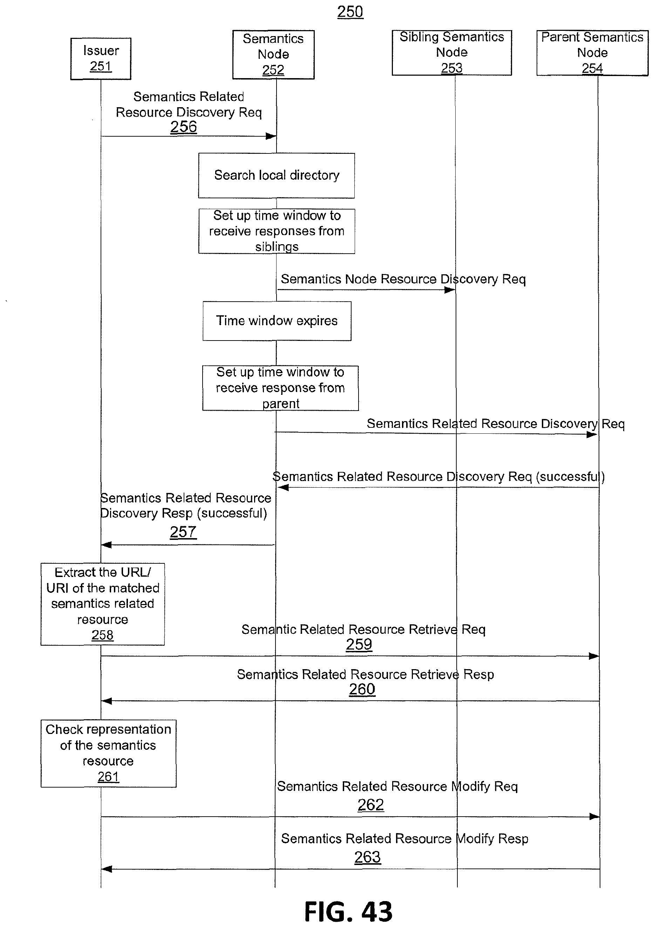

FIG. 43 is a message flow 250 further illustrating the semantics related resource discovery, retrieval, and validation process described herein. In this example, a network may contain issuer 251, semantics node 252, a sibling semantics node 253 which is a sibling of semantics node 252, and a parent semantics node 254 which is a parent to semantics node 252. At step 256, issuer 251 sends a semantics related resource discovery request to semantics node 252. As shown in message flow 250, semantics node 252 goes through several steps (similar to the process in FIG. 41) to find a semantics related resource(s) that matches the request at step 256. As illustrated, first the semantics node 252 will search its local directory. If it does not find any matching resources, it will set up a time window and forward the discovery request to its siblings, such as sibling 253. If no responses are received from its siblings, the semantics node 252 may forward its request to parent semantics node 254. In this example, it is assumed that the parent semantics node 254 does find a matching resource, and it will send a response back to the semantics node 252 indicating the address (e.g., URI/URL) of the semantics related resource(s) that it found.

At step 257, semantics node 252 then sends a semantics related resource discovery response back to the Issuer 251, which includes the address (e.g., URI/URL) of the semantics related resource(s) from the parent semantics node 254 that matched the Issuer's request. At step 259, issuer 251 sends a semantics related resource retrieve request based on the received URL. At step 260, parent semantics node 254 sends a semantics related resource retrieve response that contains the requested semantics information which may include a class, relationship, or term.

At step 261, issuer 251 checks (validates) the representation of the received semantics information from step 260. There is a possibility that the received semantics related resource sent at step 260 is not exactly what issuer 251 wants. For example, if issuer 251 requested a temperature class and the returned matching resource is a class called *temperatureReading* with an associated unit of Fahrenheit, but issuer 251 desires a temperature class with unit of Celsius, then issuer 251 can request that parent semantics node 254 modify the semantics. This can be supported by sending a semantics related resource modify request at step 262 to parent semantics node 254 to modify the semantics related resource. At step 263, the address (e.g., URL/URI) of the newly added or modified semantics related resource will be returned to issuer 251.

With reference to modification of semantics related resources, generally, the semantics node may collaborate with its siblings or parent to perform the modification, if the semantics node does not support modification itself. If the semantics node supports modification, then the semantics node may modify the class locally by adding a new class or extending the current one.

### C. Subscribing to Semantics Related Resources

In one example, a semantics node can support a client (e.g., an application, another semantics node, a device, a user, or the like) subscribing to it. As one example, a client may subscribe to the semantic node to be notified when any update to a subscribed semantic related resource. When an update occurs, the subscribing client will be notified with the new representation of the resource. In the case of a client that is a semantic node itself, the subscribed semantics related resource might be stored in another semantics node that the subscriber semantics node has no relation with (e.g., not a parent-child or sibling). In this example, a client may issue a SEMANTICS_RESOURCE_SUBSCRIBE_REQ message to a semantics node. The message identifies the semantics related resource for which the client wishes to receive notifications when the resource is updated. The semantics node will respond to the request with a SEMANTICS_RESOURCE_SUBSCRIBE_RESP message acknowledging the subscription. When the semantics related resource to which the client has subscribed is updated, the semantics node will send a SEMANTICS_RESOURCE_SUBSCRIBER_NOTIFY message to notify the client of the update.

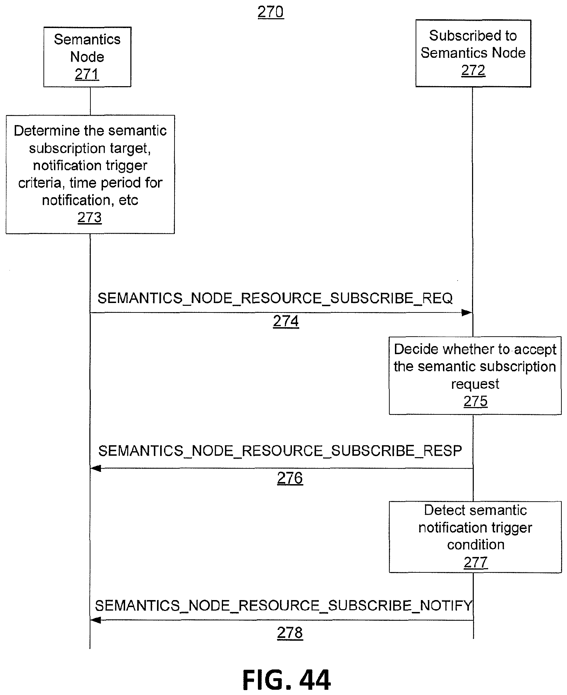

As another example, a semantics node may be interested in being updated with the semantics related resources stored and managed by one of its sibling, parent, or child semantics nodes. FIG. 44 is an exemplary flow 270 of a subscription/notification process for this situation, in accordance with one example. In this example, a semantics node 271 is updated with the semantics related resources stored and managed by a subscribed-to semantics node 272. Subscribed-to semantics node 272 may be a sibling, parent, or child of semantics node 271. At step 273, semantics node 271 identifies the subscription target and may set up notification trigger criteria such that it only receives semantics related resource notifications relevant to the trigger criteria. For example, the subscriber may configure the notification trigger criteria to specify a particular semantics related resource or a particular number of updates to a semantics related resource before a new notification is sent. Semantics node 271 may also set up the time period scheduling information for when the notification should be sent.

At step 274, semantics node 271 sends a semantics node resource subscribe request to the target semantics node 272. At step 275, the target semantics node 272 determines whether to accept the semantics subscription request of step 274. to the target semantics node 272 can decide whether to accept a subscription request, based on the existing subscribers, load of handling the subscription (e.g., load with regard to collecting update information or bandwidth used to send a notification), etc. At step 276, the target semantics node 272 sends a semantics node subscription response to semantics node 271. The response at step 276 may include confirmation of the subscription and parameters that will be used in processing the subscription. At step 277, at some point in time after step 276, the target semantics node 272 detects a semantic notification trigger condition that matches the requested trigger received at step 274. At step 278, the target semantics node 272 sends a semantics node resource subscribe notify message to update semantics node 271 with regard to a particular semantics related resource.

Generally, a semantics related resource subscription can facilitate the semantics related resource discovery from a peer semantics node or parent semantics node. For example, based on the notification messages (which would include the URIs of newly created or updated semantics related resources stored on a semantics node), a semantics node may be able to perform discovery on semantics related resources of other nodes without sending discovery requests.

### D. Linking and Association to Semantics Related Resources

The semantics related resources of a semantic node can be used in various ways. For example, semantics related resource representations may be retrieved from a semantics node and may be stored in a co-located manner in a network location where the data is stored (e.g., on a network server, on a device, on a gateway, etc.). Alternatively, the semantics of a resource may be stored on a semantic node and a link to the semantics may be co-located and stored along with the data. This semantic link may be stored in-line (i.e., embedded) within the data or may be stored separately (e.g. in a separate resource or attribute) alongside the data. Thus, through this linking to semantics-related resources, semantics can be applied to normal resources in an M2M system (e.g., <SCL>, <application>, <container> etc.). Typically, this linking will be created by the resource creator/generator, when the resources are created.

Continuing with the earlier example of a patient health monitoring application in FIG. 1, there are semantic classes defined on a semantics node and the URLs of these classes may be discovered by a patient health monitoring application. Table 17 shows examples of semantics related resources of type "class" that are relevant to the patient health monitoring application. Data may be linked to these semantic classes using a semantic relationship called ***hasType.*** As a result, for each data resource with a URI of example/healthmonitoring/datal, the semantics of the resource will be known by the following associations:

| | | |
|---|---|---|
| • example/health/patient/datal | ***hasType*** | semanticsNode1/class/patient |
| • example/health/doctor/data2 | ***hasType*** | semanticsNode1/class/doctor |
| • example/health/bp/data1 | ***hasType*** | semanticsNode1/class/bloodpressure |
| • example/health/temp/data1 | ***hasType*** | semanticsNode1/class/temperature |
| • example/health/hr/data5 | ***hasType*** | semanticsNode1/class/heartrate |

The ***hasType*** relationship may also be identified by a URL/URI which references a semantic description of the ***hasType*** relationship stored on the semantic node.

**Table 17: Example of Class Semantics Related Resources**

| Class | Fields | URL |
|---|---|---|
| Patient | Name: String | semanticsNode1/class/patient |
| | Sex: F/M | |
| | Address: String | |
| | Disease: String | |
| Doctor | Name: String | semanticsNode1/class/doctor |
| | Affiliated hospital: String | |
| | Specialty: String | |
| Blood_Pressure | Systolic Pressure: mmHg | semanticsNode1/class/bloodpressure |
| | Diastolic Pressure: mmHg | |
| Temperature | Unit: Fahrenheit | semanticsNode1/class/temperature |
| Heart Rate | Unit: bpm | semanticsNode1/class/heartrate |
| ... | | |

### E. Grouping Optimization

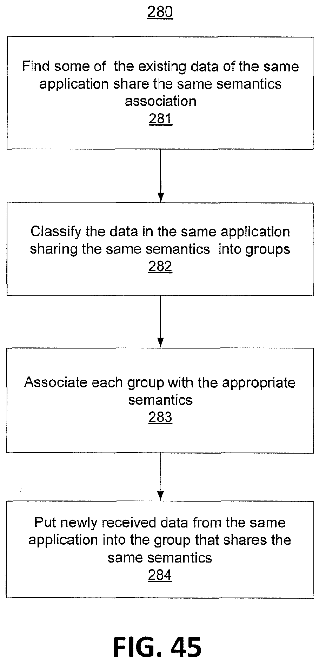

If a group of resources have some similar semantics (e.g., all resources in the same application have the same semantics), the similar semantics may be applied to the application, instead of to each individual resource under that application. FIG. 45 illustrates a method 281 of grouping of resources with the same semantics, in accordance with one example thereof. At step 281, some of the existing data of the same application is determined to share the same semantics association. At step 282, the determined data in the same application sharing the same semantics is classified into groups. At step 283, each group of step 282 is associated with the appropriate semantics. At step 284, newly received data from the same application is put into the group that shares the same semantics. The existing data of the same application may be classified into multiple groups, each of which shares the same semantics linking and association. If new data is generated from the same application, the data is put into the group that shares the same semantics.

For example, multiple instances of blood pressure monitoring data have the same semantics. Hence each instance may be associated with the same semantics (semanticsNodel/class/bloodpressure) as shown below:
- example/health/bp/datal ***hasType*** semanticsNodel/class/bloodpressure
- example/health/bp/data2 ***hasType*** semanticsNodel/class/bloodpressure
- example/health/bp/data3 ***hasType*** semanticsNodel/class/bloodpressure By supporting this grouping optimization, the following association may also be valid:
- example/health/bp ***hasType*** semanticsNodel/class/bloodpressure

### F. Pushing Semantics Related Resources

As mentioned above, the classes, relationships, and terms that are hosted in a semantics node may be discovered and used by others. Based on the frequency of the requests, some of the semantics related resources may be pushed or mirrored in another semantics node for easier discovery and access. For example, after one semantics node detects the same forwarded discovery request from another semantics node a certain number of times (e.g., exceeding a policy defined threshold); it may decide to push a mirrored copy of the semantics related resource to that semantics node.

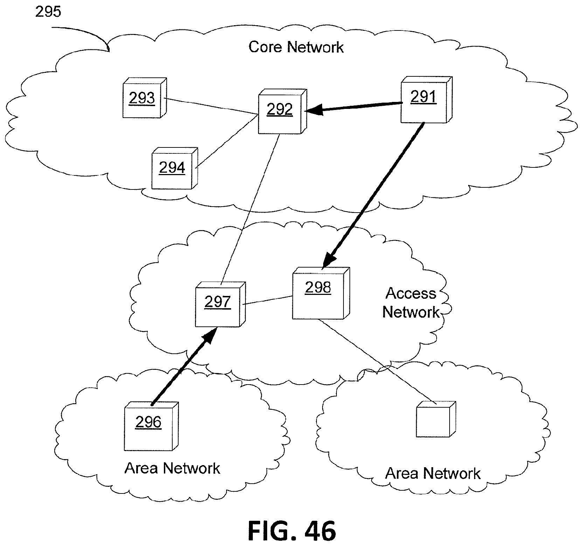

The semantics related resource pushing can happen between siblings, or between parent and child semantic nodes, as shown in FIG. 46. For example, semantics node 291 of FIG. 46 in the core network 295 may receive many discovery and retrieval requests for the same semantics related resource (e.g., temperature) from semantics node 292, semantics node 293, and semantics node 294. When discovery requests reach a defined threshold, semantics node 291 may decide to create the same semantics related resource (i.e., mirror the resource) on semantics node 292, such that semantics node 293 and semantics node 294 may access the semantics related resource with a faster response time. The semantics node 291 may create the mirrored resource on the semantics node(s) by issuing a SEMANTICS_RESOURCE_CREATE_REQ message to the other semantics nodes, which would then respond with an appropriate SEMANTICS_RESOURCE_CREATE_RESP message.

The following options may be used in order to keep the semantics related resource up-to-date. With reference to FIG. 46, semantics node 291 may automatically update a semantics related resource on semantics node 292, if there is any update to the original representation of the semantics related resource (e.g., a subscription is not needed). Alternatively, semantics node 292 may subscribe to the original semantics related resource. Based on the subscription of semantics node 292 to the semantics related resource, semantics node 292 will be notified of any change to the particular subscribed to semantics related resource. There also may be a combination of the aforementioned scenarios. For example, there may be a situation where the automatic updates of semantics node 291 occur periodically for all semantics related resources on semantics node 291, while semantics node 292 desires a more immediate update for particular subscribed to semantics related resources..

The semantics related resource pushing can happen between siblings, or between a parent and child in either direction. For example, with reference to FIG. 46, semantics node 296 may push some local semantics related resource to its parent semantics node 297. In another example, semantics node 291 may push some higher-level semantics related resource to its child semantics node 298.

### G. Data/Semantics Related Resources Movement

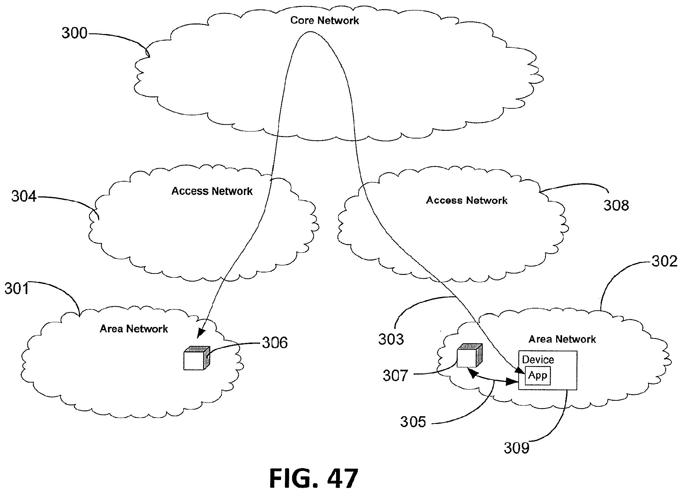

FIG. 47 illustrate a scenario in which a device moves from one network to another. In this scenario, the semantics resources relevant to the device and the data generated by the device may also need to be moved to the new location for security, overhead and/or loading due to the semantics resource retrieval.

Referring to FIG. 47, the device initially may have been located in area network 301, but has now moved to area network 302. Initially, device 309 communicated with semantics node 306. After device 309 arrives at area 302, device 309 may initially continue to communicate to semantics node 306, as demonstrated by line 303. This can result in unnecessary overhead in access network 304, access network 308, and core network 300. To address this and other issues, semantics related resources of semantics node 306 may be moved to semantics node 307 in area network 302. After moving semantics related resources to semantics node 307, device 309 does not need to traverse core network 300 for the semantics related resources but instead can now communicate with semantics node 307 as indicated by line 305.

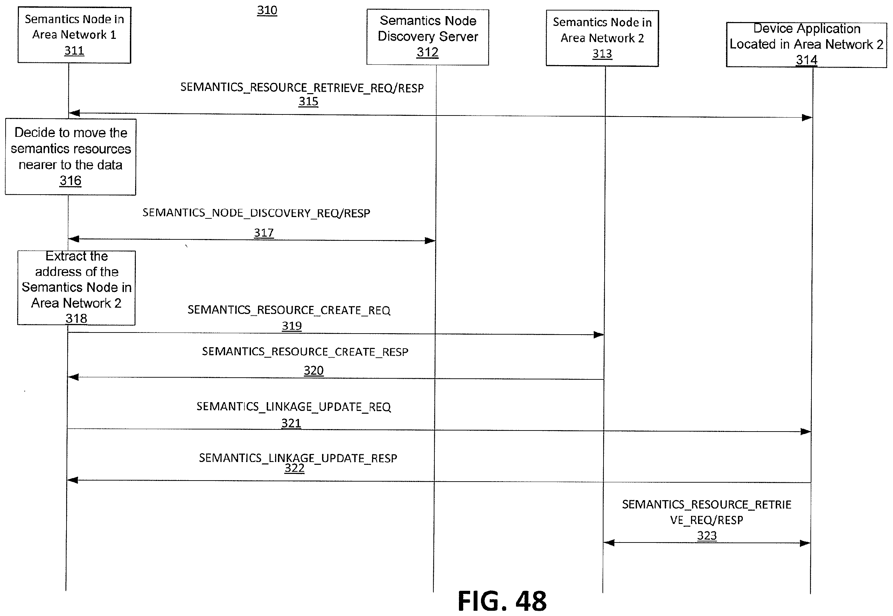

FIG. 48 is an example message flow 310 further illustrating the movement of data or semantics related resources as depicted in FIG. 47. At step 315, semantics node 311 in a first area network exchanges messages with device application 314 located in area network 2. The messages exchanged at step 315 may include, for example, a semantics related resource retrieve request and semantics related resource retrieve response. At step 316, semantics node 311 decides to move the semantics related resources associated with device application 214 to semantics node 313 located in a second area network. Semantics node 313 may be communicatively closer (takes less time to reach) than the first area network or logically closer (e.g., less hops). With reference to step 316, other devices may make the decision to move the semantics related resources, such as semantics node discovery server 312, device application 314, or another computing device (not shown).

At step 317, semantics node discovery requests and responses are exchanged between semantics node 311 and semantics node discovery server 312 in order to build the hierarchy of semantics nodes, as well as sibling relationships. At step 318, semantics node 311 determines the address of semantics node 313. At step 320, a semantics related resource create request message is sent to semantics node 313 in order to copy the semantics related resource (and other data) that is used by device application 314. Semantics node 313 responds with a semantics related resource create response message, which may include an acknowledgement that the semantics related resource and other data has been copied successfully. At step 321, a semantics linkage update request message is sent to device application 314. The message at step 321 may include instructions for device application 314 to retrieve semantics related resources from semantics node 313. At step 322, the semantics linkage update response message may include an acknowledgement that the semantics node linkage is updated. At step 323, device application 314 retrieves semantics related resources in types of class, relationship, and term from semantics node 313.

### V. ETSI M2M/oneM2M Examples

### A. ETSI M2M Architecture with Semantics Node

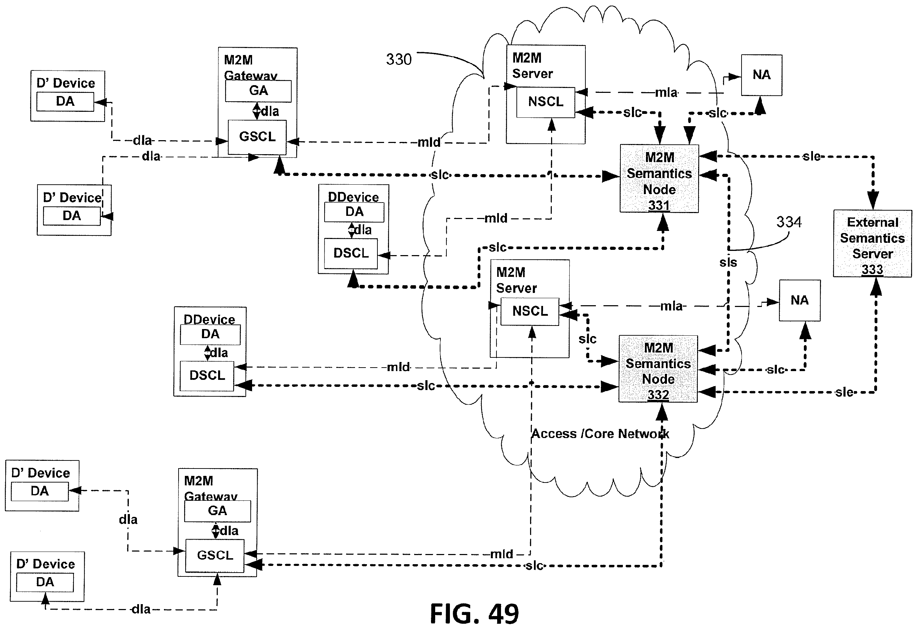

As mentioned above, the semantic node concept described herein can be used to enhance the ETSI M2M architecture. Note the examples below (e.g., FIG. 49-FIG. 55) are ETSI examples; these may be reasonably converted/implemented in oneM2M architecture as well. In one example, one or more semantics node may be located in the access/core network as a stand-alone network entity, which may be referred to as a M2M semantics node as shown in FIG. 49. In FIG. 49, M2M semantics node 331 and M2M semantics node 332 are located in the same access/core network 330. M2M semantics nodes in area/core network 330 may interface with DSCLs, GSCLs, NSCLs, and applications via the sIc reference point described above. In addition, M2M semantics node 331 and M2M semantics node 332 may interface with one another via the sIs reference point 334. M2M semantics node 331 and M2M semantics node 332 may also reference to another type of external semantics node 333 via sIe reference points. In this example, M2M semantics nodes located in the access/core network 330 may also form sibling and/or parent-child relationships.

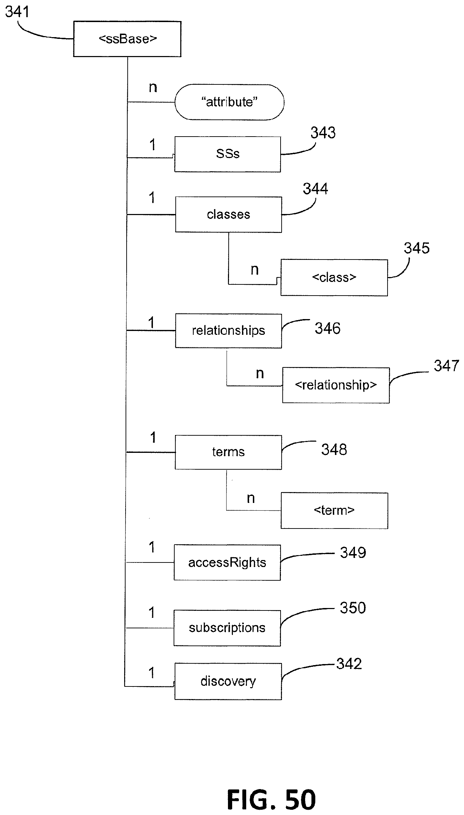

A semantics node may support a complementary resource structure as used in the service capability layer (xSCL) of the current ETSI M2M Architecture, and this resource structure may be applied to the semantics node described herein, in the manner illustrated in FIG. 50. In this example, the <ssBase> resource 341 is the root of the resource tree residing on the hosting semantics node. The <ssBase> resource 341 may contain attributes that describe the hosting semantics node. The <ssBase> resource 341 contains collection resources representing collections of SS resources 343, classes resources 344, relationships resources 346, terms resources 348, accessRights resources 349, and subscriptions resources 350, among others. Under a classes resource 344 there might be other <class> resources 345, which are the subclasses of the classes resource 344. Under a relationship resource 346 there might be other <relationship> resources 347, which are the sub-relationships of the relationship resource 346. The SSs resource collection 343 contains semantics node resources that are created or deleted when a remote semantics node registers or de-registers with the local semantics node.

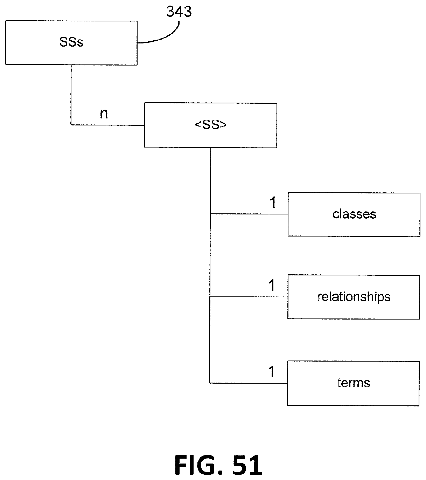

As shown in FIG. 51, each semantics node resource in a collection for SSs resource 343 may have a corresponding resource structure. These resources maintain state for remote semantics nodes registered to the local semantics node. For example, state such as contact address information, discovery information (e.g., announced semantic class, representation, and term resources), and security information (e.g., credentials used to communicate with the corresponding remote semantics node).

With reference again to FIG. 50, the classes 344, relationships 346, and terms 348 collections under <ssBase> resource 341 can each contain respective instances of semantics related resources hosted on the local semantics node. Each instance can contain a semantic representation as well as have other attributes associated with it, such as discovery related information such as tags. These collections of semantics related resources can be accessed by clients having the proper access rights to do so. The accessRights resource 349 under <ssBase> resource 341 can contain instances of accessRight resources. These accessRight resources 349 can define the instances of accessRights that control which clients are granted access to which semantics related resources and operations supported by the semantics node. Alternatively, other instances of accessRights collections can be supported in the resource structure to provide finer grain access controls (not shown in FIG. 50). The collection of subscriptions resources 350 can contain instances of subscription resources. Instances of subscription resources can be created by clients who wish to receive semantic notifications from the semantics node when specified notification trigger criteria events occur. The discovery resource 342 supports client semantic discovery requests. These discovery requests can support search criteria (e.g., semantic related resources having specific types of attributes). The semantics node can respond to discovery requests with a list of resource addresses (e.g., URIs) that match the search criteria (if any). The semantics node can also support forwarding of requests to other semantics nodes (e.g., forwarding of discovery requests to child, sibling, or parent semantics nodes).

### B. xSCL with Semantics Capability

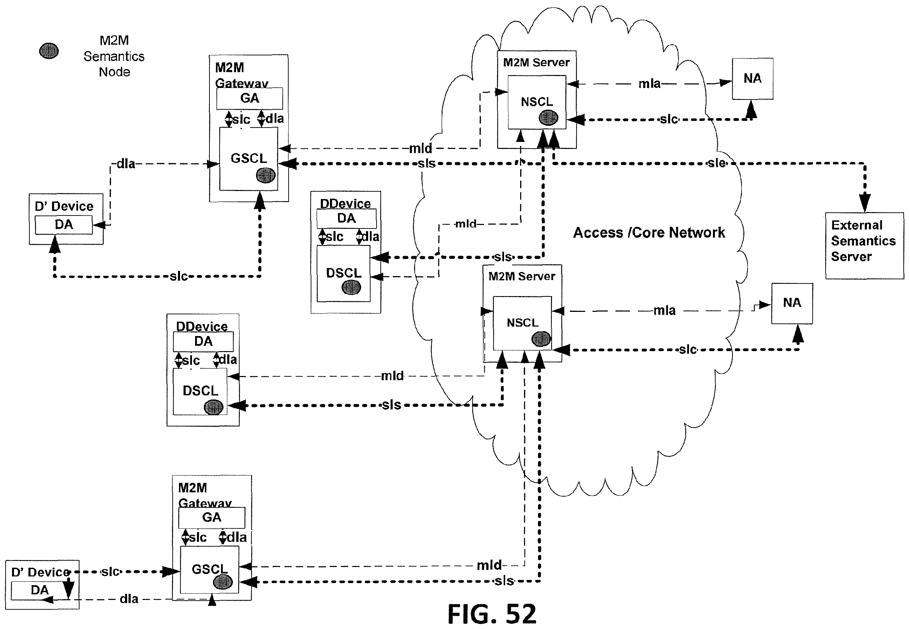

In another example illustrated in FIG. 52, an M2M semantics node may be deployed as an embedded capability within a DSCL, GSCL, and/or NSCL of the ETSI M2M architecture rather than as a separate standalone semantics node. In this embedded example, the sIs reference point may remain separate or the ETSI M2M mId reference point may be enhanced to support sIs functionality. Likewise, the sIc reference point may remain separate, or the ETSI M2M mIa and dIa reference points may be enhanced to support sIc functionality. In this example, M2M semantics nodes located in the GSCL or DSCL may establish parent-child relationships with the NSCL(s) they are registered with. In addition, the semantics nodes located in GSCLs or DSCLs may also establish sibling relationships with one another.

To support the example of FIG. 52, the xSCL may have the resource structure shown in FIG. 53. The resource collection of the semantics node contains semantics node resources that are created or deleted when a remote SCL having semantics node capability registers or de-registers with the local SCL. Each semantics node resource in this collection may have a corresponding resource structure as shown in FIG. 53. These resources maintain state for remote semantics nodes registered to the local SCL. For example, state such as semantic discovery information (e.g., announced semantic class, representation, and term resources), etc.

The classes, relationships, and terms collections under the <sclBase> resource 361 may each contain respective instances of semantics related resources hosted on the local SCL. Each instance may contain a semantic representation as well as have other attributes associated with it, such as discovery related information such as tags. These collections of semantic related resources may be accessed by clients having the proper access rights to do so.

### C. Use Case Example of ETSI M2M Semantics Implementation

The semantics related resources managed by a semantics node may be associated and linked to resources in the ETSI M2M resource structure, such as <sclBase>, <application>, <container>, <contentInstance>, and the like. The following discussion illustrates how semantics related resources may be used to provide semantics information for a <contentInstance>.

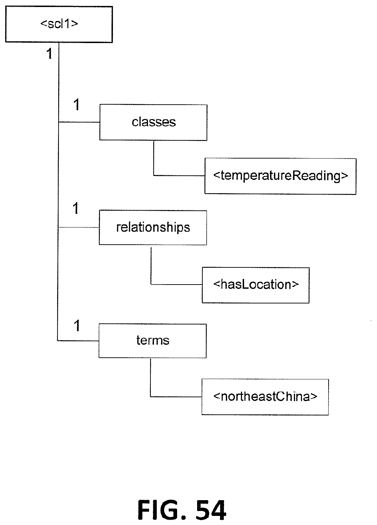

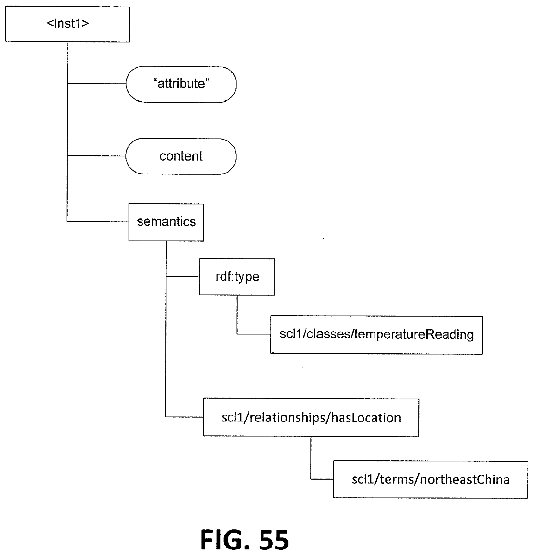

In this example, assume that a **temperatureReading** class is defined and stored on scl1, and has URI of *scl1*/*classes*/*temperatureReading.* The relationship **hasLocation** is defined and stored on sel1 as well, and has URI of *sel1*/*relationships*/*hasLocation.* Additionally, the term 'northeast China' is defined and stored on sel1 too, and has URI of *sel1*/*terms*/*northeastChina.* FIG. 54 shows the semantics related resource structure on <scll>, which is an example of the xSCL resource structure shown in FIG. 53. This resource structure determines the URIs of the semantics related resources. The contentInstance has a URI of gscl2/applications/app1/containers/<temperature>/contentInstances/ <inst1>. By enhancing the contentlnstance with the semantics as shown in the xSCL resource structure of FIG. 55, the content of the contentInstance is able to be effectively described and interpreted without ambiguity.

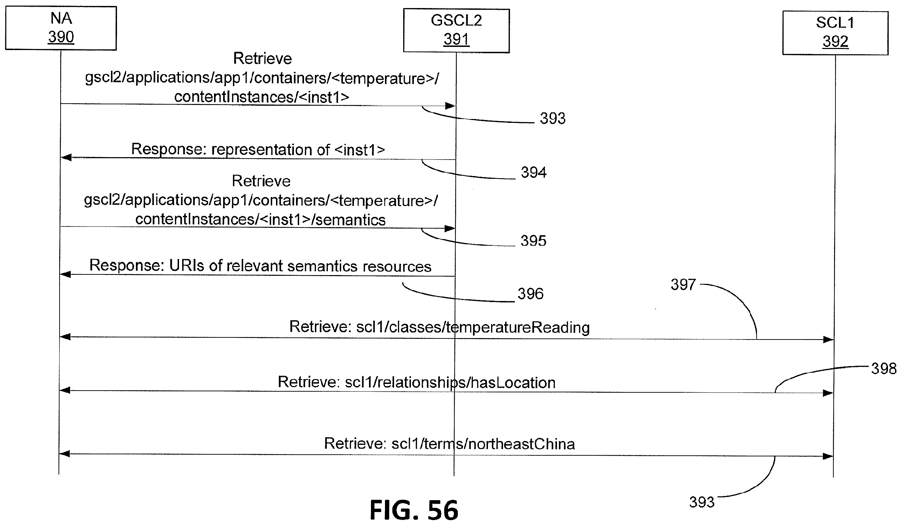

FIG. 56 is a message flow illustrating one example of resource and semantics retrieval. At step 393, NA 390 sends a RETRIEVE request to GSCL2 391 for a data resource. The data resource, for example, may be a blood pressure sensor reading, core temperature sensor reading, oxygen saturation sensor reading, or motion accelerometer sensor reading, among other things. At step 394, GSCL2 391 returns the representation of the data resource. In order to understand the data resource, the NA needs to retrieve the semantics of the data resource. Accordingly, at step 395, NA 390 sends a retrieve request to the GSCL 391. At step 396, GSCL2 391 returns a list of URIs of the relevant semantics related resources for the data resource, which are stored on SCL1 392. At step 397 through 399, NA 390 exchanges RETRIEVE messages with SCL1 392 for the semantics related resources temperatureReading, hasLocation, and northeastChina, respectively. With these semantics related resources, NA 390 is able to understand the data resources, and therefore can use and manipulate the data resources.

### VI. 3GPP MTC Architecture Examples

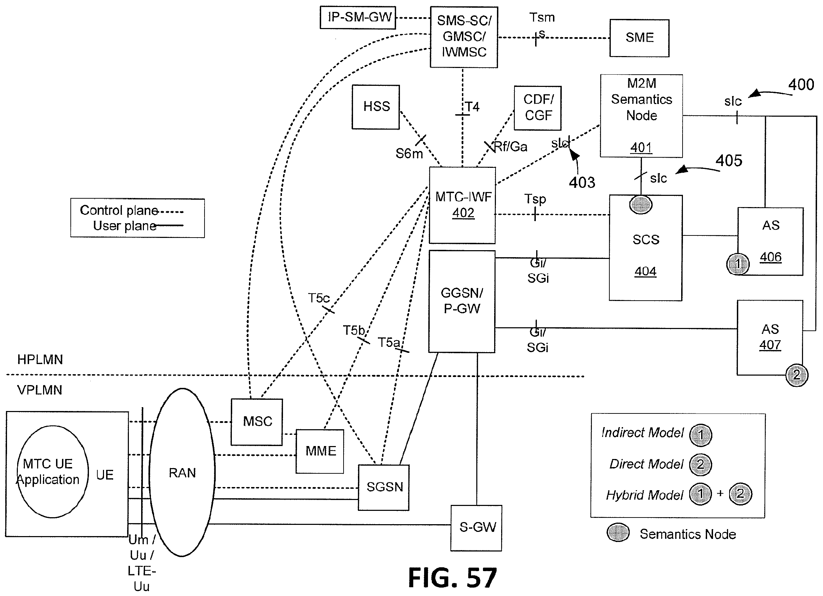

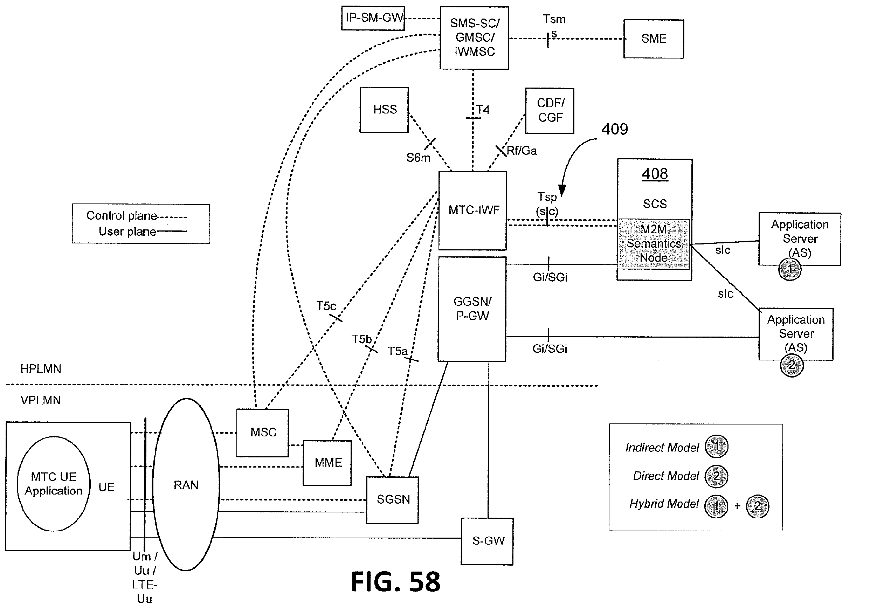

As further mentioned above, the 3GPP MTC architecture may also be enhanced with the semantics support provided by the semantics nodes described herein. As shown in FIG. 57, in one example, an M2M semantics node 401 may be located outside the 3GPP core network boundary. As further shown, SCS 404 may be enhanced to support a semantics capability and may interface with M2M semantics node 401 via the sIs reference point (not shown). M2M semantics node 401 may also interface with 3GPP machine type communication inter-working function (MTC-IWF) 402 via sIc reference point 403. Application server (AS) 406 and AS 407 may communicate with the M2M semantics node 401 via sIc reference point 400. FIG. 58 illustrates another example for a 3GPP MTC architecture with a semantics node. In this example, the semantic node has been integrated into an SCS 408. As further shown in FIG. 58 at 409, in this example the sIc referent point may be part of 3GPP MTC Tsp.

While the oneM2M, 3GPP, and ETSI M2M architectures are described by way of background herein and may be used to illustrate various examples described hereinafter, it is understood that implementations of the examples described hereinafter may vary while remaining within the scope of the present disclosure. One skilled in the art will also recognize that the disclosed examples are not limited to implementations using the 3GPP or ETSI M2M architectures discussed above, but rather may be implemented in other architectures and systems, such as oneM2M, MQTT, and other M2M systems and architectures.

FIG. 60A is a diagram of an example machine-to machine (M2M), Internet of Things (IoT), or Web of Things (WoT) communication system 10 in which one or more disclosed examples, such as FIG. 12 or FIG. 11 may be implemented. Generally, M2M technologies provide building blocks for the IoT/WoT, and any M2M device, gateway or service platform may be a component of the IoT/WoT as well as an IoT/WoT service layer, etc.

As shown in FIG. 60A, the M2M/ IoT/WoT communication system 10 includes a communication network 12. The communication network 12 may be a fixed network *(e.g.,* Ethernet, Fiber, ISDN, PLC, or the like) or a wireless network (*e.g*., WLAN, cellular, or the like) or a network of heterogeneous networks. For example, the communication network 12 may comprise of multiple access networks that provides content such as voice, data, video, messaging, broadcast, or the like to multiple users. For example, the communication network 12 may employ one or more channel access methods, such as code division multiple access (CDMA), time division multiple access (TDMA), frequency division multiple access (FDMA), orthogonal FDMA (OFDMA), single-carrier FDMA (SC-FDMA), and the like. Further, the communication network 12 may comprise other networks such as a core network, the Internet, a sensor network, an industrial control network, a personal area network, a fused personal network, a satellite network, a home network, or an enterprise network for example.

As shown in FIG. 60A, the M2M/ IoT/WoT communication system 10 may include the Infrastructure Domain and the Field Domain. The Infrastructure Domain refers to the network side of the end-to-end M2M deployment, and the Field Domain refers to the area networks, usually behind an M2M gateway. The Field Domain includes M2M gateways 14 and terminal devices 18. It will be appreciated that any number of M2M gateway devices 14 and M2M terminal devices 18 may be included in the M2M/ IoT/WoT communication system 10 as desired. Each of the M2M gateway devices 14 and M2M terminal devices 18 are configured to transmit and receive signals via the communication network 12 or direct radio link. The M2M gateway device 14 allows wireless M2M devices (e.g. cellular and non-cellular) as well as fixed network M2M devices (e.g., PLC) to communicate either through operator networks, such as the communication network 12 or direct radio link. For example, the M2M devices 18 may collect data and send the data, via the communication network 12 or direct radio link, to an M2M application 20 or M2M devices 18. The M2M devices 18 may also receive data from the M2M application 20 or an M2M device 18. Further, data and signals may be sent to and received from the M2M application 20 via an M2M service layer 22, as described below. M2M devices 18 and gateways 14 may communicate via various networks including, cellular, WLAN, WPAN (e.g., Zigbee, 6LoWPAN, Bluetooth), direct radio link, and wireline for example.

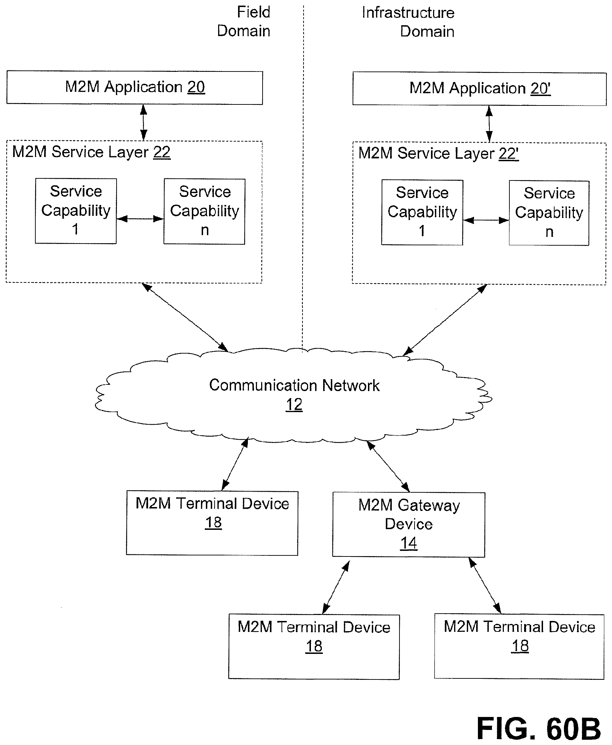

Referring to FIG. 60B, the illustrated M2M service layer 22 in the field domain provides services for the M2M application 20, M2M gateway devices 14, and M2M terminal devices 18 and the communication network 12. It will be understood that the M2M service layer 22 may communicate with any number of M2M applications, M2M gateway devices 14, M2M terminal devices 18, and communication networks 12 as desired. The M2M service layer 22 may be implemented by one or more servers, computers, or the like. The M2M service layer 22 provides service capabilities that apply to M2M terminal devices 18, M2M gateway devices 14 and M2M applications 20. The functions of the M2M service layer 22 may be implemented in a variety of ways, for example as a web server, in the cellular core network, in the cloud, etc.

Similar to the illustrated M2M service layer 22, there is the M2M service layer 22' in the Infrastructure Domain. M2M service layer 22' provides services for the M2M application 20' and the underlying communication network 12' in the infrastructure domain. M2M service layer 22' also provides services for the M2M gateway devices 14 and M2M terminal devices 18 in the field domain. It will be understood that the M2M service layer 22' may communicate with any number of M2M applications, M2M gateway devices and M2M terminal devices. The M2M service layer 22' may interact with a service layer by a different service provider. The M2M service layer 22' may be implemented by one or more servers, computers, virtual machines (e.g., cloud/compute/storage farms, etc.) or the like.

Referring also to FIG. 60B, the M2M service layer 22 and 22' provide a core set of service delivery capabilities that diverse applications and verticals can leverage. These service capabilities enable M2M applications 20 and 20' to interact with devices and perform functions such as data collection, data analysis, device management, security, billing, service/device discovery etc. Essentially, these service capabilities free the applications of the burden of implementing these functionalities, thus simplifying application development and reducing cost and time to market. The service layer 22 and 22' also enables M2M applications 20 and 20' to communicate through various networks 12 and 12' in connection with the services that the service layer 22 and 22' provide.

In some examples, M2M applications 20 and 20' may include desired applications that communicate using an ontology publishing message, as discussed herein. The M2M applications 20 and 20' may include applications in various industries such as, without limitation, transportation, health and wellness, connected home, energy management, asset tracking, and security and surveillance. As mentioned above, the M2M service layer, running across the devices, gateways, and other servers of the system, supports functions such as, for example, data collection, device management, security, billing, location tracking/geofencing, device/service discovery, and legacy systems integration, and provides these functions as services to the M2M applications 20 and 20'.

The M2M ontology management of the present application may be implemented as part of a service layer. The service layer is a software middleware layer that supports value-added service capabilities through a set of application programming interfaces (APIs) and underlying networking interfaces. An M2M entity (e.g., an M2M functional entity such as a device, gateway, or service/platform that may be implemented by a combination of hardware and software) may provide an application or service. Both ETSI M2M and oneM2M use a service layer that may contain the M2M ontology management and semantics interoperability messages of the present invention. ETSI M2M's service layer is referred to as the Service Capability Layer (SCL). The SCL may be implemented within an M2M device (where it is referred to as a device SCL (DSCL)), a gateway (where it is referred to as a gateway SCL (GSCL)) and/or a network node (where it is referred to as a network SCL (NSCL)). The oneM2M service layer supports a set of Common Service Functions (CSFs) (i.e. service capabilities). An instantiation of a set of one or more particular types of CSFs is referred to as a Common Services Entity (CSE), which can be hosted on different types of network nodes (e.g. infrastructure node, middle node, application-specific node). Further, the M2M ontology management and semantics interoperability messages of the present application can be implemented as part of an M2M network that uses a Service Oriented Architecture (SOA) and/or a resource-oriented architecture (ROA) to access services such as the M2M ontology management and semantics interoperability messages of the present application.

FIG. 60C is a system diagram of an example M2M device 30, such as an M2M terminal device 18 or an M2M gateway device 14 for example. As shown in FIG. 60C, the M2M device 30 may include a processor 32, a transceiver 34, a transmit/receive element 36, a speaker/microphone 38, a keypad 40, a display/touchpad 42, non-removable memory 44, removable memory 46, a power source 48, a global positioning system (GPS) chipset 50, and other peripherals 52. It will be appreciated that the M2M device 30 may include any subcombination of the foregoing elements while remaining consistent with an example. This device may be a device that uses the disclosed systems and methods for M2M ontology management and semantics interoperability. M2M device 30 (e.g., MOP 721, ODC 723, OPC 726, OCC 724, OR 727, gateway 704, ambient sensor 706, blood pressure monitor 701, weighing scale 702, publisher 741, and others) may be an exemplary implementation that performs the disclosed systems and methods for M2M ontology management and semantics interoperability.

The processor 32 may be a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Array (FPGAs) circuits, any other type of integrated circuit (IC), a state machine, and the like. The processor 32 may perform signal coding, data processing, power control, input/output processing, and/or any other functionality that enables the M2M device 30 to operate in a wireless environment. The processor 32 may be coupled to the transceiver 34, which may be coupled to the transmit/receive element 36. While FIG. 60C depicts the processor 32 and the transceiver 34 as separate components, it will be appreciated that the processor 32 and the transceiver 34 may be integrated together in an electronic package or chip. The processor 32 may perform application-layer programs (e.g., browsers) and/or radio access-layer (RAN) programs and/or communications. The processor 32 may perform security operations such as authentication, security key agreement, and/or cryptographic operations, such as at the access-layer and/or application layer for example.

The transmit/receive element 36 may be configured to transmit signals to, or receive signals from, an M2M service platform 22. For example, in an example, the transmit/receive element 36 may be an antenna configured to transmit and/or receive RF signals. The transmit/receive element 36 may support various networks and air interfaces, such as WLAN, WPAN, cellular, and the like. In an example, the transmit/receive element 36 may be an emitter/detector configured to transmit and/or receive IR, UV, or visible light signals, for example. In yet another example, the transmit/receive element 36 may be configured to transmit and receive both RF and light signals. It will be appreciated that the transmit/receive element 36 may be configured to transmit and/or receive any combination of wireless or wired signals.

In addition, although the transmit/receive element 36 is depicted in FIG. 60C as a single element, the M2M device 30 may include any number of transmit/receive elements 36. More specifically, the M2M device 30 may employ MIMO technology. Thus, in an example, the M2M device 30 may include two or more transmit/receive elements 36 (*e.g.,* multiple antennas) for transmitting and receiving wireless signals.

The transceiver 34 may be configured to modulate the signals that are to be transmitted by the transmit/receive element 36 and to demodulate the signals that are received by the transmit/receive element 36. As noted above, the M2M device 30 may have multi-mode capabilities. Thus, the transceiver 34 may include multiple transceivers for enabling the M2M device 30 to communicate via multiple RATs, such as UTRA and IEEE 802.11, for example.

The processor 32 may access information from, and store data in, any type of suitable memory, such as the non-removable memory 44 and/or the removable memory 46. The non-removable memory 44 may include random-access memory (RAM), read-only memory (ROM), a hard disk, or any other type of memory storage device. The removable memory 46 may include a subscriber identity module (SIM) card, a memory stick, a secure digital (SD) memory card, and the like. In other examples, the processor 32 may access information from, and store data in, memory that is not physically located on the M2M device 30, such as on a server or a home computer. The processor 32 may be configured to control lighting patterns, images, or colors on the display or indicators 42 in response to whether the M2M ontology management messages (e.g., converting an ontology to a compatible message) in some of the examples described herein are successful or unsuccessful (e.g., exception handling (EHR), SLMP, or link profile submissions, etc.), or otherwise indicate the status of OPC 726, OCC 724, OR 727, ODC 723, or other semantics associated processes and components herein. For example, an organization of ontologies on a display as similarly described herein in FIG. 20 through FIG. 23. The control lighting patterns, images, or colors on the display or indicators 42 may be reflective of the status of any of the method flows or components in the FIG.'s illustrated or discussed herein (e.g., FIG. 11 through FIG. 61, etc.). Disclosed herein are messages and procedures of M2M ontology management and semantics interoperability. The messages and procedures can be extended to provide interface/API for users to request resource-related resources via an input source (e.g., speaker/microphone 38, keypad 40, or display/touchpad 42) and request, configure, or query resources for M2M ontology management and semantics interoperability, among other things, which may be displayed on display 42.

The processor 32 may receive power from the power source 48, and may be configured to distribute and/or control the power to the other components in the M2M device 30. The power source 48 may be any suitable device for powering the M2M device 30. For example, the power source 48 may include one or more dry cell batteries (e.g., nickel-cadmium (NiCd), nickel-zinc (NiZn), nickel metal hydride (NiMH), lithium-ion (Li-ion), etc.), solar cells, fuel cells, and the like.

The processor 32 may also be coupled to the GPS chipset 50, which is configured to provide location information (*e.g*., longitude and latitude) regarding the current location of the M2M device 30. It will be appreciated that the M2M device 30 may acquire location information by way of any suitable location-determination method while remaining consistent with an example.

The processor 32 may further be coupled to other peripherals 52, which may include one or more software and/or hardware modules that provide additional features, functionality and/or wired or wireless connectivity. For example, the peripherals 52 may include an accelerometer, an e-compass, a satellite transceiver, a sensor, a digital camera (for photographs or video), a universal serial bus (USB) port, a vibration device, a television transceiver, a hands free headset, a Bluetooth® module, a frequency modulated (FM) radio unit, a digital music player, a media player, a video game player module, an Internet browser, and the like.

FIG. 60D is a block diagram of an exemplary computing system 90 on which, for example, the M2M service platform 22 of FIG. 60A and FIG. 60B may be implemented. Computing system 90 may comprise a computer or server and may be controlled primarily by computer readable instructions, which may be in the form of software, wherever, or by whatever means such software is stored or accessed. Such computer readable instructions may be executed within central processing unit (CPU) 91 to cause computing system 90 to do work. In many known workstations, servers, and personal computers, central processing unit 91 is implemented by a single-chip CPU called a microprocessor. In other machines, the central processing unit 91 may comprise multiple processors. Coprocessor 81 is an optional processor, distinct from main CPU 91, that performs additional functions or assists CPU 91. CPU 91 and/or coprocessor 81 may receive, generate, and process data related to the disclosed systems and methods for M2M ontology management and semantics interoperability, such as receiving an ontology publishing message.

In operation, CPU 91 fetches, decodes, and executes instructions, and transfers information to and from other resources via the computer's main data-transfer path, system bus 80. Such a system bus connects the components in computing system 90 and defines the medium for data exchange. System bus 80 typically includes data lines for sending data, address lines for sending addresses, and control lines for sending interrupts and for operating the system bus. An example of such a system bus 80 is the PCI (Peripheral Component Interconnect) bus.

Memory devices coupled to system bus 80 include random access memory (RAM) 82 and read only memory (ROM) 93. Such memories include circuitry that allows information to be stored and retrieved. ROMs 93 generally contain stored data that cannot easily be modified. Data stored in RAM 82 can be read or changed by CPU 91 or other hardware devices. Access to RAM 82 and/or ROM 93 may be controlled by memory controller 92. Memory controller 92 may provide an address translation function that translates virtual addresses into physical addresses as instructions are executed. Memory controller 92 may also provide a memory protection function that isolates processes within the system and isolates system processes from user processes. Thus, a program running in a first mode can access only memory mapped by its own process virtual address space; it cannot access memory within another process's virtual address space unless memory sharing between the processes has been set up.

In addition, computing system 90 may contain peripherals controller 83 responsible for communicating instructions from CPU 91 to peripherals, such as printer 94, keyboard 84, mouse 95, and disk drive 85.

Display 86, which is controlled by display controller 96, is used to display visual output generated by computing system 90. Such visual output may include text, graphics, animated graphics, and video. Display 86 may be implemented with a CRT-based video display, an LCD-based flat-panel display, gas plasma-based flat-panel display, or a touch-panel. Display controller 96 includes electronic components required to generate a video signal that is sent to display 86.

Further, computing system 90 may contain network adaptor 97 that may be used to connect computing system 90 to an external communications network, such as network 12 of FIG. 60A and FIG. 60B.

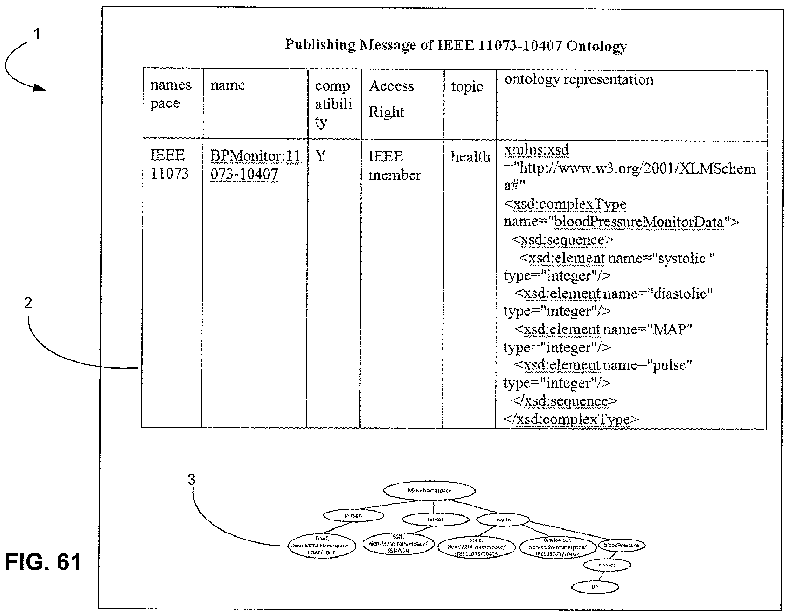

FIG. 61 illustrates an exemplary display that may be generated based on the methods and systems discussed herein. Display interface 1 (e.g., touch screen display) may provide text in block 2 associated with M2M ontology and semantics, such as the parameters of Table 1 through 15. In another example, progress of any of the steps (e.g., sent messages or success of steps) discussed herein may be displayed in block 2. In addition, graphical output 3 may be displayed on display interface 1. Graphical output 3 may be the topology of the ontology, a graphical output of the progress of any method or systems discussed herein, or the like

It is understood that any or all of the systems, methods and processes described herein may be embodied in the form of computer executable instructions (*i.e*., program code) stored on a computer-readable storage medium which instructions, when executed by a machine, such as a computer, server, M2M terminal device, M2M gateway device, or the like, perform and/or implement the systems, methods and processes described herein. Specifically, any of the steps, operations or functions described above may be implemented in the form of such computer executable instructions. Computer readable storage media include both volatile and nonvolatile, removable and non-removable media implemented in any method or technology for storage of information, but such computer readable storage media do not includes signals. Computer readable storage media include, but are not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other physical medium which can be used to store the desired information and which can be accessed by a computer.

In describing preferred examples of the subject matter of the present disclosure, as illustrated in the Figures, specific terminology is employed for the sake of clarity. Semantics related resources can be an ontology (an ontology could be composed of class, relationship and term), or an independent class, relationship, term.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art (e.g., skipping steps, combining steps, or adding steps between exemplary methods disclosed herein).

## Claims

1. A device for machine-to-machine, M2M, ontology processing, the device comprising:
a processor (721); and
a memory coupled with the processor, the memory comprising executable instructions that when executed by the processor cause the processor to effectuate operations comprising:
receiving an ontology publishing message comprising an ontology from a non-M2M namespace;
determining that a format representing the ontology is different from a Web Ontology Language, OWL, format used in an M2M namespace;
responsive to determining that the format representing the ontology is different from the OWL format used in the M2M namespace, converting the ontology to a format which is compatible with the OWL format used in the M2M namespace based on a concept name mapping proposal agreed between the M2M-namespace and the non-M2M namespace;
classifying the converted ontology including determining that the converted ontology belongs to the non-M2M namespace;
determining, based on the determination that the converted ontology belongs to the non-M2M namespace, a non-M2M namespace hierarchy of an ontology repository in which to store the converted ontology, the non-M2M namespace hierarchy comprising an individual namespace level as a first level, a second level containing ontologies under each of the individual namespaces in the first level and a third level including classes, relationships and terms defined in each of the ontologies contained in the second level;
extracting the classes and properties of the converted ontology;
storing the converted ontology under a namespace of the first level of the non-M2M namespace hierarchy of the ontology repository (727), and storing the extracted classes and properties of the converted ontology under classes and relationships of the third level correspondingly;
creating a new topic in an M2M namespace hierarchy of the ontology repository, the M2M hierarchy comprising a topic level as a first level, a second level containing ontologies or virtual ontologies which are ontologies relevant to a topic published from non-M2M namespaces, and a third level containing classes, relationships and terms defined in each ontology in the second level;
storing a link of the converted ontology as a virtual ontology under the created new topic,
accepting, by an ontology discovery component of the device, a discovery request from an M2M application, the discovery request including a request type field indicating whether the M2M application seeks an entire ontology, a class of an ontology or a relationship of an ontology, a search keys field indicating search keys transmitted by M2M application and a response type field indicating whether the M2M application seeks Uniform Resource Identifiers (URIs) of the entire ontology, the class of the ontology or the relationship of the ontology sought by the M2M application or URIs and representations of the entire ontology, the class of the ontology or the relationship of the ontology sought by the M2M application;
analyzing the discovery request and searching the ontology repository at the topic level of the M2M namespace hierarchy to find a matching topic which matches a topic indicated by the search keys transmitted by the M2M application,
determining that the matching topic is the created new topic under which the link of the converted ontology is stored as the virtual ontology, and in response,
transmitting, in accordance with the indications in the request type field and the response type field of the discovery request, a URI of the entire converted ontology, a class of the converted ontology or a relationship of the converted ontology or the URI and a representation of the entire converted ontology, the class of the converted ontology or the relationship of the converted ontology to the M2M application.

2. The device of claim 1, wherein, the ontology publishing message includes a message body comprising a schema of the ontology and a value of one or more fields, and the concept name mapping proposal is agreed by
reading the message body of the ontology publishing message including the schema of the ontology followed by the value of each field,
parsing each of the one or more fields of the ontology publishing message based on the schema in the ontology publishing message,
creating the concept mapping proposal based on a possible mapping between concept names that may be used by M2M namespaces and non-M2M namespaces which is maintained at the M2M device.

3. The device of claim 1, further operations comprising providing instructions to a display device to display the stored classes and properties of the converted ontology.

4. A method for machine-to-machine, M2M, ontology processing performed by a device, the method comprising:
receiving, by a processor of the device, an ontology publishing message comprising an ontology from a non-M2M namespace;
determining, by the processor of the device, that a format representing the ontology is different from a Web Ontology Language, OWL, format used in an M2M namespace;
responsive to determining that the format representing the ontology is different from the OWL format used in the M2M namespace, converting, by the processor of the device, the ontology to a format which is compatible with the OWL format used in the M2M namespace based on a concept name mapping proposal agreed between the M2M-namespace and the non-M2M namespace;
classifying, by the processor of the device, the converted ontology including determining that the converted ontology belongs to the non-M2M namespace;
determining, based on the determination that the converted ontology belongs to the non-M2M namespace, by the processor of the device, a non-M2M namespace hierarchy of an ontology repository in which to store the converted ontology, the non-M2M namespace hierarchy comprising an namespace level as a first level, a second level containing ontologies under each of the individual namespaces in the first level and a third level including classes, relationships and terms defined in each of the ontologies contained in the second level;
extracting, by the processor of the device, the classes and properties of the converted ontology,;
storing, by the processor of the device, the converted ontology under a namespace of the first level of the non-M2M namespace hierarchy of the ontology repository (727), and storing the extracted classes and properties of the converted ontology under classes and relationships of the third level correspondingly;
creating a new topic in an M2M namespace hierarchy of the ontology repository, the M2M hierarchy comprising a topic level as a first level, a second level containing ontologies or virtual ontologies which are ontologies relevant to a topic published from non-M2M namespaces, and a third level containing classes, relationships and terms defined in each ontology in the second level;
storing a link of the converted ontology as a virtual ontology under the created new topic,
accepting, by an ontology discovery component of the processor of the device, a discovery request from an M2M application, the discovery request including a request type field indicating whether the M2M application seeks an entire ontology, a class of an ontology or a relationship of an ontology, a search keys field indicating search keys transmitted by M2M application and a response type field indicating whether the M2M application seeks Uniform Resource Identifiers (URIs) of the entire ontology, the class of the ontology or the relationship of the ontology sought by the M2M application or URIs and representations of the entire ontology, the class of the ontology or the relationship of the ontology sought by the M2M application;
analyzing, by the processor of the device, the discovery request and searching the ontology repository at the topic level of the M2M namespace hierarchy to find a matching topic which matches a topic indicated by the search keys transmitted by the M2M application,
determining that the matching topic is the created new topic under which the link of the converted ontology is stored as the virtual ontology, and in response,
transmitting, in accordance with the indications in the request type field and the response type field of the discovery request, by the processor of the device, a URI of the entire converted ontology a class of the converted ontology or a relationship of the converted ontology or the URI and a representation of the entire converted ontology, the class of the converted ontology or the relationship of the converted ontology to the M2M application.

5. A computer-readable storage medium having a computer program stored thereon, the computer program being loadable into a data-processing unit and adapted to cause the data- processing unit to execute method steps according to claim 4 when the computer program is run by the data-processing unit.

## Patentansprüche

1. Vorrichtung für Machine-to-Machine, M2M, Ontologie-Verarbeitung, wobei die Vorrichtung umfasst:
einen Prozessor (721), und
einen mit dem Prozessor gekoppelten Speicher, wobei der Speicher ausführbare Befehle umfasst, die, wenn sie von dem Prozessor ausgeführt werden, den Prozessor veranlassen, Operationen zu bewirken, die umfassen:
Empfangen einer Ontologieveröffentlichungsnachricht, die eine Ontologie aus einem Nicht-M2M-Namensraum umfasst;
Bestimmen, dass sich ein Format, das die Ontologie repräsentiert, von einem in einem M2M-Namensraum verwendeten Format der "Web Ontology Language", OWL, unterscheidet;
als Reaktion auf das Bestimmen, dass sich das Format, das die Ontologie repräsentiert, von dem im M2M-Namensraum verwendeten OWL-Format unterscheidet, Konvertieren der Ontologie in ein Format, das mit dem im M2M-Namensraum verwendeten OWL-Format kompatibel ist, basierend auf einem zwischen dem M2M-Namensraum und dem Nicht-M2M-Namensraum vereinbarten Vorschlag zum Mappen von Konzeptnamen;
Klassifizieren der konvertierten Ontologie, einschließlich des Bestimmens, dass die konvertierte Ontologie zu dem Nicht-M2M-Namensraum gehört;
Bestimmen, basierend auf der Bestimmung, dass die konvertierte Ontologie zu dem Nicht-M2M-Namensraum gehört, einer Nicht-M2M-Namensraumhierarchie eines Ontologie-Repositorys, in dem die konvertierte Ontologie zu speichern ist, wobei die Nicht-M2M-Namensraumhierarchie eine individuelle Namensraumebene als eine erste Ebene, eine zweite Ebene, die Ontologien unter jedem der individuellen Namensräume in der ersten Ebene enthält, und eine dritte Ebene umfasst, die Klassen, Beziehungen und Begriffe beinhaltet, die in jeder der in der zweiten Ebene enthaltenen Ontologien definiert sind;
Extrahieren der Klassen und Eigenschaften der konvertierten Ontologie;
Speichern der konvertierten Ontologie unter einem Namensraum der ersten Ebene der Nicht-M2M-Namensraumhierarchie des Ontologie-Repositorys (727), und entsprechendes Speichern der extrahierten Klassen und Eigenschaften der konvertierten Ontologie unter Klassen und Beziehungen der dritten Ebene;
Erstellen eines neuen Themas in einer M2M-Namensraumhierarchie des Ontologie-Repositorys, wobei die M2M-Hierarchie eine Themenebene als erste Ebene umfasst, eine zweite Ebene, die Ontologien oder virtuelle Ontologien enthält, die Ontologien sind, die für ein Thema relevant sind, das von Nicht-M2M-Namensräumen veröffentlicht wird, und eine dritte Ebene, die Klassen, Beziehungen und Begriffe enthält, die in jeder Ontologie in der zweiten Ebene definiert sind;
Speichern eines Links der konvertierten Ontologie als virtuelle Ontologie unter dem erstellten neuen Thema, Akzeptieren einer Ermittlungsanforderung von einer M2M-Anwendung durch eine Ontologieermittlungskomponente der Vorrichtung, wobei die Ermittlungsanforderung ein Anforderungstypfeld beinhaltet, das angibt, ob die M2M-Anwendung eine gesamte Ontologie, eine Klasse einer Ontologie oder eine Beziehung einer Ontologie sucht, ein Suchschlüssel-Feld, das die von der M2M-Anwendung übertragenen Suchschlüssel angibt, und ein Antworttyp-Feld, das angibt, ob die M2M-Anwendung nach Uniform Resource Identifiers (URI) der gesamten Ontologie, der Klasse der Ontologie oder der Beziehung der von der M2M-Anwendung gesuchten Ontologie oder nach URI und Darstellungen der gesamten Ontologie, der Klasse der Ontologie oder der Beziehung der von der M2M-Anwendung gesuchten Ontologie sucht;
Analysieren der Ermittlungsanforderung und Durchsuchen des Ontologie-Repository auf der Themenebene der M2M-Namensraumhierarchie, um ein übereinstimmendes Thema zu finden, das mit einem Thema übereinstimmt, das durch die von der M2M-Anwendung übertragenen Suchschlüssel angegeben wird, Bestimmen, dass das übereinstimmende Thema das erstellte neue Thema ist, unter dem der Link der konvertierten Ontologie als die virtuelle Ontologie gespeichert ist, und als Reaktion, Übertragen eines URI der gesamten konvertierten Ontologie, einer Klasse der konvertierten Ontologie oder einer Beziehung der konvertierten Ontologie oder des URI und einer Darstellung der gesamten konvertierten Ontologie, der Klasse der konvertierten Ontologie oder der Beziehung der konvertierten Ontologie an die M2M-Anwendung gemäß den Angaben im Feld für den Anforderungstyp und im Feld für den Antworttyp der Ermittlungsanforderung.

2. Vorrichtung nach Anspruch 1, wobei die Ontologieveröffentlichungsnachricht einen Nachrichtentext beinhaltet, der ein Schema der Ontologie und einen Wert eines oder mehrerer Felder umfasst, und der Vorschlag für das Mappen des Konzeptnamens vereinbart wird durch
Lesen des Nachrichtentextes der Ontologieveröffentlichungsnachricht, der das Schema der Ontologie, gefolgt von den Werten der einzelnen Felder, beinhaltet,
Parsen jedes der ein oder mehreren Felder der Ontologieveröffentlichungsnachricht basierend auf dem Schema in der Ontologieveröffentlichungsnachricht,
Erstellen des Vorschlags für das Mappen von Konzepten basierend auf einem möglichen Mappen zwischen Konzeptnamen, die von M2M-Namensräumen und Nicht-M2M-Namensräumen verwendet werden können, die auf der M2M-Vorrichtung gepflegt werden.

3. Vorrichtung nach Anspruch 1, die ferner Operationen umfasst, die Anweisungen an eine Anzeigevorrichtung bereitstellen, um die gespeicherten Klassen und Eigenschaften der konvertierten Ontologie anzuzeigen.

4. Verfahren zur von einer Vorrichtung durchgeführten Machine-to-Machine, M2M, Ontologie-Verarbeitung, wobei das Verfahren umfasst:
Empfangen einer Ontologieveröffentlichungsnachricht, die eine Ontologie aus einem Nicht-M2M-Namensraum umfasst, durch einen Prozessor der Vorrichtung;
Bestimmen, dass sich ein Format, das die Ontologie repräsentiert, von einem in einem M2M-Namensraum verwendeten Format der "Web Ontology Language", OWL, unterscheidet, durch einen Prozessor der Vorrichtung;
als Reaktion auf das Bestimmen, dass sich das Format, das die Ontologie repräsentiert, von dem im M2M-Namensraum verwendeten OWL-Format unterscheidet, Konvertieren der Ontologie in ein Format, das mit dem im M2M-Namensraum verwendeten OWL-Format kompatibel ist, durch einen Prozessor der Vorrichtung, basierend auf einem zwischen dem M2M-Namensraum und dem Nicht-M2M-Namensraum vereinbarten Vorschlag zum Mappen von Konzeptnamen;
Klassifizieren der konvertierten Ontologie, einschließlich des Bestimmens, dass die konvertierte Ontologie zu dem Nicht-M2M-Namensraum gehört, durch einen Prozessor der Vorrichtung;
Bestimmen, basierend auf der Bestimmung, dass die konvertierte Ontologie zu dem Nicht-M2M-Namensraum gehört, durch den Prozessor der Vorrichtung, einer Nicht-M2M-Namensraumhierarchie eines Ontologie-Repositorys, in dem die konvertierte Ontologie zu speichern ist, wobei die Nicht-M2M-Namensraumhierarchie eine Namensraumebene als eine erste Ebene, eine zweite Ebene, die Ontologien unter jedem der einzelnen Namensräume in der ersten Ebene enthält, und eine dritte Ebene umfasst, die Klassen, Beziehungen und Begriffe beinhaltet, die in jeder der in der zweiten Ebene enthaltenen Ontologien definiert sind;
Extrahieren der Klassen und Eigenschaften der konvertierten Ontologie durch den Prozessor der Vorrichtung;
Speichern der konvertierten Ontologie durch den Prozessor der Vorrichtung unter einem Namensraum der ersten Ebene der Nicht-M2M-Namensraumhierarchie des Ontologie-Repositorys (727), und entsprechendes Speichern der extrahierten Klassen und Eigenschaften der konvertierten Ontologie unter Klassen und Beziehungen der dritten Ebene;
Erstellen eines neuen Themas in einer M2M-Namensraumhierarchie des Ontologie-Repositorys, wobei die M2M-Hierarchie eine Themenebene als erste Ebene umfasst, eine zweite Ebene, die Ontologien oder virtuelle Ontologien enthält, bei denen es sich um Ontologien handelt, die für ein Thema relevant sind, das von Nicht-M2M-Namensräumen veröffentlicht wird, und eine dritte Ebene, die Klassen, Beziehungen und Begriffe enthält, die in jeder Ontologie in der zweiten Ebene definiert sind;
Speichern eines Links der konvertierten Ontologie als virtuelle Ontologie unter dem erstellten neuen Thema, Akzeptieren einer Ermittlungsanforderung von einer M2M-Anwendung durch eine Ontologieermittlungskomponente des Prozessors der Vorrichtung, wobei die Ermittlungsanforderung ein Anforderungstypfeld beinhaltet, das angibt, ob die M2M-Anwendung eine gesamte Ontologie, eine Klasse einer Ontologie oder eine Beziehung einer Ontologie sucht, ein Suchschlüssel-Feld, das die von der M2M-Anwendung übertragenen Suchschlüssel angibt, und ein Antworttyp-Feld, das angibt, ob die M2M-Anwendung nach Uniform Resource Identifiers (URI) der gesamten Ontologie, der Klasse der Ontologie oder der Beziehung der von der M2M-Anwendung gesuchten Ontologie oder nach URI und Darstellungen der gesamten Ontologie, der Klasse der Ontologie oder der Beziehung der von der M2M-Anwendung gesuchten Ontologie sucht;
Analysieren der Ermittlungsanforderung und Durchsuchen des Ontologie-Repository auf der Themenebene der M2M-Namensraumhierarchie durch den Prozessor der Vorrichtung, um ein übereinstimmendes Thema zu finden, das mit einem Thema übereinstimmt, das durch die von der M2M-Anwendung übertragenen Suchschlüssel angegeben wird,
Bestimmen, dass das übereinstimmende Thema das erstellte neue Thema ist, unter dem der Link der konvertierten Ontologie als die virtuelle Ontologie gespeichert ist, und als Reaktion,
Übertragen eines URI der gesamten konvertierten Ontologie, einer Klasse der konvertierten Ontologie oder einer Beziehung der konvertierten Ontologie oder
des URI und einer Darstellung der gesamten konvertierten Ontologie, der Klasse der konvertierten Ontologie oder der Beziehung der konvertierten Ontologie an die M2M-Anwendung gemäß den Angaben im Feld für den Anforderungstyp und im Feld für den Antworttyp der Ermittlungsanforderung durch den Prozessor der Vorrichtung.

5. Computerlesbares Speichermedium mit einem darauf gespeicherten Computerprogramm, wobei das Computerprogramm in eine Datenverarbeitungseinheit geladen werden kann und angepasst ist, die Datenverarbeitungseinheit zu veranlassen, Verfahrensschritte nach Anspruch 4 auszuführen, wenn das Computerprogramm durch die Datenverarbeitungseinheit abläuft.

## Revendications

1. Dispositif de traitement d'ontologies de machine à machine, M2M, le dispositif comprenant :
un processeur (721) ; et
une mémoire couplée au processeur, la mémoire comprenant des instructions exécutables qui,
lorsqu'elles sont exécutées par le processeur, amènent le processeur à effectuer des opérations comprenant :
la réception d'un message de publication d'ontologie comprenant une ontologie provenant d'un espace de noms non M2M ;
la détermination du fait qu'un format représentant l'ontologie est différent d'un format de Langage d'Ontologie Web, OWL, utilisé dans un espace de noms M2M ;
en réponse à la détermination du fait que le format représentant l'ontologie est différent du format OWL utilisé dans l'espace de noms M2M, la conversion de l'ontologie en un format qui est compatible avec le format OWL utilisé dans l'espace de noms M2M sur la base d'une proposition de mise en correspondance de noms de concepts convenue entre l'espace de noms M2M et l'espace de noms non M2M ;
la classification de l'ontologie convertie comprenant la détermination du fait que l'ontologie convertie appartient à l'espace de noms non M2M ;
la détermination, sur la base de la détermination du fait que l'ontologie convertie appartient à l'espace de noms non M2M, d'une hiérarchie d'espaces de noms non M2M d'un référentiel d'ontologies dans lequel doit être stockée l'ontologie convertie, la hiérarchie d'espaces de noms non M2M comprenant un niveau d'espace de noms individuel en tant que premier niveau, un deuxième niveau contenant des ontologies sous chacun des espaces de noms individuels du premier niveau et un troisième niveau comportant des classes, des relations et des termes définis dans chacune des ontologies contenues dans le deuxième niveau ;
l'extraction des classes et propriétés de l'ontologie convertie ;
le stockage de l'ontologie convertie sous un espace de noms du premier niveau de la hiérarchie d'espaces de noms non M2M du référentiel d'ontologies (727), et le stockage de manière correspondante des classes et propriétés extraites de l'ontologie convertie sous les classes et relations du troisième niveau ;
la création d'un nouveau sujet dans une hiérarchie d'espaces de noms M2M du référentiel d'ontologies, la hiérarchie M2M comprenant un niveau de sujet en tant que premier niveau, un deuxième niveau contenant des ontologies ou des ontologies virtuelles qui sont des ontologies relevant d'un sujet publié provenant d'espaces de noms non M2M, et un troisième niveau contenant des classes, des relations et des termes définis dans chaque ontologie du deuxième niveau ;
le stockage d'un lien de l'ontologie convertie en tant qu'ontologie virtuelle sous le nouveau sujet créé, l'acceptation, par un composant de découverte d'ontologies du dispositif, d'une demande de découverte provenant d'une application M2M, la demande de découverte comportant un champ de type de demande indiquant si l'application M2M recherche la totalité d'une ontologie,
une classe d'une ontologie ou une relation d'une ontologie, un champ de clés de recherche indiquant des clés de recherche transmises par l'application M2M et un champ de type de réponse indiquant si l'application M2M recherche des Identifiants de Ressources Uniformes (URIs) de la totalité de l'ontologie, la classe de l'ontologie ou la relation de l'ontologie recherchée par l'application M2M ou des URIs et des représentations de la totalité de l'ontologie, de la classe de l'ontologie ou de la relation de l'ontologie recherchée par l'application M2M ;
l'analyse de la demande de découverte et la recherche dans le référentiel d'ontologies au niveau du sujet de la hiérarchie de l'espace de noms M2M pour trouver un sujet correspondant qui correspond à un sujet indiqué par les clés de recherche transmises par l'application M2M,
la détermination du fait que le sujet correspondant est le nouveau sujet créé sous lequel le lien de l'ontologie convertie est stocké en tant qu'ontologie virtuelle, et en réponse,
la transmission, conformément aux indications contenues dans le champ de type de demande et le champ de type de réponse de la demande de découverte, d'un URI de la totalité de l'ontologie convertie, d'une classe de l'ontologie convertie ou d'une relation de l'ontologie convertie ou de l'URI et d'une représentation de la totalité de l'ontologie convertie, de la classe de l'ontologie convertie ou de la relation de l'ontologie convertie à l'application M2M.

2. Dispositif selon la revendication 1, dans lequel le message de publication d'ontologie comporte un corps de message comprenant un schéma de l'ontologie et une valeur d'un ou plusieurs champs, et la proposition de mise en correspondance de noms de concepts est acceptée par
la lecture du corps de message du message de publication d'ontologie comportant le schéma de l'ontologie suivi de la valeur de chaque champ, l'analyse syntaxique de chacun desdits un ou plusieurs champs du message de publication d'ontologie sur la base du schéma contenu dans le message de publication d'ontologie,
la création de la proposition de mise en correspondance de concepts sur la base d'une mise en correspondance possible entre des noms de concepts qui peuvent être utilisés par des espaces de noms M2M et des espaces de noms non M2M, qui est maintenue au niveau du dispositif M2M.

3. Dispositif selon la revendication 1, des opérations supplémentaires comprenant la fourniture d'instructions à un dispositif d'affichage pour afficher les classes et propriétés stockées de l'ontologie convertie.

4. Procédé de traitement d'ontologies de machine à machine, M2M, exécuté par un dispositif, le procédé comprenant :
la réception, par un processeur du dispositif, d'un message de publication d'ontologie comprenant une ontologie provenant d'un espace de noms non M2M ;
la détermination, par le processeur du dispositif, du fait qu'un format représentant l'ontologie est différent d'un format de Langage d'Ontologie Web, OWL, utilisé dans un espace de noms M2M ;
en réponse à la détermination du fait que le format représentant l'ontologie est différent du format OWL utilisé dans l'espace de noms M2M, la conversion, par le processeur du dispositif, de l'ontologie en un format qui est compatible avec le format OWL utilisé dans l'espace de noms M2M sur la base d'une proposition de mise en correspondance de noms de concepts convenue entre l'espace de noms M2M et l'espace de noms non M2M ;
la classification, par le processeur du dispositif, de l'ontologie convertie comprenant la détermination du fait que l'ontologie convertie appartient à l'espace de noms non M2M ;
la détermination, sur la base de la détermination du fait que l'ontologie convertie appartient à l'espace de noms non M2M, par le processeur du dispositif, d'une hiérarchie d'espaces de noms non M2M d'un référentiel d'ontologies dans lequel doit être stockée l'ontologie convertie, la hiérarchie d'espaces de noms non M2M comprenant un niveau d'espace de noms en tant que premier niveau, un deuxième niveau contenant des ontologies sous chacun des espaces de noms individuels du premier niveau et un troisième niveau comportant des classes, des relations et des termes définis dans chacune des ontologies contenues dans le deuxième niveau ;
l'extraction, par le processeur du dispositif, des classes et propriétés de l'ontologie convertie ;
le stockage, par le processeur du dispositif, de l'ontologie convertie sous un espace de noms du premier niveau de la hiérarchie d'espaces de noms non M2M du référentiel d'ontologies (727), et le stockage de manière correspondante des classes et propriétés extraites de l'ontologie convertie sous les classes et relations du troisième niveau ;
la création d'un nouveau sujet dans une hiérarchie d'espaces de noms M2M du référentiel d'ontologies, la hiérarchie M2M comprenant un niveau de sujet en tant que premier niveau, un deuxième niveau contenant des ontologies ou des ontologies virtuelles qui sont des ontologies relevant d'un sujet publié provenant d'espaces de noms non M2M, et un troisième niveau contenant des classes, des relations et des termes définis dans chaque ontologie du deuxième niveau ;
le stockage d'un lien de l'ontologie convertie en tant qu'ontologie virtuelle sous le nouveau sujet créé, l'acceptation, par un composant de découverte d'ontologies du processeur du dispositif, d'une demande de découverte provenant d'une application M2M, la demande de découverte comportant un champ de type de demande indiquant si l'application M2M recherche la totalité d'une ontologie, une classe d'une ontologie ou une relation d'une ontologie, un champ de clés de recherche indiquant des clés de recherche transmises par l'application M2M et un champ de type de réponse indiquant si l'application M2M recherche des Identifiants de Ressources Uniformes (URIs) de la totalité de l'ontologie, la classe de l'ontologie ou la relation de l'ontologie recherchée par l'application M2M ou des URIs et des représentations de la totalité de l'ontologie, de la classe de l'ontologie ou de la relation de l'ontologie recherchée par l'application M2M ;
l'analyse, par le processeur du dispositif, de la demande de découverte et la recherche dans le référentiel d'ontologies au niveau du sujet de la hiérarchie de l'espace de noms M2M pour trouver un sujet correspondant qui correspond à un sujet indiqué par les clés de recherche transmises par l'application M2M,
la détermination du fait que le sujet correspondant est le nouveau sujet créé sous lequel le lien de l'ontologie convertie est stocké en tant qu'ontologie virtuelle, et en réponse,
la transmission, conformément aux indications contenues dans le champ de type de demande et le champ de type de réponse de la demande de découverte, par le processeur du dispositif, d'un URI de la totalité de l'ontologie convertie, d'une classe de l'ontologie convertie ou d'une relation de l'ontologie convertie ou de l'URI et d'une représentation de la totalité de l'ontologie convertie, de la classe de l'ontologie convertie ou de la relation de l'ontologie convertie à l'application M2M.

5. Support de stockage lisible par ordinateur sur lequel est stocké un programme informatique, le programme informatique pouvant être chargé dans une unité de traitement de données et étant conçu pour amener l'unité de traitement de données à exécuter des étapes de procédé selon la revendication 4 lorsque le programme informatique est exécuté par l'unité de traitement de données.
